# EUROPEAN PATENT SPECIFICATION

(11) **EP 2 908 132 B1**
(45) Date of publication and mention of the grant of the patent: **24.04.2019**
(21) Application number: 15153870.9
(22) Date of filing: 02.03.2012
(51) Int. Cl.: G01N 33/50, G01N 33/574

(54) **Prediction of drug sensitivity of lung tumors based on molecular and genetic signatures**
Vorhersage der Arzneimittelsensitivität von Tumoren der Lunge auf der Basis von molekularen und genetischen Signaturen
Prédiction de la sensibilité aux médicaments de tumeurs du poumon sur la base de signatures génétiques et moléculaires

(30) Priority: 02.03.2011 US 201161448479 P
(43) Date of publication of application: 19.08.2015
(62) Divisional of application: 12708484.6
(73) Proprietor: Pierian Holdings, Inc., Franklin, TN 37067 (US)
(72) Inventor: Gong, Hua, San Diego, CA 92130-2274 (US); Sharat, Singh, Rancho Santa Fe, CA 92127 (US)
(74) Representative: J A Kemp

(56) References cited:
- HUA C. GONG ET AL: "Signatures of Drug Sensitivity in Nonsmall Cell Lung Cancer", INTERNATIONAL JOURNAL OF PROTEOMICS, vol. 2011, 3 June 2011 (2011-06-03), pages 1-13, XP055030274, ISSN: 2090-2166, DOI: 10.1155/2011/215496
- YAUCH R L ET AL: "Epithelial versus mesenchymal phenotype determines in vitro sensitivity and predicts clinical activity of erlotinib in lung cancer patients", CLINICAL CANCER RESEARCH, THE AMERICAN ASSOCIATION FOR CANCER RESEARCH, US, vol. 11, no. 24, PART 1, 15 December 2005 (2005-12-15), pages 8686-8698, XP002391561, ISSN: 1078-0432, DOI: 10.1158/1078-0432.CCR-05-1492
- MUKOHARA T ET AL: "Differential effects of gefitinib and cetuximab on non-small-cell lung cancers bearing epidermal growth factor receptor mutations", JOURNAL OF THE NATIONAL CANCER INSTITUTE, OXFORD UNIVERSITY PRESS, GB, vol. 97, no. 16, 17 August 2005 (2005-08-17), pages 1185-1194, XP002447440, ISSN: 0027-8874
- KAZUYA MACHIDA ET AL: "Characterizing Tyrosine Phosphorylation Signaling in Lung Cancer Using SH2 Profiling", PLOS ONE, vol. 5, no. 10, 1 January 2010 (2010-01-01), page e13470, XP055030560, ISSN: 1932-6203, DOI: 10.1371/journal.pone.0013470
- AMANN J ET AL: "Aberrant epidermal growth factor receptor signaling and enhanced sensitivity to EGFR inhibitors in lung cancer", CANCER RESEARCH, AMERICAN ASSOCIATION FOR CANCER RESEARCH, US, vol. 65, no. 1, 1 January 2005 (2005-01-01), pages 226-235, XP002397276, ISSN: 0008-5472

## Description

### CROSS-REFERENCE TO RELATED APPLICATION

This application claims priority to U.S. Provisional Application No. 61/448,479, filed March 2, 2011.

### BACKGROUND OF THE INVENTION

Lung cancer is the leading cause of cancer-related deaths worldwide, resulting in 1.35 million new cases and 1.8 million deaths per year according to the World Health Organization (WHO) estimation in 2009 (World Health Organization Fact Sheet No 297 February 2009; http://www.who.int/mediacentre/factsheets/fs297/en/index.html). Lung cancer is generally classified histologically into two major types, small cell lung cancer (SCLC) and non-small cell lung cancer (NSCLC). Approximately 85-90% of lung cancers are NSCLC representing three major subtypes based on tumor cell size, shape and composition, with adenocarcinoma accounting for 40%, squamous cell lung carcinoma 25-30%, and large-cell lung carcinoma accounting for 10-15% of all lung cancers (Vaporciyan et al., Holland-Frei Cancer Medicine, 5th Edition, BC Decker, pgs. 1227-1292 (2000); Roggli et al., Hum Pathol., Jun;16(6):569-79 (1985)).

Although less than optimal, current conventional treatment for lung cancer consists of surgery for operable candidates and chemotherapy for disease-advanced patients, with the mean survival for most advanced lung cancer patients being less than one year (Schiller et al., N Engl J Med. Jan 10; 346(2):92-8 (2002)). During the last decade, considerable progress has been made in the treatment of NSCLC due to the emergence of new targeted therapies specific to the oncogenic tyrosine kinase pathways activated in tumor cells. For example, two epidermal growth factor receptor (EGFR) tyrosine kinase inhibitors (TKI), Gefitinib (Iressa) and Erlotinib (Tarceva) have been FDA-approved for the treatment of locally advanced or metastatic NSCLC that has failed at least one prior chemotherapy regimen (Comis RL, The Oncologist, Aug; 20(7):467-70, 2005; Ramalingam S., Sandler AB, The Oncologist, Jun: 11(6):655-65 (2006)). Other receptor tyrosine kinase (RTK) pathway inhibitors, such as Sunitinib (Sutent), which targets the platelet-derived growth factor receptors and vascular endothelial growth factor receptors, as well as Crizotinib, a hepatocyte growth factor RTK inhibitor, are in advanced clinical trials for NSCLC (Papaetis GS, Syrigos KN, BioDrugs., Dec; 23(6):377-89 (2009); Neal JW, Sequist LV, Curr Treat Options Oncol., Jun; 11(1-2):36-44 (2010)).

The advances made in targeted therapy for NSCLC are based on understanding the mechanism by which mutated genes confer a neoplastic phenotype on tumor cells and how the targeted interruption of these oncogenic pathways leads to clinical response. Thus, analysis of a pathway-focused panel of biomarkers in fresh tumor tissue samples collected from patients could pave the way for determining if the markers are associated with the optimal clinical therapy and may provide predictive value in identifying responsive patients. In addition, drug combinations targeted against the receptors affecting downstream signaling molecules may overcome pathway activation and drug resistance often seen in NSCLC therapy. Yauch *et al* reports that epithelial versus mesenchymal phenotype determines *in vitro* sensitivity and predicts clinical activity of erlotinib in lung cancer patients (Clinical Cancer Research, vol. 11, no. 24, part 1, 2005, pages 8686-8698). Mukohara *et al* reports differential effects of gefitinib and cetuximab on non-small-cell lung cancers bearing epidermal growth factor receptor mutations (Journal of The National Cancer Institute, vol. 97, no. 16, 2005, pages 1185-1194). Machida *et al* reports characterizing tyrosine phosphorylation signaling in lung cancer using SH2 profiling *(*PLoS ONE, vol. 5, no. 10, 1, 2010, page e13470). Amann *et al* reports aberrant epidermal growth factor receptor signaling and enhanced sensitivity to EGFR inhibitors in lung cancer (Cancer Research, vol. 65, no. 1, 2005, pages 226-235). However, difficulties in predicting efficacy in targeted therapy is due to the limited knowledge of the activated oncogenic pathways in a patient's tumor so that the appropriate inhibitor(s) are not prescribed. The present invention satisfies this need and provides other advantages as well.

### BRIEF SUMMARY OF THE INVENTION

The present disclosure provides methods for predicting therapeutic efficacy or response to one or a combination of anticancer drugs in a subject having a lung cancer such as non-small cell lung cancer (NSCLC). In particular, the methods of the present disclosure comprise analyzing a sample such as a tumor tissue sample obtained from a subject having lung cancer to determine the presence, expression level, activation level, and/or genotype of one or more markers to obtain a marker profile, and comparing the marker profile with known marker profiles obtained from one or more lung cancer cell lines such as a library of cell lines to thereby predict therapeutic efficacy or response to one or a combination of anticancer drugs. Accordingly, the present disclosure is particularly useful in predicting therapeutic efficacy or response to one or more anticancer drugs by analyzing one or a panel of signal transduction pathway biomarkers and/or mutated genes in tumor tissue obtained from a subject with lung cancer to guide treatment options for the subject based upon similarities in marker profiles obtained from the tumor tissue and lung cancer cell lines and the drug sensitivity of those lung cancer cell lines.

As described herein, receptor tyrosine kinase pathway activation and gene mutations in both human lung tumor cell lines and human lung tumor tissue samples were profiled to define molecular pathways that are useful for evaluating the efficacy of targeted therapies. A panel of kinase inhibitors was used to determine whether blocking pathway activation affected the anchorage-dependent and independent growth of the tumor cell lines. Hierarchical clustering of primary tumor samples with the corresponding tumor cell lines based on their pathway signatures revealed potential treatment options for the primary tumors based on the tumor cell line response to the panel of kinase inhibitors. Subsets of tumors were identified as candidates for treatment with inhibitors of EGFR, c-Met, IGF-1R, MEK, and PI3K. The EGFR pathway in EGFR mutant cell lines HCC827 and H1975 were found to be highly activated and sensitive to inhibitors blocking EGFR signaling. H1993 is a c-Met amplified cell line showing c-Met and EGFR pathway activation and responsiveness to c-Met inhibitor treatment. IGF-1R pathway activated H358 and A549 cells are sensitive to IGF-1R inhibition. The downstream PI3K inhibitor, BEZ-235, effectively inhibited tumor cell growth in most of the cell lines tested, except the H1993 and H1650 cells, while the MEK inhibitor PD-325901 was effective in blocking the growth of KRAS mutated cell line H1734, but not the other KRAS mutated cell lines, H358, A549 and H460. Primary tumors were identified with similar profiles for EGFR, c-Met and IGF-1R pathway activation and those patients were predicted to be candidates for treatment with specific individual or combinations of kinase inhibitors. Thus, the present disclosure combines lung cancer cell line response to tyrosine kinase inhibitors, pathway biomarker information and/or mutational status, and the identification of similar pathways within subsets of primary tumor samples, to inform appropriate future treatment options for lung cancer patients.

In certain aspects, the present invention provides a method for predicting therapeutic efficacy or response to an anticancer drug in a subject having lung cancer, said method comprising:
(a) determining the phosphorylation level of HER1, HER2, and HER3 in a cellular extract produced from a cancer cell which has been isolated from said subject;
(b) comparing the phosphorylation level of HER1, HER2, and HER3 in the cellular extract to a phosphorylation level of HER1, HER2, and HER3 in each of the following lung cancer cell lines: HCC827, H1975, H1734, H1993, H358, H1650, A549, and H460;
   wherein each anticancer drug selected from the group consisting of a HER1 inhibitor, a HER1/2 inhibitor, a HER1/2/4 inhibitor, a c-Met inhibitor, an IGF-1R inhibitor, a MEK inhibitor, a PI3K inhibitor, and a combination thereof has been determined to produce or not produce a response in each of the lung cancer cell lines;
(c) identifying similarities between the phosphorylation level of HER1, HER2, and HER3 in the cellular extract and in the lung cancer cell lines; and
(d) predicting that the lung cancer of the subject will respond to the same anticancer drug or combination thereof that produces a response in a lung cancer cell line of step (b) based on similarities between the phosphorylation level of HER1, HER2, and HER3 in the cellular extract and the phosphorylation level of HER1, HER2, and HER3 in the lung cancer cell line.

In some instances, the cellular extract is further or alternatively analyzed to determine the genotype of one or more (*e.g*., a plurality of) markers such as KRAS, P53, and/or STK11. In certain embodiments, the cellular extract is produced from a cancer cell which has been isolated from a primary lung tumor tissue sample such as, *e.g.,* a lung adenocarcinoma sample.

The anticancer drugs include specific as well as multiple RTK inhibitors, *e.g*., HER1/2/4 (epidermal growth factor receptors) inhibitors (*e.g*., erlotinib for HER1, lapatinib for HER1/2, gefitinib for HER1/2/4, and BIBW-2992, an irreversible inhibitor for HER1/2); c-Met (hepatocyte growth factor receptor) inhibitor, PF-2341066; IGF-1R (insulin-like growth factor-1 receptor) inhibitor BMS-536924; MEK (mitogen-activated protein kinase kinase) inhibitor, PD-325901; and PI3K (phosphatidylinositol-3-kinase) and mTOR (mammalian target of rapamycin) inhibitor BEZ-235. In certain instances, a combination of two kinase inhibitors, *e.g*., one inhibiting the appropriate RTK and the other inhibiting a downstream signaling pathway (or a combination of two downstream kinase inhibitors) is recommended and/or selected.

Phosphorylation levels can be determined using any of a variety of techniques. In certain embodiments, phosphorylation level is detected with an immunoassay such as a single detection assay or a proximity dual detection assay (*e.g.,* a Collaborative Enzyme Enhanced Reaction (CEER™) assay) as described herein.

The Collaborative Enzyme Enhanced Reactive Immunoassay (CEER™) technology is described in the following patent documents: PCT Publication Nos. WO 2008/036802, WO 2009/012140, WO 2009/108637, WO 2010/132723, WO 2011/008990, and WO 2011/050069; and PCT Application No. PCT/US2011/066624.

Other objects, features, and advantages of the present invention will be apparent to one of skill in the art from the following detailed description and figures.

### BRIEF DESCRIPTION OF THE DRAWINGS

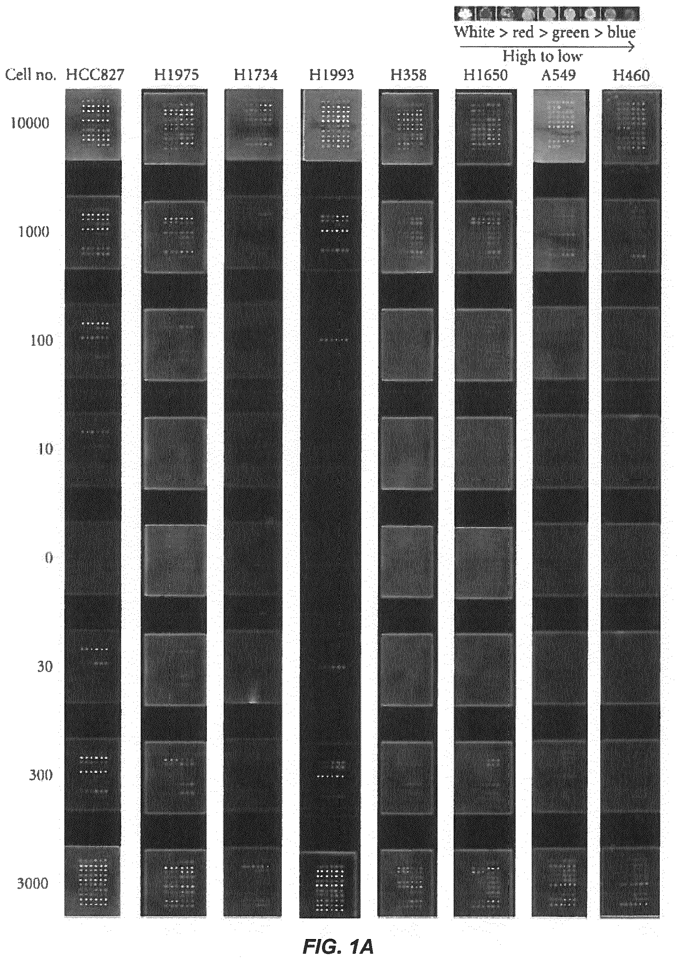

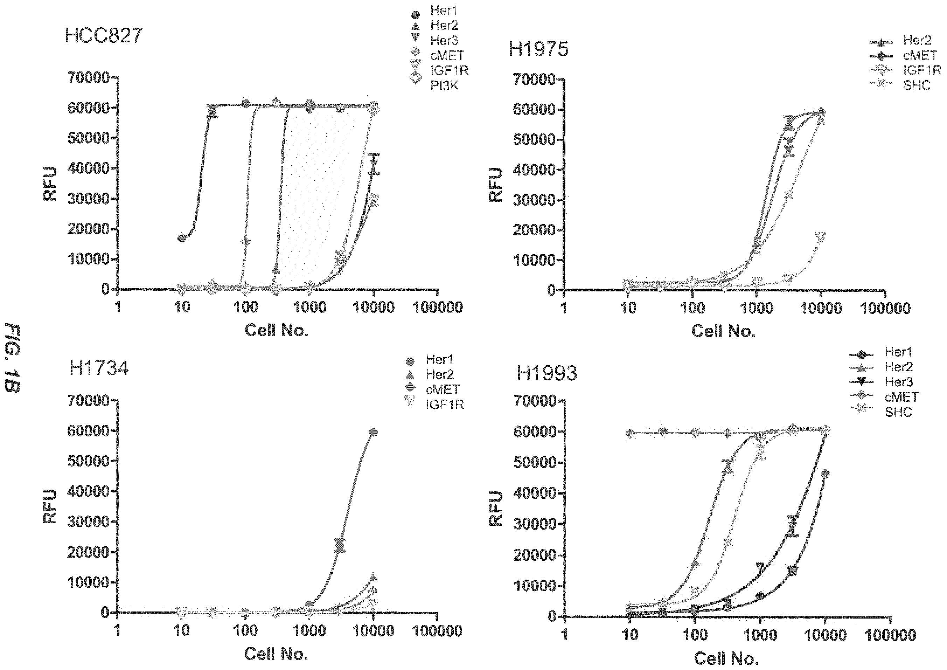

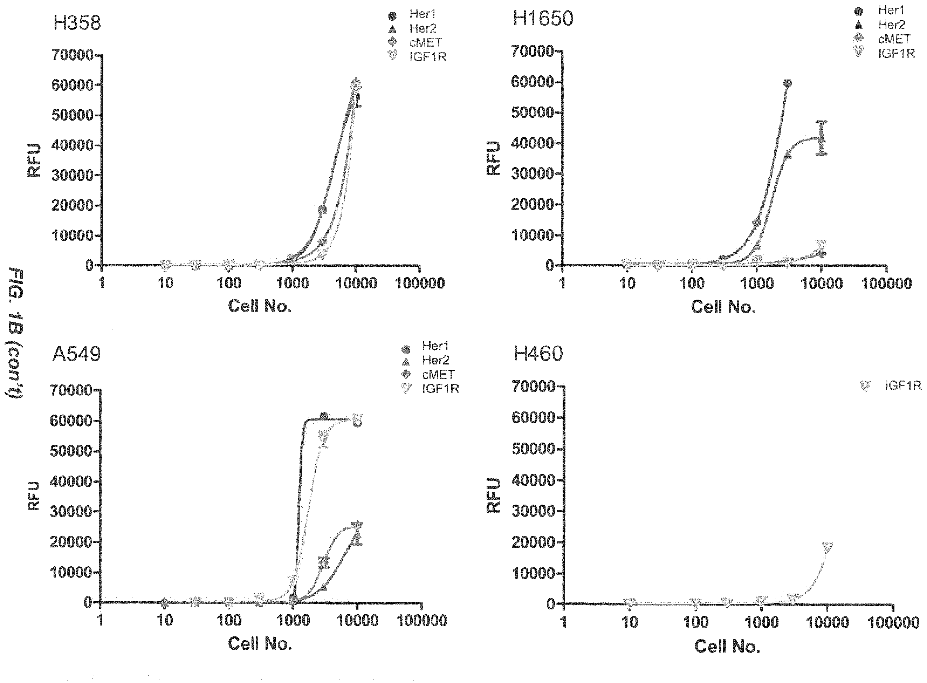

**Figure 1****.** Profiling of the activated (p-Tyr) RTK signaling pathways in the lung tumor cell lines. (A) Slide images of the CEER™ assay obtained from the eight tumor cell lines. (B) Graphical representation of the activated (p-Tyr) RTK pathways in the lung tumor cell lines profiled by the CEER™ assay. The cells were cultured in the presence of 10% FBS in their respective medium until 80% confluence and cell lysates were prepared in lysis buffer. The activation signal was determined from the harvested lysate. In each cell line the activated pathways are shown in RFU (relative fluorescent units) based on the lysate obtained from the number of cells being assayed.

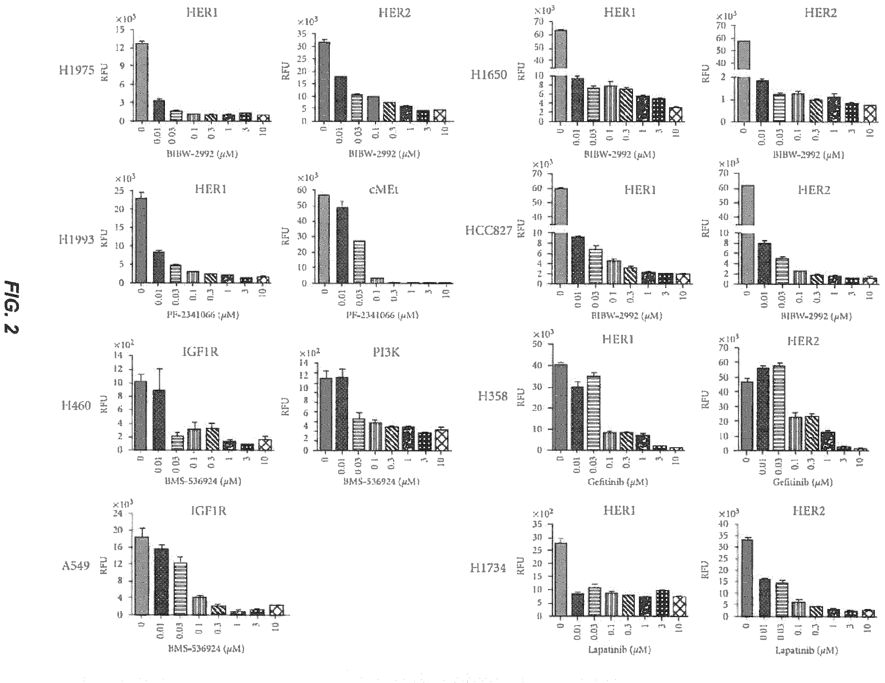

**Figure 2****.** Inhibition of signaling pathway activation in lung tumor cell lines by kinase inhibitors. Lung tumor cells were cultured in 10% FBS until reaching ∼80% confluence and then the cells were starved in serum free medium for overnight, followed by 4-hour treatment with the inhibitors. Cell lysates were then prepared and used for determination of the pathway activation signals by the CEER™ assay.

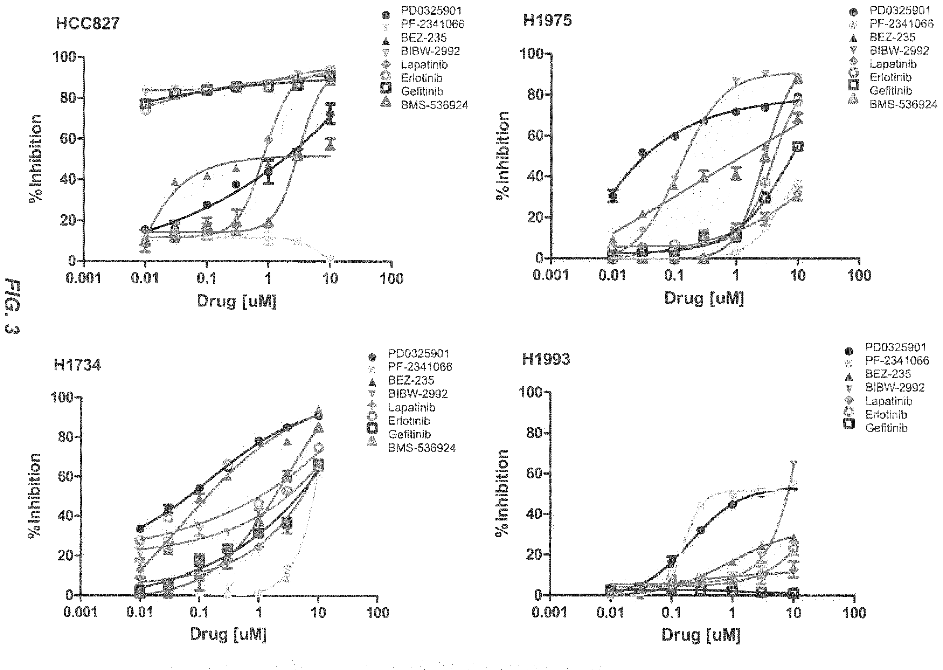

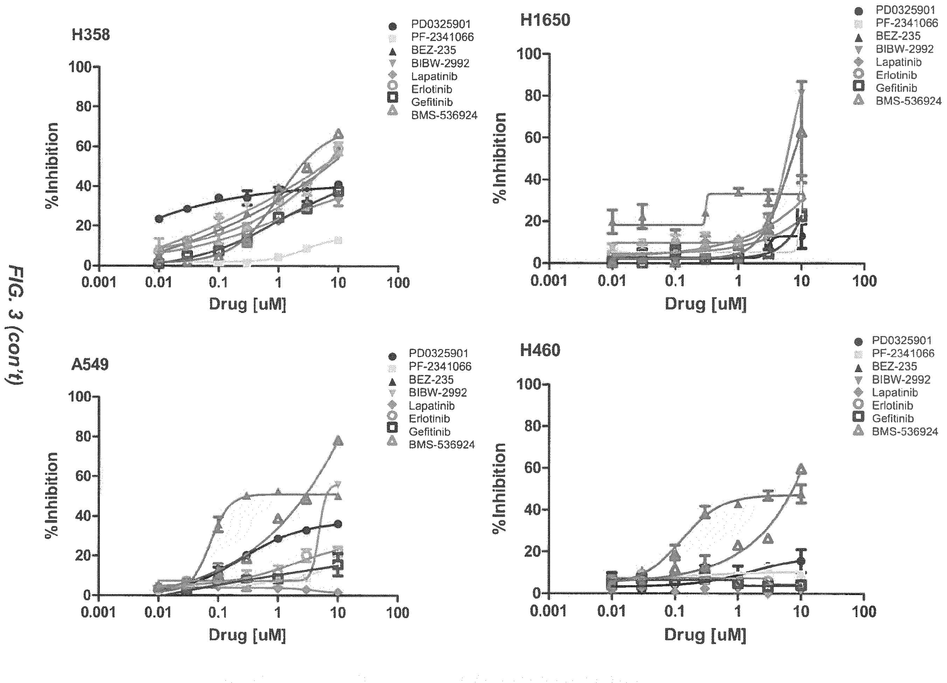

**Figure 3****.** Inhibition of lung tumor cell growth by kinase inhibitors. Lung tumor cells were cultured in 5% FBS plus increasing concentrations of the indicated inhibitors, ranging from 0.01-10 µM, for 48 hours. Determination of cell proliferation was performed with the CellTiter-Glo Luminescent Cell Viability Assay.

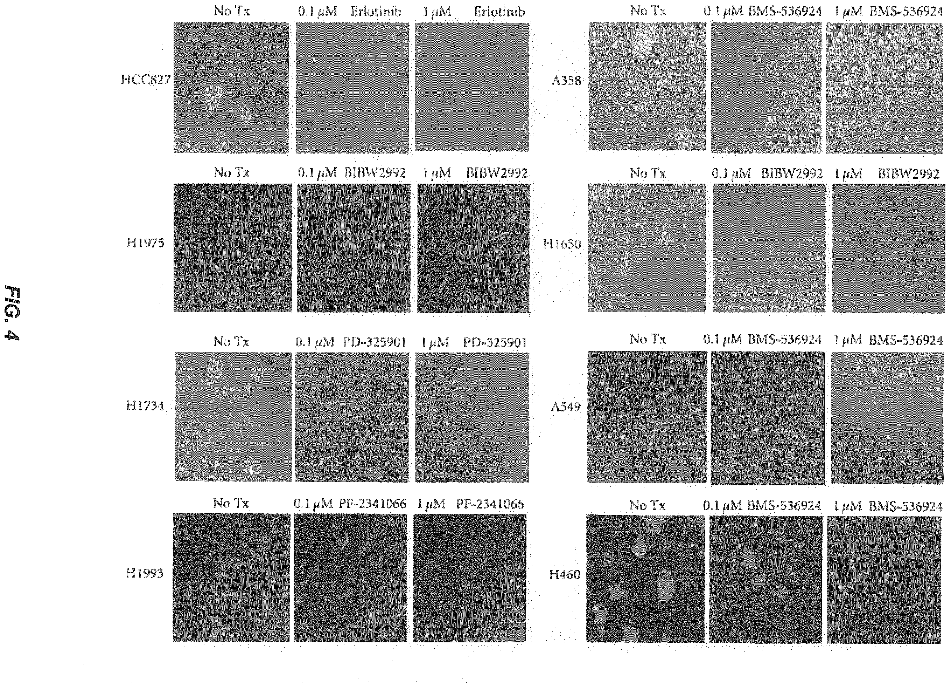

**Figure 4****.** Inhibition of anchorage-independent growth of lung tumor cell lines by selected inhibitors. Each selected cell line was treated with the indicated inhibitor at 0.1 µM and 1 µM concentrations for two weeks and cell colony size formation was scored under the Nikon inverted-phase microscope.

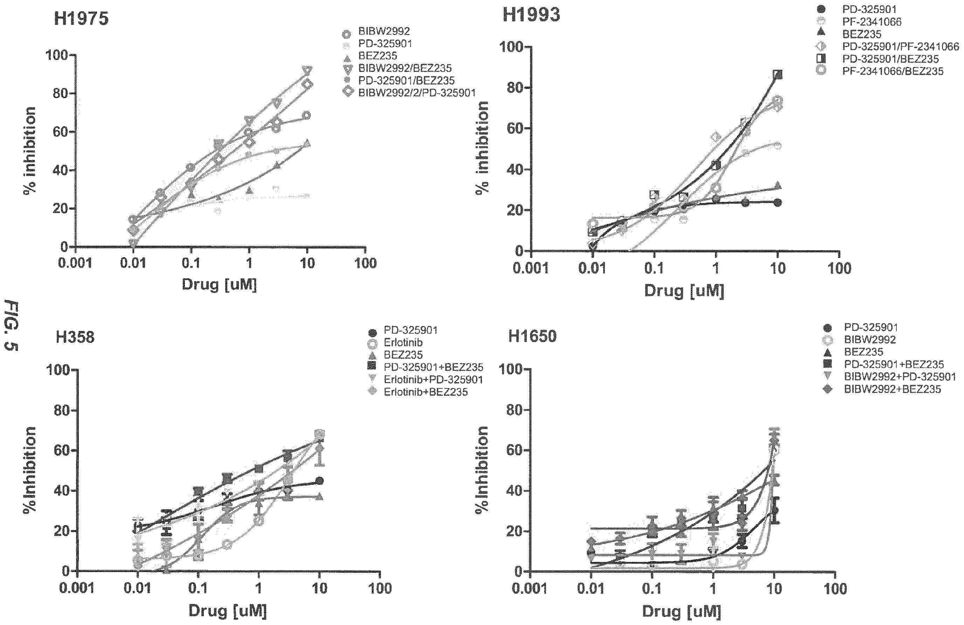

**Figure 5****.** Inhibition of lung tumor cell growth by a combination of two kinase inhibitors. Lung tumor cell lines were cultured in 5% FBS plus increasing concentrations of the indicated single kinase inhibitor or a combination of the two indicated kinase inhibitors. Determination of cell proliferation was performed with the CellTiter-Glo Luminescent Cell Viability Assay.

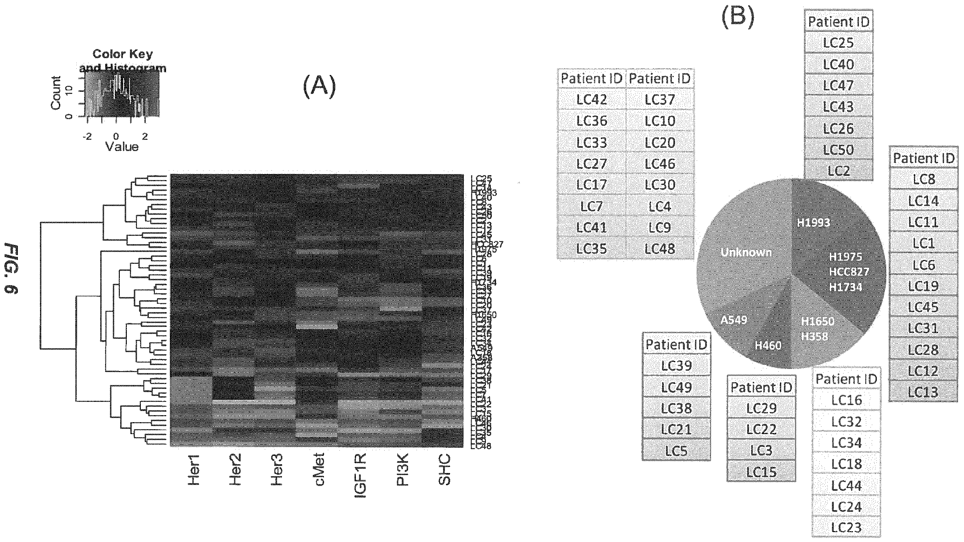

**Figure 6****.** (A) Heat Map representing the activated signaling pathways found in the 50 lung tumor tissue samples and eight lung tumor cell lines. Each row constitutes all the pathway markers determined from an individual tumor sample organized in columns. The legend denotes markers that are present at lower and higher levels. (B) Clustering of the 50 lung tumor samples with the corresponding lung tumor cell lines based on the similarities in the markers between the tissue samples and the cell lines.

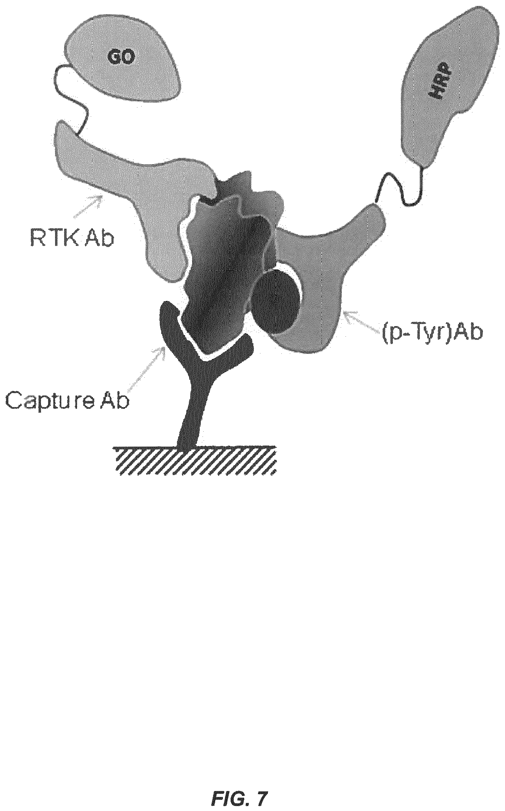

**Figure 7****.** Illustration of the CEER™ assay principle. The assay is based on the formation of an immuno-complex that requires the co-localization of two detecting antibodies against the captured target protein shown in black.

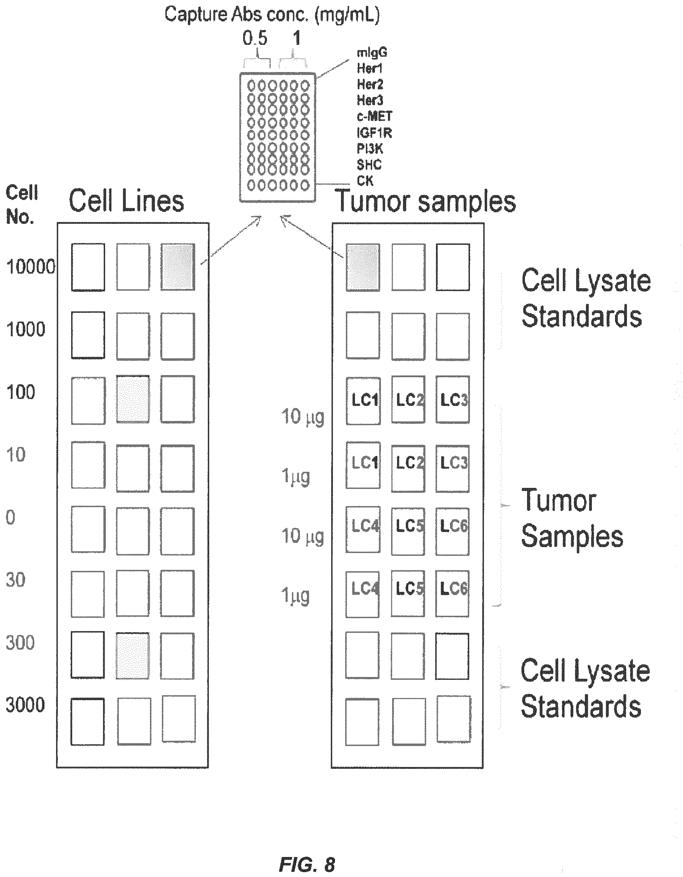

**Figure 8****.** Illustration of the CEER™ assay protocol for determination of the activated signaling pathways in lung tumor cell lines (left panel) and lung tumor tissue samples and cell lines (right panel).

### DETAILED DESCRIPTION OF THE INVENTION

### I. Introduction

As illustrated in the Example below, clustering of marker profiles obtained from lung tumor tissue samples with known marker profiles obtained from their corresponding cell lines provides insight into targeted therapy based on the anticancer drug treatment results obtained from the tumor cell lines. Notably, pathway profiling of samples collected from lung cancer patients showed similarities in the markers between lung tumor tissue samples and tumor cell lines. Accordingly, the marker profile obtained from a lung cancer sample (*e.g*., cancer cells isolated from lung tumor tissue) is compared to known marker profiles obtained from one or more lung cancer cell lines, and similarities in the expression level, activation (*e.g*., phosphorylation) level, and/or genotype of the same marker(s) in both the tumor tissue sample and the lung cancer cell line indicate that the lung tumor is predicted to respond to the same anticancer drug or combinations thereof that inhibited proliferation of the lung cancer cell line. As such, the present disclosure finds utility in predicting therapeutic efficacy or response to one or a cocktail of anticancer drugs by analyzing one or a panel of pathway biomarkers and/or mutated genes to discriminate and cluster different lung tumor tissue samples with the corresponding tumor cell lines to guide treatment options for the subject having lung cancer based on the drug sensitivity of the tumor cell lines.

### II. Definitions

As used herein, the following terms have the meanings ascribed to them unless specified otherwise.

The term "cancer" is intended to include any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, lung cancer (*e.g*., non-small cell lung cancer); digestive and gastrointestinal cancers such as colorectal cancer, gastrointestinal stromal tumors, gastrointestinal carcinoid tumors, colon cancer, rectal cancer, anal cancer, bile duct cancer, small intestine cancer, and stomach (gastric) cancer; esophageal cancer; gallbladder cancer; liver cancer; pancreatic cancer; appendix cancer; breast cancer; ovarian cancer; renal cancer (*e.g*., renal cell carcinoma); cancer of the central nervous system; skin cancer; lymphomas; choriocarcinomas; head and neck cancers; osteogenic sarcomas; and blood cancers. As used herein, a "tumor" comprises one or more cancerous cells. In a preferred embodiment, the lung tumor is derived from a subject with a non-small cell lung cancer such as, for example, a squamous cell carcinoma, an adenocarcinoma, a large cell carcinoma, bronchoalveolar carcinoma (BAC), or oat cell carcinoma.

The term "analyte" or "marker" as used herein includes any molecule of interest, typically a macromolecule such as a polypeptide or polynucleotide, whose presence, amount (expression level), activation state or level, genotype (*e.g*., mutation status), and/or identity is determined. In certain instances, the analyte or marker is a signal transduction molecule. In certain instances, the analyte or marker is a gene such as an oncogene or a tumor suppressor gene.

The term "signal transduction molecule" or "signal transducer" includes proteins and other molecules that carry out the process by which a cell converts an extracellular signal or stimulus into a response, typically involving ordered sequences of biochemical reactions inside the cell. Examples of signal transduction molecules include, but are not limited to, receptor tyrosine kinases such as EGFR (*e.g*., EGFR/HER1/ErbB1, HER2/Neu/ErbB2, HER3/ErbB3, HER4/ErbB4), VEGFR1/FLT1, VEGFR2/FLK1/KDR, VEGFR3/FLT4, FLT3/FLK2, PDGFR (*e.g*., PDGFRA, PDGFRB), c-KIT/SCFR, INSR (insulin receptor), IGF-IR, IGF-IIR, IRR (insulin receptor-related receptor), CSF-1R, FGFR 1-4, HGFR 1-2, CCK4, TRK A-C, c-MET, RON, EPHA 1-8, EPHB 1-6, AXL, MER, TYRO3, TIE 1-2, TEK, RYK, DDR 1-2, RET, c-ROS, V-cadherin, LTK (leukocyte tyrosine kinase), ALK (anaplastic lymphoma kinase), ROR 1-2, MUSK, AATYK 1-3, and RTK 106; truncated forms of receptor tyrosine kinases such as truncated HER2 receptors with missing amino-terminal extracellular domains (*e.g*., p95ErbB2 (p95m), p110, p95c, p95n, *etc*.); receptor tyrosine kinase dimers (*e.g*., p95HER2/HER3, p95HER2/HER2, HER2/HER2, HER2/HER3, HER1/HER2, HER2/HER3, HER2/HER4, *etc*.); non-receptor tyrosine kinases such as BCR-ABL, Src, Frk, Btk, Csk, Abl, Zap70, Fes/Fps, Fak, Jak, Ack, and LIMK; tyrosine kinase signaling cascade components such as AKT (*e.g*., AKT1, AKT2, AKT3), MEK (MAP2K1), ERK2 (MAPK1), ERK1 (MAPK3), PI3K (*e.g*., PIK3CA (p110), PIK3R1 (p85)), PDK1, PDK2, phosphatase and tensin homolog (PTEN), SGK3, 4E-BP1, P70S6K (*e.g*., p70 S6 kinase splice variant alpha I), protein tyrosine phosphatases (*e.g*., PTP1B, PTPN13, BDP1, *etc*.), RAF, PLA2, MEKK, JNKK, JNK, p38, Shc (p66), Ras (*e.g*., K-Ras, N-Ras, H-Ras), Rho, Rac1, Cdc42, PLC, PKC, p53, cyclin D1, STAT1, STAT3, phosphatidylinositol 4,5-bisphosphate (PIP2), phosphatidylinositol 3,4,5-trisphosphate (PIP3), mTOR, BAD, p21, p27, ROCK, IP3, TSP-1, NOS, GSK-3β, RSK 1-3, JNK, c-Jun, Rb, CREB, Ki67, and paxillin; nuclear hormone receptors such as estrogen receptor (ER), progesterone receptor (PR), androgen receptor, glucocorticoid receptor, mineralocorticoid receptor, vitamin A receptor, vitamin D receptor, retinoid receptor, thyroid hormone receptor, and orphan receptors; nuclear receptor coactivators and repressors such as amplified in breast cancer-1 (AIB1) and nuclear receptor corepressor 1 (NCOR), respectively; and combinations thereof.

The term "component of a HER2 signaling pathway" includes any one or more of an upstream ligand of HER2, binding partner of HER2, and/or downstream effector molecule that is modulated through HER2. Examples of HER2 signaling pathway components include, but are not limited to, heregulin, HER1/ErbB1, HER2/ ErbB2, HER3/ErbB3, HER4/ErbB4, AKT (*e.g*., AKT1, AKT2, AKT3), MEK (MAP2K1), ERK2 (MAPK1), ERK1 (MAPK3), PI3K (*e.g*., PIK3CA (p110), PIK3R1 (p85)), PDK1, PDK2, PTEN, SGK3, 4E-BP1, P70S6K (*e.g*., splice variant alpha I), protein tyrosine phosphatases (*e.g*., PTP1B, PTPN13, BDP1, *etc*.), HER2 dimers (*e.g*., p95HER2/HER3, p95HER2/HER2, HER2/HER2, HER2/HER3, HER1/HER2, HER2/HER3, HER2/HER4, *etc*.), GSK-3β, PIP2, PIP3, p27, and combinations thereof.

The term "component of a c-Met signaling pathway" includes any one or more of an upstream ligand of c-Met, binding partner of c-Met, and/or downstream effector molecule that is modulated through c-Met. Examples of c-Met signaling pathway components include, but are not limited to, hepatocyte growth factor/scatter factor (HGF/SF), Plexin B1, CD44v6, AKT (*e.g*., AKT1, AKT2, AKT3), MEK (MAP2K1), ERK2 (MAPK1), ERK1 (MAPK3), STAT (*e.g*., STAT1, STAT3), PI3K (*e.g*., PIK3CA (p110), PIK3R1 (p85)), GRB2, Shc (p66), Ras (*e.g*., K-Ras, N-Ras, H-Ras), GAB1, SHP2, SRC, GRB2, CRKL, PLCγ, PKC (*e.g*., PKCα, PKCβ, PKCδ), paxillin, FAK, adducin, RB, RB1, PYK2, and combinations thereof.

The term "activation state" refers to whether a particular signal transduction molecule such as a HER2 or c-Met signaling pathway component is activated. Similarly, the term "activation level" refers to what extent a particular signal transduction molecule such as a HER2 or c-Met signaling pathway component is activated. The activation state typically corresponds to the phosphorylation, ubiquitination, and/or complexation status of one or more signal transduction molecules. Non-limiting examples of activation states (listed in parentheses) include: HER1/EGFR (EGFRvIII, phosphorylated (p-) EGFR, EGFR:Shc, ubiquitinated (u-) EGFR, p-EGFRvIII); ErbB2 (p-ErbB2, p95HER2 (truncated ErbB2), p-p95HER2, ErbB2:Shc, ErbB2:PI3K, ErbB2:EGFR, ErbB2:ErbB3, ErbB2:ErbB4); ErbB3 (p-ErbB3, ErbB3:PI3K, p-ErbB3:PI3K, ErbB3:Shc); ErbB4 (p-ErbB4, ErbB4:Shc); c-MET (p-c-MET, c-Met:HGF complex); AKT1 (p-AKT1); AKT2 (p-AKT2); AKT3 (p-AKT3); PTEN (p-PTEN); P70S6K (p-P70S6K); MEK (p-MEK); ERK1 (p-ERK1); ERK2 (p-ERK2); PDK1 (p-PDK1); PDK2 (p-PDK2); SGK3 (p-SGK3); 4E-BP1 (p-4E-BP1); PIK3R1 (p-PIK3R1); c-KIT (p-c-KIT); ER (p-ER); IGF-1R (p-IGF-1R, IGF-1R:IRS, IRS:PI3K, p-IRS, IGF-1R:PI3K); INSR (p-INSR); FLT3 (p-FLT3); HGFR1 (p-HGFR1); HGFR2 (p-HGFR2); RET (p-RET); PDGFRA (p-PDGFRA); PDGFRB (p-PDGFRB); VEGFR1 (p-VEGFR1, VEGFR1:PLCγ, VEGFR1:Src); VEGFR2 (p-VEGFR2, VEGFR2:PLCy, VEGFR2:Src, VEGFR2:heparin sulphate, VEGFR2:VE-cadherin); VEGFR3 (p-VEGFR3); FGFR1 (p-FGFR1); FGFR2 (p-FGFR2); FGFR3 (p-FGFR3); FGFR4 (p-FGFR4); TIE1 (p-TIE1); TIE2 (p-TIE2); EPHA (p-EPHA); EPHB (p-EPHB); GSK-3β (p-GSK-3β); NFKB (p-NFKB), IKB (p-IKB, p-P65:IKB); BAD (p-BAD, BAD:14-3-3); mTOR (p-mTOR); Rsk-1 (p-Rsk-1); Jnk (p-Jnk); P38 (p-P38); STAT1 (p-STAT1); STAT3 (p-STAT3); FAK (p-FAK); RB (p-RB); Ki67; p53 (p-p53); CREB (p-CREB); c-Jun (p-c-Jun); c-Src (p-c-Src); paxillin (p-paxillin); GRB2 (p-GRB2), Shc (p-Shc), Ras (p-Ras), GAB1 (p-GAB1), SHP2 (p-SHP2), GRB2 (p-GRB2), CRKL (p-CRKL), PLCγ (p-PLCγ), PKC (*e.g*., p-PKCα, p-PKCβ, p-PKCδ), adducin (p-adducin), RB1 (p-RB1), and PYK2 (p-PYK2).

The term "oncogene" includes a gene that has the potential to cause cancer. Non-limiting examples of oncogenes include growth factors or mitogens such as c-Sis; receptor tyrosine kinases such as EGFR, HER2, PDGFR, and VEGFR; cytoplasmic tyrosine kinases such as Abl and kinases in the Src-family, Syk-ZAP-70 family, and BTK family of tyrosine kinases; cytoplasmic serine/threonine kinases and their regulatory subunits such as PIK3CA, PIK3R1, and RAF (*e.g*., RAF-1, A-RAF, B-RAF); regulatory GTPases such as RAS (*e.g*., KRAS); transcription factors such as MYC; and combinations thereof.

The term "tumor suppressor gene" includes a gene that has the potential to protect a cell from becoming a cancerous cell. Non-limiting examples of tumor suppressor genes include the TP53 gene (also known as the P53 gene), which encodes p53 (also known as protein 53 or tumor protein 53); kinases such as, *e.g*., tyrosine kinases or serine/threonine kinases including serine/threonine kinase 11 (STK11); the RB1 gene, which encodes the Retinoblastoma protein (pRb); PTEN; VHL; APC; CD95; ST5; YPEL3; ST7; ST14; and combinations thereof.

The term "KRAS mutation" includes any one or more mutations in the KRAS (which can also be referred to as KRAS2 or RASK2) gene. Examples of KRAS mutations include, but are not limited to, G35A (G12D), G35T (G12V), G34T (G12C), G34A (G12S), G35C (G12A), G34C (G12R), G38A (G13D), G37T (G13C), C181A (Q61K), A183T (Q61H), A183C (Q61H), A182G (Q61R), and combinations thereof.

The term "P53 mutation" includes any one or more mutations in the P53 (which can also be referred to as TP53) gene. Examples of P53 mutations include, but are not limited to, C726G (C242W), G818T (R273L), and combinations thereof. Additional P53 mutations known in the art result in protein variants including, but not limited to, Q5H, S6L, D7H, P8S, V10I, E11K/Q, S15R, Q16L, E17D, K24N, E28A, V31I, S33T, P34L, L35F, P36L, S37P/T, A39P/V, D42Y, L43S, M44I/T/V, L45M, S46F/P, P47L/S, D48G, D49H/N/Y, Q52H, W53C/G, F54L/Y, E56K/V. P58Q/T, G59C/D/N/, P60L/Q/S, D61G/N, E62D, A63T/V, R65T,M66I/R, P67L/R/S, E68G/Q, A69D/G/T/V, A70T, P71T, P72C/G/H/L/R, V73E/L/M, A74T, P75L/R/S, A76G/T, P77A, A78V, A79G/T/V, P80L/S, T81I, P82L/S, A83E/V, A84G/V, P85L/S, A86V, P87Q, A88T/V/L, P89L/S, S90F/Y, W91C, P92A/L/S, L93M/P, S94L/T, S95F/T, S96C/F/P, V97A/F/I, P98L/S/F/P, Q100R, K101N/R, T102I, Q104H/L, G105C/D/R/S/V, S106G/R, Y107C/D/H, G108D/S, F109C/L/S, R110C/G/H/L/P/S, L111M/P/Q/R, g112D/S, F113C/G/I/L/S/V, H115Y, S116C/F/P/, G117E/R, T118A/I/R, A118D/T, K120E/M/Q/R, S121F, V122L, T123I/N, C124G/R/S/W/Y, T125A/K/M/P/R, Y126C/D/F/G/H/N/S. S127C/F/P/T/Y, P128A/L/R, A129D/G/T/V, L130F/H/I/P/R/V, N131D/H/I/K/S/T/Y, K132E/M/N/Q/R/T/W, M133I/K/L/R/T/V, F134C/I//L/S/V, C135F/G/R/S/T/W/Y, Q136E/H/K/P/R, L1137M/P/Q/V/A138D/P/S/T/V, K139E/N/Q/R/T, T140A/I/N/P/S, C141A/F/G/R/S/W/Y, AND P142A/F/H/L/R/S/T, V143A/E/G/L/M, Q144H/K/L/P/R, L145M/P/Q/R/V, W146C/G/L/R/S, V147A/D/E/F/G/I, D148A/E/G/N/V/Y, S149F/P/T, T150A/I/K/N/P/R, P151H/L/R/S/T/, P152A/L/Q/R/S/T. P153A/F/H/L/R/S/T, G154A/C/D/I/S/V, T155A/I/M/N/P/S, R156R/C/G/H/L/P/S, V157A/D/F/G/I/L, R158C/F/G/H/K/P/W/S, R159D/F/G/P/S/T/V, M160I/K/T/V, A161D/F/G/P/S/T/V, I162F/M/N/S/T/V, Y163C/D/F/H/N/S, K164E/M/N/Q/R/T, Q165E/H/L/P/R, S166A/G/L/P, Q167H/K/L/R, H168D/L/N/P/Q/R/V/Y, M169I/K/T/V/, T170A/K/M/P/S, E171A/D/G/K/Q/V, V172A/D/F/G/I, V173A/E/G/L/M/W, R174G/K/M/S/T/W, R175C/G/H/L/P/Q/S, C176F/G/R/S/W/Y, P177A/F/H/I/L/R/S/T, H178D/L/N/P/Q/R/Y, H179L/N/P/Q/R/Y, E180A/D/G/K/Q/V, R181C/G/H/L/P/S, C182R/S/Y, S183Y/L/P, D184G/H/N/V/Y, S185G/I/N/R/T, D186E/G/H/N/V/Y, G187C/D/N/R/S/V, L188P/V, A189D/G/P/S/T/V, P190A/H/L/R/S/T, P191H/L/R/S/T, Q192H/K/L/P/R, H193D/L/N/P/Q/R/Y, L194F/H/I/P/R/V, I195F/L/N/S/T/V/Y, R196G/L/P/Q/S, V197E/G/L/M, E198D/G/K/Q/V, G199A/E/R/V, N200D/I/K/P/S/T, L201F/P/S, R202C/G/H/L/P/S, V203A/E/L/M/W, E204A/D/G/K/Q/V, Y205C/D/F/H/N/S, L206F/M, D207E/G/H/N/V/Y, D208E/G/H/I/N/V/Y, R209I/K/S/T, N210D/H/I/K/S/T/Y, T211A/I/N/P/S, F212I/L/S/V/Q, R213G/L/P/Q/W, H214D/P/Q/R/Y, S215C/G/I/K/N/R/T, V216A/E/G/L/M/W, V416W, V217A/E/G/I/L/M, V218A/E/G/L/M, P219C/H/L/R/S/T, Y220C/D/F/H/N/S, E221A/D/G/K/Q, P222A/L/Q/R/S/T, P223A/H/L/R/S/T, E224D/G/K/V, V225A/D/F/G/I/L. G226A/D/N/S/V, S227C/F/P/T, D228A/E/G/H/N/P/V/Y, C229G/N/R/S/Y, T230A/I/N/P/S, T231A/I/N/S, I232F/L/N/S/T/V, H233D/L/P/Q/R/Y, Y234C/D/F/H/K/N,Q/S, N235D/H/I/M/S/T/Y, Y236C/D/F/H/N/S, M237I/K/L/R/T/V, C238F/G/H/R/S/W/Y, N239D/H/I/K/S/T/Y, S240C/G/I/N/P/R/T, S241A/C/F/P/T/Y, C242F/G/R/S/W/Y, M243I/K/L/R/T/V, G244A/C/D/E/R/S/V, G245A/C/D/E/F/H/L/N/R/S/V, M246I/K/L/R/T/V, N247D/F/I/K/S/T/Y, R248C/G/L/P/Q/W, R249G/I/K/M/N/S/T/W, P250A/F/H/L/N/Q/S/T, I251F/L/M/N/S/T/V, L252F/H/I/P/V, T253A/I/N/P/S, I254D/F/L/M/N/S/T/V, I255F/M/N/S/T/V, T256K/P/S, L257P/Q/R/V, E258A/D/G/K/L/Q/V, D259A/E/G/H/N/P/S/V/Y, S260A/C/F/P/T/Y, S261C/G/I/N/R, G262C/D/H/S/V, N263D/H/I/K/S, L264I/P/Q/R/V, L265M/P/Q/R, G266A/E/R/V, R267G/H/P/Q/W, N268F/H/I/K/S/Y, S269C/G/I/N/R/T, F270C/I/L/S/V/Y, E271A/D/G/K/P/Q/R/V, V272A/E/G/L/M, R273C/G/H/L/N/P/Q/S/Y, C274A/D/F/G/I/L, C275F/G/R/S/W/Y, A276D/G/P/S/T/V, C277F/G/R/S/W/Y, P278A/F/H/L/R/S/T, G279E/R/V/W, R280K/P/S/T, D281A/E/G/H/N/R/V/Y, R282H/L/P/Q/W, R283C/G/H/L/P/S, T284A/I/K/P, E285A/D/G/K/Q/V, E286A/D/G/K/L/Q/V, E287A/D/G/K/V, N288K/S/T/Y/, L289F/H/PR/V, R290C/H/L, K291E/M/N/Q/R/T, K292E/G/I/N/Q/R/T, G293A/R/V/W, E294A/D/G/K/Q/V, P295H/L/R/S, H296C/D/L/N/P/Q/R/Y, H297D/N/P/R/Q, E298A/D/K/Q/V, L299P/Q/R/V, P300A/L/R/S, P301A/L/Q/S/T, G302A/E/R/V, S303C/I/N/T, T304A/I/N/S/, K305E/M/N/R/T, R306P/Q, A307P/S/T, L308M/V, P309R/S, N310I/T, N311H/K/S/T, T312I/S/, S313C/I/N/R, S315C/F/P, P316L/T, Q317H/KL/P/R, P318L, L319E/N/R, K320N, K312E/R, P322L/R, L323G/M/P/R/V, D324E/S/Y, G325A/E/V, E326G, Y327H/S, F328L/S/V, T329I/S, L330H/P/R, Q331H/P/R, I332V, G334V/W, R335G/H/L, R337C/H/L/P, F338I/L, E339K/Q, F341C, R342L/P/Q, E343G, L344P/R, E346A, A3347G/T. L348F/S, E349D, D352H, A353T, Q354E/K/R, G356A/W, E358D/K, G360A/V, R363K, A364P/T/V, H365R/Y, S366A, K370Q, S376A/T, R379H, F385L, G389W, S392L, KM132-133ML, MA160-161IP/IS/IT, QH167-168HD/YL, HM168-169LI, MT169-170IS, HH-179QS, QH192-193HN/HY, LR201-202FC, VE203-204LV, EE207-208EY, MG243-244IC/IS, NR247-248IP/KW, RP249-250SA/SS, GN262-263PD, DR281-282EW, and combinations thereof.

The term "STK11 mutation" includes any one or more mutations in the STK11 (which can also be referred to as LKB1) gene. Examples of STK11 mutations include, but are not limited to, C109T (Q37Ter), G595T (E199Ter), C108A (Y36Ter), T145G (Y49D), G169T (E57Ter), T200C (L67P), A250T (K84Ter), G290+36T, G403C (G135R), G488A (G163D), C508T (Q170Ter), G580A (D194N), G580T (D194Y), A581T (D194V), G595A (E199K), G717C (W239C), C738G (Y246Ter), C759A(Y253Ter), C842T (P281L), G996A (W332Ter), C1062G (F354L), G169del (E57K frameshift), TTGT787-790del (L263-F264 frameshift), C842del (P281R frameshift), a kinase domain mutation, and combinations thereof.

The term "BRAF mutation" includes any one or more mutations in the BRAF (which can also be referred to as serine/threonine-protein kinase B-Raf or B-Raf) gene. Examples of BRAF mutations include, but are not limited to, V600E, R461I, I462S, G463E, G463V, G465A, G465E, G465V, G468A, G468E, N580S, E585K, D593V, F594L, G595R, L596V, T598I, V599D, V599E, V599K, V599R, K600E, A727V, and combinations thereof.

The term "PIK3CA mutation" includes any one or more mutations in the PIK3CA (which can also be referred to as PI3K or p110-alpha) gene. Examples of PIK3CA mutations include, but are not limited to, E545A, E545G, E545K, Q546E, Q546K, H1047R, H1047L, 3204insA, and combinations thereof.

The term "EGFR mutation" includes any one or more mutations in the EGFR (which can also be referred to as HER1 or ErbB1) gene. Examples of EGFR mutations include, but are not limited to, deletions in exon 19 such as L858R, G719S, G719S, G719C, L861Q and S7681, as well as insertions in exon 20 such as T790M, and combinations thereof.

As used herein, the term "dilution series" is intended to include a series of descending concentrations of a particular sample (*e.g*., cell lysate) or reagent (*e.g*., antibody). A dilution series is typically produced by a process of mixing a measured amount of a starting concentration of a sample or reagent with a diluent (*e.g*., dilution buffer) to create a lower concentration of the sample or reagent, and repeating the process enough times to obtain the desired number of serial dilutions. The sample or reagent can be serially diluted at least 2, 3, 4, 5, 6, 7, 8, 9, 10, 15, 20, 25, 30, 35, 40, 45, 50, 100, 500, or 1000-fold to produce a dilution series comprising at least 2, 3, 4, 5, 6, 7, 8, 9, 10, 11, 12, 13, 14, 15, 16, 17, 18, 19, 20, 25, 30, 35, 40, 45, or 50 descending concentrations of the sample or reagent. For example, a dilution series comprising a 2-fold serial dilution of a capture antibody reagent at a 1 mg/ml starting concentration can be produced by mixing an amount of the starting concentration of capture antibody with an equal amount of a dilution buffer to create a 0.5 mg/ml concentration of the capture antibody, and repeating the process to obtain capture antibody concentrations of 0.25 mg/ml, 0.125 mg/ml, 0.0625 mg/ml, 0.0325 mg/ml, *etc.*

The term "superior dynamic range" as used herein refers to the ability of an assay to detect a specific analyte in as few as one cell or in as many as thousands of cells. For example, the immunoassays described herein possess superior dynamic range because they advantageously detect a particular signal transduction molecule of interest in about 1-10,000 cells (*e.g*., about 1, 5, 10, 25, 50, 75, 100, 250, 500, 750, 1000, 2500, 5000, 7500, or 10,000 cells) using a dilution series of capture antibody concentrations.

The term "sample" as used herein includes any biological specimen obtained from a patient. Samples include, without limitation, whole blood, plasma, serum, red blood cells, white blood cells (*e.g*., peripheral blood mononuclear cells), ductal lavage fluid, ascites, pleural efflux, nipple aspirate, lymph (*e.g*., disseminated tumor cells of the lymph node), bone marrow aspirate, saliva, urine, stool (*i.e*., feces), sputum, bronchial lavage fluid, tears, fine needle aspirate (*e.g*., harvested by random periareolar fine needle aspiration), any other bodily fluid, a tissue sample (*e.g*., tumor tissue) such as a biopsy of a tumor *(e.g.,* needle biopsy) or a lymph node (*e.g*., sentinel lymph node biopsy), a tissue sample (*e.g*., tumor tissue) such as a surgical resection of a tumor, and cellular extracts thereof. In some aspects, the sample is whole blood or a fractional component thereof such as plasma, serum, or a cell pellet. In other aspects, the sample is obtained by isolating circulating cells of a solid tumor from whole blood or a cellular fraction thereof using any technique known in the art. In yet other aspects, the sample is a formalin fixed paraffin embedded (FFPE) tumor tissue sample, *e.g*., from a solid tumor such as lung cancer. In particular aspects, the sample is a tumor lysate or extract prepared from frozen tissue obtained from a subject having lung cancer.

A "biopsy" refers to the process of removing a tissue sample for diagnostic or prognostic evaluation, and to the tissue specimen itself. Any biopsy technique known in the art can be applied to the methods and compositions of the present invention. The biopsy technique applied will generally depend on the tissue type to be evaluated and the size and type of the tumor (*i.e*., solid or suspended (*i.e*., blood or ascites)), among other factors. Representative biopsy techniques include excisional biopsy, incisional biopsy, needle biopsy (*e.g*., core needle biopsy, fine-needle aspiration biopsy, *etc*.), surgical biopsy, and bone marrow biopsy. Biopsy techniques are discussed, for example, in Harrison's Principles of Internal Medicine, Kasper, et al., eds., 16th ed., 2005, Chapter 70, and throughout Part V. One skilled in the art will appreciate that biopsy techniques can be performed to identify cancerous and/or precancerous cells in a given tissue sample.

The term "subject" or "patient" or "individual" typically includes humans, but can also include other animals such as, *e.g*., other primates, rodents, canines, felines, equines, ovines, porcines, and the like.

An "array" or "microarray" comprises a distinct set and/or dilution series of capture antibodies immobilized or restrained on a solid support such as, for example, glass (*e.g*., a glass slide), plastic, chips, pins, filters, beads (*e.g*., magnetic beads, polystyrene beads, *etc*.), paper, membrane (*e.g*., nylon, nitrocellulose, polyvinylidene fluoride (PVDF), *etc*.), fiber bundles, or any other suitable substrate. The capture antibodies are generally immobilized or restrained on the solid support via covalent or noncovalent interactions (*e.g*., ionic bonds, hydrophobic interactions, hydrogen bonds, Van der Waals forces, dipole-dipole bonds). In certain instances, the capture antibodies comprise capture tags which interact with capture agents bound to the solid support. The arrays used in the assays described herein typically comprise a plurality of different capture antibodies and/or capture antibody concentrations that are coupled to the surface of a solid support in different known/addressable locations.

The term "capture antibody" is intended to include an immobilized antibody which is specific for (*i.e*., binds, is bound by, or forms a complex with) one or more analytes of interest in a sample such as a cellular extract. In particular aspects, the capture antibody is restrained on a solid support in an array. Suitable capture antibodies for immobilizing any of a variety of signal transduction molecules on a solid support are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA).

The term "detection antibody" as used herein includes an antibody comprising a detectable label which is specific for (*i.e*., binds, is bound by, or forms a complex with) one or more analytes of interest in a sample. The term also encompasses an antibody which is specific for one or more analytes of interest, wherein the antibody can be bound by another species that comprises a detectable label. Examples of detectable labels include, but are not limited to, biotin/streptavidin labels, nucleic acid (*e.g*., oligonucleotide) labels, chemically reactive labels, fluorescent labels, enzyme labels, radioactive labels, and combinations thereof. Suitable detection antibodies for detecting the activation state and/or total amount of any of a variety of signal transduction molecules are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA). As a non-limiting example, phospho-specific antibodies against various phosphorylated forms of signal transduction molecules such as EGFR, c-KIT, c-Src, FLK-1, PDGFRA, PDGFRB, AKT, MAPK, PTEN, Raf, and MEK are available from Santa Cruz Biotechnology.

The term "activation state-dependent antibody" includes a detection antibody which is specific for (*i.e*., binds, is bound by, or forms a complex with) a particular activation state of one or more analytes of interest in a sample. In preferred aspects, the activation state-dependent antibody detects the phosphorylation, ubiquitination, and/or complexation state of one or more analytes such as one or more signal transduction molecules. In some aspects, the phosphorylation of members of the EGFR family of receptor tyrosine kinases and/or the formation of heterodimeric complexes between EGFR family members is detected using activation state-dependent antibodies. In particular aspects, activation state-dependent antibodies are useful for detecting one or more sites of phosphorylation in one or more of the following signal transduction molecules (phosphorylation sites correspond to the position of the amino acid in the human protein sequence): EGFR/HER1/ErbB1 (*e.g*., tyrosine (Y) 1068); ErbB2/HER2 (*e.g*., Y1248); ErbB3/HER3 (*e.g*., Y1289); ErbB4/HER4 (*e.g*., Y1284); c-Met (*e.g*., Y1003, Y1230, Y1234, Y1235, and/or Y1349); SGK3 *(e.g.,* threonine (T) 256 and/or serine (S) 422); 4E-BP1 (*e.g*., T70); ERK1 (*e.g*., T185, Y187, T202, and/or Y204); ERK2 (*e.g*., T185, Y187, T202, and/or Y204); MEK *(e.g.,* S217 and/or S221); PIK3R1 (*e.g*., Y688); PDK1 (*e.g*., S241); P70S6K (*e.g*., T229, T389, and/or S421); PTEN (*e.g*., S380); AKT1 (*e.g*., S473 and/or T308); AKT2 (*e.g*., S474 and/or T309); AKT3 (*e.g*., S472 and/or T305); GSK-3β (*e.g*., S9); NFKB (*e.g*., S536); IKB (*e.g*., S32); BAD (*e.g*., S112 and/or S136); mTOR (*e.g*., S2448); Rsk-1 (*e.g*., T357 and/or S363); Jnk (*e.g*., T183 and/or Y185); P38 (*e.g.,* T180 and/or Y182); STAT3 (*e.g*., Y705 and/or S727); FAK (*e.g.,* Y397, Y576, S722, Y861, and/or S910); RB (*e.g.*, S249, T252, S612, and/or S780); RB1 (*e.g.,* S780); adducin (*e.g*., S662 and/or S724); PYK2 (*e.g*., Y402 and/or Y881); PKCα (*e.g*., S657); PKCα/β (*e.g*., T368 and/or T641); PKCδ (*e.g*., T505); p53 (*e.g*., S392 and/or S20); CREB (*e.g*., S133); c-Jun (*e.g*., S63); c-Src (*e.g*., Y416); and paxillin (*e.g*., Y31 and/or Y118).

The term "activation state-independent antibody" includes a detection antibody which is specific for (*i.e*., binds, is bound by, or forms a complex with) one or more analytes of interest in a sample irrespective of their activation state. For example, the activation state-independent antibody can detect both phosphorylated and unphosphorylated forms of one or more analytes such as one or more signal transduction molecules.

The term "incubating" is used synonymously with "contacting" and "exposing" and does not imply any specific time or temperature requirements unless otherwise indicated.

"Receptor tyrosine kinases" or "RTKs" include a family of fifty-six (56) proteins characterized by a transmembrane domain and a tyrosine kinase motif. RTKs function in cell signaling and transmit signals regulating growth, differentiation, adhesion, migration, and apoptosis. The mutational activation and/or overexpression of receptor tyrosine kinases transforms cells and often plays a crucial role in the development of cancers. RTKs have become targets of various molecularly targeted agents such as trastuzumab, cetuximab, gefitinib, erlotinib, sunitinib, imatinib, nilotinib, and the like. One well-characterized signal transduction pathway is the MAP kinase pathway, which is responsible for transducing the signal from epidermal growth factor (EGF) to the promotion of cell proliferation in cells.

The term "gene" and variants thereof includes the segment of DNA involved in producing a polypeptide chain; it includes regions preceding and following the coding region, such as the promoter and 3'-untranslated region, respectively, as well as intervening sequences (introns) between individual coding segments (exons).

The term "genotype" and variants thereof refers to the genetic composition of an organism, including, for example, whether a diploid organism is heterozygous or homozygous for one or more variant alleles of interest.

The term "polymorphism" and variants thereof refers to the occurrence of two or more genetically determined alternative sequences or alleles in a population. A "polymorphic site" refers to the locus at which divergence occurs. Preferred polymorphic sites have at least two alleles, each occurring at a particular frequency in a population. A polymorphic locus may be as small as one base pair (*e.g*., single nucleotide polymorphism or SNP). Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allele is arbitrarily designated as the reference allele, and other alleles are designated as alternative alleles, "variant alleles," or "variances." The allele occurring most frequently in a selected population can sometimes be referred to as the "wild-type" allele. Diploid organisms may be homozygous or heterozygous for the variant alleles. The variant allele may or may not produce an observable physical or biochemical characteristic ("phenotype") in an individual carrying the variant allele. For example, a variant allele may alter the enzymatic activity of a protein encoded by a gene of interest or in the alternative the variant allele may have no effect on the enzymatic activity of an encoded protein.

The term "single nucleotide polymorphism (SNP)" and variants thereof refers to a change of a single nucleotide within a polynucleotide, including within an allele. This can include the replacement of one nucleotide by another, as well as deletion or insertion of a single nucleotide. Most typically, SNPs are biallelic markers, although tri- and tetra-allelic markers can also exist. By way of non-limiting example, a nucleic acid molecule comprising SNP A\C may include a C or A at the polymorphic position. For combinations of SNPs, the term "haplotype" is used, *e.g*., the genotype of the SNPs in a single DNA strand that are linked to one another. In some aspects, the term "haplotype" can be used to describe a combination of SNP alleles, *e.g.,* the alleles of the SNPs found together on a single DNA molecule. In further aspects, the SNPs in a haplotype can be in linkage disequilibrium with one another.

### III. Description

The present disclosure provides methods for predicting therapeutic efficacy or response to one or a combination of anticancer drugs in a subject having a lung cancer such as non-small cell lung cancer (NSCLC). In particular, the methods of the present disclosure comprise analyzing a sample such as a tumor tissue sample obtained from a subject having lung cancer to determine the presence, expression level, activation level, and/or genotype of one or more markers to obtain a marker profile, and comparing the marker profile with known marker profiles obtained from one or more lung cancer cell lines such as a library of cell lines to thereby predict therapeutic efficacy or response to one or a combination of anticancer drugs. Accordingly, the present disclosure is particularly useful in predicting therapeutic efficacy or response to one or more anticancer drugs by analyzing one or a panel of signal transduction pathway biomarkers and/or mutated genes in tumor tissue obtained from a subject with lung cancer to guide treatment options for the subject based upon similarities in marker profiles obtained from the tumor tissue and lung cancer cell lines and the drug sensitivity of those lung cancer cell lines.

In certain aspects, the present disclosure provides a method for predicting therapeutic efficacy or response to an anticancer drug in a subject having lung cancer, the method comprising:
(a) determining the expression level and/or activation (*e.g*., phosphorylation) level of one or more markers in a cellular extract produced from a cancer cell isolated from the subject to obtain a marker profile (*e.g*., test marker profile);
(b) comparing the marker profile obtained in step (a) with known marker profiles (*e.g*., reference marker profiles) for one or more lung cancer cell lines (*e.g*., to identify similarities and/or differences between the expression level and/or activation level of one or more of the markers in the test and reference marker profiles); and
(c) predicting therapeutic efficacy or response to an anticancer drug based on similarities between the marker profile obtained in step (a) and one or more of the known marker profiles for the one or more lung cancer cell lines.

In some aspects of the disclosure, the similarities between the test and reference marker profiles correspond to similarities in the expression level and/or activation level of one or more of the markers between the marker profile obtained in step (a) and the known marker profiles for the one or more lung cancer cell lines.

The lung cancer is predicted to respond to the same anticancer drug that produces a response in a lung cancer cell line having a similar marker profile (*e.g*., the lung cancer cell line exhibits sensitivity to the anticancer drug). In certain instances, the lung cancer is predicted to respond to the same anticancer drug that suppresses or inhibits the growth (*e.g*., proliferation) of a lung cancer cell line having a similar marker profile as the marker profile obtained in step (a). As a non-limiting example, the response of the lung cancer cell line having a similar marker profile may correspond to suppression or inhibition of the growth of the lung cancer cell line.

The known marker profiles (*e.g*., reference marker profiles) comprise a library of known marker profiles, *e.g.,* a library of known marker profiles for the one or more lung cancer cell lines. The known marker profiles (*e.g*., reference marker profiles) have known therapeutic drugs associated with their efficacies.

The step of predicting therapeutic efficacy or response to an anticancer drug is based on a therapeutic profile. In certain instances, the therapeutic profile comprises a collection or a library of known therapeutic (*e.g*., anticancer) drugs associated with efficacy or response in the one or more lung cancer cell lines.

In certain other aspects, the present disclosure provides a method for selecting a suitable anticancer drug for the treatment of a lung cancer, the method comprising:
(a) determining the expression level and/or activation (*e.g*., phosphorylation) level of one or more markers in a cellular extract produced from a cancer cell isolated from a subject to obtain a marker profile (*e.g*., test marker profile);
(b) comparing the marker profile obtained in step (a) with known marker profiles (*e.g*., reference marker profiles) for one or more lung cancer cell lines (*e.g*., to identify similarities and/or differences between the expression level and/or activation level of one or more of the markers in the test and reference marker profiles); and
(c) selecting a suitable anticancer drug for the treatment of the lung cancer based on similarities between the marker profile obtained in step (a) and one or more of the known marker profiles for the one or more lung cancer cell lines.

In other aspects of the disclosure, the similarities between the test and reference marker profiles correspond to similarities in the expression level and/or activation level of one or more of the markers between the marker profile obtained in step (a) and the known marker profiles for the one or more lung cancer cell lines.

In certain other aspects, the suitable anticancer drug is selected as the same anticancer drug that produces a response in a lung cancer cell line having a similar marker profile (*e.g*., the lung cancer cell line exhibits sensitivity to the anticancer drug). In certain instances, the suitable anticancer drug is selected as the same anticancer drug that suppresses or inhibits the growth (*e.g*., proliferation) of a lung cancer cell line having a similar marker profile as the marker profile obtained in step (a). As a non-limiting example, the response of the lung cancer cell line having a similar marker profile may correspond to suppression or inhibition of the growth of the lung cancer cell line.

In some aspects, the known marker profiles (*e.g*., reference marker profiles) comprise a library of known marker profiles, *e.g*., a library of known marker profiles for the one or more lung cancer cell lines. In certain instances, the known marker profiles (*e.g*., reference marker profiles) have known therapeutic drugs associated with their efficacies.

In some aspects, the step of selecting a suitable anticancer drug is based on a therapeutic profile. In certain instances, the therapeutic profile comprises a collection or a library of known therapeutic (*e.g*., anticancer) drugs associated with efficacy or response in the one or more lung cancer cell lines.

In certain aspects, the expression level and/or activation level of the one or more markers is calibrated against a standard curve generated for each of these markers.

In certain aspects, the cancer cell is isolated from a tumor tissue sample such as a primary lung tumor sample. In particular instances, a cellular extract is produced from a cancer cell isolated from the tumor tissue sample.

In some aspects, the one or more lung cancer cell lines is selected from the group consisting of non-small cell lung cancer (NSCLC) cells lines including HCC827, H1975, H1734, H1993, H358, H1650, A549, H460, HCC827-CR, HCC827-HGF, HCC827-GR, H292, VMRC-LCD, and combinations thereof. In particular embodiments, the one or more lung cancer cell lines is selected from the group consisting of HCC827, H1975, H1734, H1993, H358, H1650, A549, H460, and combinations thereofThe anticancer drug may be selected from the group consisting of HER1 inhibitors, HER1/2 inhibitors, and/or HER1/2/4 inhibitors (*e.g*., erlotinib for HER1, lapatinib for HER1/2, gefitinib for HER1/2/4, and BIBW-2992, an irreversible inhibitor for HER1/2); c-Met inhibitors (*e.g*., PF-2341066); IGF-1R inhibitors (*e.g*., BMS-536924); MEK inhibitors (*e.g*., PD-325901); PI3K and mTOR inhibitors (*e.g*., BEZ-235); and combinations thereof. Additional examples of anticancer drugs are described below.

In one embodiment, the lung cancer cell line is HCC827 and the known marker profile for HCC827 comprises highly activated HER1 and HER2 (*e.g*., high activation levels) and drug sensitivity to HER1/2 inhibitors such as BIBW-2992 or lapatinib, HER1/2/4 inhibitors such as gefitinib, HER1 inhibitors such as erlotinib, or combinations thereof. In certain embodiments, a lung cancer having a similar marker profile as the HCC827 lung cancer cell line (*e.g*., sharing a similar pattern of expression and/or activation levels for the determined markers) is predicted to respond to the same anticancer drug or a combination of anticancer drugs to which the HCC827 lung cancer cell line responds. As such, one or more of the anticancer drugs to which HCC827 exhibits sensitivity can be selected for the treatment of the lung cancer based upon similarities between the test and HCC827 reference marker profiles.

In one embodiment, the lung cancer cell line is H1975 and the known marker profile for H1975 comprises an elevated level of activated HER1 and/or HER2 (*e.g*., high activation levels) and drug sensitivity to HER1/2 inhibitors such as BIBW-2992, HER1 inhibitors such as erlotinib, IGF-1R inhibitors such as BMS-536924, or combinations thereof. In certain instances, the H1975 lung cancer cell line is particularly sensitive to a combination of a HER1/2 inhibitor such as BIBW-2992 with either a MEK inhibitor such as PD-325901 or a PI3K inhibitor such as BEZ-235. In other instances, the H1975 lung cancer cell line is particularly sensitive to a combination of a HER1 inhibitor such as erlotinib and a c-Met inhibitor such as SU11274. In some embodiments, a lung cancer having a similar marker profile as the H1975 lung cancer cell line (*e.g*., sharing a similar pattern of expression and/or activation levels for the determined markers) is predicted to respond to the same anticancer drug or a combination of anticancer drugs to which the H1975 lung cancer cell line responds. As such, one or more of the anticancer drugs to which H1975 exhibits sensitivity can be selected for the treatment of the lung cancer based upon similarities between the test and H1975 reference marker profiles.

In one embodiment, the lung cancer cell line is H1734 and the known marker profile for H1734 comprises an elevated level of activated HER1 and/or HER2 (*e.g*., high activation levels) and drug sensitivity to HER1/2 inhibitors such as lapatinib, HER1 inhibitors such as erlotinib, MEK inhibitors such as PD-325901, cyclic AMP-response element-binding protein (CREB) inhibitors such as Ro-31-8220, or combinations thereof. In some embodiments, a lung cancer having a similar marker profile as the H1734 lung cancer cell line (*e.g*., sharing a similar pattern of expression and/or activation levels for the determined markers) is predicted to respond to the same anticancer drug or a combination of anticancer drugs to which the H1734 lung cancer cell line responds. As such, one or more of the anticancer drugs to which H1734 exhibits sensitivity can be selected for the treatment of the lung cancer based upon similarities between the test and H1734 reference marker profiles.

In one embodiment, the lung cancer cell line is H1993 and the known marker profile for H1993 comprises highly activated c-Met (*e.g*., high activation levels) and drug sensitivity to c-Met inhibitors such as PF-2341066, MEK inhibitors such as PD-325901, or combinations thereof. In certain instances, the H1993 lung cancer cell line is particularly sensitive to a combination of a c-Met inhibitor such as PF-2341066 with either a MEK inhibitor such as PD-325901 or a PI3K inhibitor such as BEZ-235. In certain other instances, the H1993 lung cancer cell line is particularly sensitive to a combination of a MEK inhibitor such as PD-325901 and a PI3K inhibitor such as BEZ-235. In some embodiments, a lung cancer having a similar marker profile as the H1993 lung cancer cell line (*e.g*., sharing a similar pattern of expression and/or activation levels for the determined markers) is predicted to respond to the same anticancer drug or a combination of anticancer drugs to which the H1993 lung cancer cell line responds. As such, one or more of the anticancer drugs to which H1993 exhibits sensitivity can be selected for the treatment of the lung cancer based upon similarities between the test and H1993 reference marker profiles.

In one embodiment, the lung cancer cell line is H358 and the known marker profile for H358 comprises an elevated level of activated HER1 and/or HER2 (*e.g*., high activation levels) and drug sensitivity to HER1/2 inhibitors such as lapatinib, HER1 inhibitors such as erlotinib, HER1/2/4 inhibitors such as gefitinib, IGF-1R inhibitors such as BMS-536924, or combinations thereof. In certain embodiments, the H358 lung cancer cell line is particularly sensitive to a combination of a HER1 inhibitor such as erlotinib with either a MEK inhibitor such as PD-325901 or a PI3K inhibitor such as BEZ-235. In other embodiments, the H358 lung cancer cell line is particularly sensitive to a combination of a MEK inhibitor such as PD-325901 and a PI3K inhibitor such as BEZ-235. In some embodiments, a lung cancer having a similar marker profile as the H358 lung cancer cell line (*e.g*., sharing a similar pattern of expression and/or activation levels for the determined markers) is predicted to respond to the same anticancer drug or a combination of anticancer drugs to which the H358 lung cancer cell line responds. As such, one or more of the anticancer drugs to which H358 exhibits sensitivity can be selected for treatment of the lung cancer based upon similarities between the test and H358 reference marker profiles.

In one embodiment, the lung cancer cell line is H1650 and the known marker profile for H1650 comprises an elevated level of activated HER1 and/or HER2 (*e.g*., high activation levels) and drug sensitivity to HER1/2 inhibitors such as BIBW-2992, IGF-1R inhibitors, or combinations thereof. In certain embodiments, the H1650 lung cancer cell line is particularly sensitive to a combination of a HER1/2 inhibitor such as BIBW-2992 with a PI3K inhibitor such as BEZ-235. In other embodiments, the H1650 lung cancer cell line is particularly sensitive to a combination of a MEK inhibitor such as PD-325901 and a PI3K inhibitor such as BEZ-235. In further embodiments, the H1650 lung cancer cell line is particularly sensitive to a combination of a HER1/2 inhibitor or HER1/2/4 inhibitor such as gefitinib and an IGF-1R inhibitor. In some embodiments, a lung cancer having a similar marker profile as the H1650 lung cancer cell line (*e.g*., sharing a similar pattern of expression and/or activation levels for the determined markers) is predicted to respond to the same anticancer drug or a combination of anticancer drugs to which the H1650 lung cancer cell line responds. As such, one or more of the anticancer drugs to which H1650 exhibits sensitivity can be selected for the treatment of the lung cancer based upon similarities between the test and H1650 reference marker profiles.

In one embodiment, the lung cancer cell line is A549 and the known marker profile for A549 comprises an elevated level of activated IGF-1R (*e.g*., high activation levels) and drug sensitivity to IGF-1R inhibitors such as BMS-536924, MEK inhibitors such as PD-325901, PI3K inhibitors such as BEZ-235, or combinations thereof. In some embodiments, the A549 lung cancer cell line is particularly sensitive to a combination of a radiotherapeutic agent such as ¹³¹I-RC-160 and an anti-metabolite such as 5-fluorocytosine (5-FC). In some instances, a lung cancer having a similar marker profile as the A549 lung cancer cell line (*e.g*., sharing a similar pattern of expression and/or activation levels for the determined markers) is predicted to respond to the same anticancer drug or a combination of anticancer drugs to which the A549 lung cancer cell line responds. As such, one or more of the anticancer drugs to which A549 exhibits sensitivity can be selected for the treatment of the lung cancer based upon similarities between the test and A549 reference marker profiles.

In one embodiment, the lung cancer cell line is H460 and the known marker profile for H460 comprises an elevated level of activated IGF-1R (*e.g*., high activation levels) and drug sensitivity to IGF-1R inhibitors such as BMS-536924, PI3K inhibitors such as BEZ-235, or combinations thereof. In some instances, a lung cancer having a similar marker profile as the H460 lung cancer cell line (*e.g*., sharing a similar pattern of expression and/or activation levels for the determined markers) is predicted to respond to the same anticancer drug or a combination of anticancer drugs to which the H460 lung cancer cell line responds. As such, one or more of the anticancer drugs to which H460 exhibits sensitivity can be selected for the treatment of the lung cancer based upon similarities between the test and H460 reference marker profiles.

The lung cancer cell line HCC827-CR is also disclosed. The known marker profile for HCC827-CR comprises an elevated level of activated AKT (*e.g*., high activation levels) and drug sensitivity to a combination of an inhibitor of PI3K/AKT signaling pathway activity (*e.g*., a PI3K inhibitor) and a HER1/2/4 inhibitor such as gefitinib or a monoclonal antibody such as cetuximab. A lung cancer having a similar marker profile as the HCC827-CR lung cancer cell line (*e.g*., sharing a similar pattern of expression and/or activation levels for the determined markers) is predicted to respond to the same anticancer drug or a combination of anticancer drugs to which the HCC827-CR lung cancer cell line responds. As such, one or more of the anticancer drugs to which HCC827-CR exhibits sensitivity can be selected for the treatment of the lung cancer based upon similarities between the test and HCC827-CR reference marker profiles.

The lung cancer cell line is HCC827-HGF or HCC827-GR is also disclosed. The known marker profile for these cell lines comprises an elevated level of activated c-Met (*e.g*., high activation levels) and drug sensitivity to a combination of a HER1/2/4 inhibitor such as gefitinib and a c-Met inhibitor or an anti-HGF antibody such as TAK-701. A lung cancer having a similar marker profile as one of these lung cancer cell lines (*e.g*., sharing a similar pattern of expression and/or activation levels for the determined markers) is predicted to respond to the same anticancer drug or a combination of anticancer drugs to which the lung cancer cell line responds. As such, one or more of the anticancer drugs to which one of these lung cancer cell lines exhibits sensitivity can be selected for the treatment of the lung cancer based upon similarities between the test and HCC827-HGF or HCC827-GR reference marker profiles.

The lung cancer cell line is H292 is also disclosed. The known marker profile for H292 comprises an elevated level of activated HER1 (*e.g*., high activation levels) and drug sensitivity to a combination of an anti-HER1 monoclonal antibody such as cetuximab and a chemotherapeutic agent such as docetaxel. A lung cancer having a similar marker profile as the H292 lung cancer cell line (*e.g*., sharing a similar pattern of expression and/or activation levels for the determined markers) is predicted to respond to the same anticancer drug or a combination of anticancer drugs to which the H292 lung cancer cell line responds. As such, one or more of the anticancer drugs to which H292 exhibits sensitivity can be selected for the treatment of the lung cancer based upon similarities between the test and H292 reference marker profiles.

In accordance with this disclosure, the expression level and/or activation level of the one or more markers is expressed as a relative fluorescence unit (RFU) value that corresponds to the signal intensity for a particular analyte of interest that is determined using, *e.g*., a proximity assay such as the Collaborative Enzyme Enhanced Reactive Immunoassay (CEER) described herein. In other aspects of the disclosure , the expression level and/or activation level of the one or more markers is expressed as "-", "±", "+", "++", "+++", or "++++" that corresponds to increasing signal intensity for a particular analyte of interest that is determined using, *e.g*., a proximity assay such as CEER. In some instances, an undetectable or minimally detectable level of expression or activation of a particular analyte of interest that is determined using, *e.g.,* a proximity assay such as CEER, may be expressed as "-" or "±". In other instances, a low level of expression or activation of a particular analyte of interest that is determined using, *e.g.,* a proximity assay such as CEER, may be expressed as "+". In yet other instances, a moderate level of expression or activation of a particular analyte of interest that is determined using, *e.g.,* a proximity assay such as CEER, may be expressed as "++". In still yet other instances, a high level of expression or activation of a particular analyte of interest that is determined using, *e.g.,* a proximity assay such as CEER, may be expressed as "+++". In further instances, a very high level of expression or activation of a particular analyte of interest that is determined using, *e.g.,* a proximity assay such as CEER, may be expressed as "++++".

In other aspects of the disclosure, the expression level and/or activation level of the one or more markers is quantitated by calibrating or normalizing the RFU value that is determined using, *e.g.,* a proximity assay such as CEER, against a standard curve generated for a particular analyte of interest. In certain instances, a computed units (CU) value can be calculated based upon the standard curve. In other instances, the CU value can be expressed as "-", "±", "+", "++", "+++", or "++++" in accordance with the description above for signal intensity. Example 3 of US 20120231965 provides a non-limiting example of data analysis for the quantitation of signal transduction pathway proteins in cells such as cancer cells.

In particular aspects of the disclosure, the expression level and/or activation level of a marker of interest in a cellular extract produced from a cancer cell isolated from a subject *(e.g.,* lung tumor tissue) is considered to be "similar" to the expression level and/or activation level of the identical marker in a lung cancer cell line when the level of expression and/or activation of the two markers is at least about 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, or 100% identical to one another.

In certain aspects, the methods of the present invention further comprise a step (a') comprising genotyping nucleic acid obtained from said cancer cell to determine the presence or absence of a variant allele in a gene,
wherein said gene is optionally selected from the group consisting of an oncogene, a tumor suppressor gene, and combinations thereof, or
wherein said oncogene is optionally selected from the group consisting of KRAS, BRAE, PIK3CA, EGER, and combinations thereof, or
wherein said tumor suppressor gene is optionally selected from the group consisting of P53, STK11, and combinations thereof.

Determining the presence or absence of said variant allele in conjunction with determining the phosphorylation level of HER1, HER2, and HER3 may further aid or improve the prediction of therapeutic efficacy or response to an anticancer drug. The allele may comprises a single nucleotide polymorphism (SNP).

In particular aspects, the methods of the present disclosure further comprise the following step:
(a') genotyping for the presence or absence of a variant allele (*e.g*., somatic mutation) at a polymorphic site in a gene such as an oncogene or a tumor suppressor gene (*e.g*., one or more somatic mutations at one, two, three, four, five, or more polymorphic sites such as a single nucleotide polymorphism (SNP) in one or more of these genes) in a cellular extract produced from an isolated cancer cell (*e.g*., an aliquot of the cellular extract used in step (a)).

In other words, the genotyping step (a') comprises analyzing the cellular extract to determine the presence or absence of a variant allele (*e.g*., SNP) in one or more genes such as oncogenes (*e.g*., KRAS) or tumor suppressor genes (*e.g*., P53 and/or STK11). In these aspects, the marker profile obtained in step (a) further comprises the results from the genotyping in step (a'), such that the marker profile comprises the expression level and/or activation level of one or more markers in a cellular extract and the genotype of one or more genes in the cellular extract. In these aspects, the known marker profiles (*e.g*., reference marker profiles) for the one or more lung cancer cell lines in step (b) comprise the expression level, activation level, and/or genotype of the same markers used to generate the marker profile in step (a). In these aspects, the step of predicting therapeutic efficacy or response to an anticancer drug is further based on similarities between the genotypes of one or more genes of interest in the marker profile obtained in step (a) and the marker profiles for the one or more lung cancer cell lines in step (b), *e.g.,* whether or not variant alleles are present or absent at the same polymorphic site in one or more genes of interest. In related aspects, the step of selecting a suitable anticancer drug for treatment of the lung cancer is further based on similarities between the genotypes of one or more genes of interest in the marker profile obtained in step (a) and the marker profiles for the one or more lung cancer cell lines in step (b), *e.g.,* whether or not variant alleles are present or absent at the same polymorphic site in one or more genes of interest.

In particular aspects, the methods of the disclosure comprise determining the presence or absence of a variant allele in conjunction with determining the expression level and/or activation level of one or more markers to further aid and/or improve the prediction of therapeutic efficacy or response to an anticancer drug.

The presence or absence of a variant allele (*e.g*., somatic mutation) in a gene of interest can be determined using an assay described in Section VI below. Assays that can be used to determine somatic mutation or variant allele status include, but are not limited to, electrophoretic analysis, restriction length polymorphism analysis, sequence analysis, hybridization analysis, PCR analysis, allele-specific hybridization, oligonucleotide ligation allele-specific elongation/ligation, allele-specific amplification, single-base extension, molecular inversion probe, invasive cleavage, selective termination, restriction length polymorphism, sequencing, single strand conformation polymorphism (SSCP), single strand chain polymorphism, mismatch-cleaving, denaturing gradient gel electrophoresis, and combinations thereof. These assays have been well-described and standard methods are known in the art. *See, e.g.,* Ausubel et al., Current Protocols in Molecular Biology, John Wiley & Sons, Inc. New York (1984-2008), Chapter 7 and Supplement 47; Theophilus et al., "PCR Mutation Detection Protocols," Humana Press, (2002); Innis et al., PCR Protocols, San Diego, Academic Press, Inc. (1990); Maniatis, et al., Molecular Cloning: A Laboratory Manual, Cold Spring Harbor Lab., New York, (1982); Ausubel et al., Current Protocols in Genetics and Genomics, John Wiley & Sons, Inc. New York (1984-2008); and Ausubel et al., Current Protocols in Human Genetics, John Wiley & Sons, Inc. New York (1984-2008. In particular aspects, the presence or absence of one or more variant alleles (*e.g*., one or more somatic mutations) in one or more genes of interest (*e.g*., oncogenes such as KRAS and/or tumor suppressor genes such as P53 or STK11) is determined using a genotyping assay as described in WO2013/026027, which claims priority from U.S. Provisional Application No. 61/525,137, filed August 18, 2011, and U.S. Provisional Application No. 61/588,151, filed January 18, 2012.

In certain instances, the methods of the present disclosure may further comprise a step of providing the result of the prediction or selection in step (c) to a user (*e.g*., a clinician such as an oncologist or a general practitioner) in a readable format. In some instances, the method may further comprise sending or reporting the result of the prediction or selection in step (c) to a clinician, *e.g*., an oncologist or a general practitioner. In other instances, the method may further comprise recording or storing the result of the prediction or selection in step (c) in a computer database or other suitable machine or device for storing information, *e.g.,* at a laboratory.

To preserve the *in situ* activation states, signal transduction proteins are typically extracted shortly after the cells are isolated, preferably within 96, 72, 48, 24, 6, or 1 hr, more preferably within 30, 15, or 5 minutes. The isolated cells may also be incubated with growth factors usually at nanomolar to micromolar concentrations for about 1-30 minutes to resuscitate or stimulate signal transducer activation (*see, e.g.,* Irish et al., Cell, 118:217-228 (2004)). Stimulatory growth factors include epidermal growth factor (EGF), heregulin (HRG), TGF-α, PIGF, angiopoietin (Ang), NRG1, PGF, TNF-α, VEGF, PDGF, IGF, FGF, HGF, cytokines, and the like. To evaluate potential anticancer therapies for an individual patient, the isolated cells can be incubated with one or more anticancer drugs of varying doses prior to, during, and/or after growth factor stimulation. Growth factor stimulation can be performed for a few minutes or hours (*e.g*., about 1-5 minutes to about 1-6 hours). After isolation, treatment with the anticancer drug, and/or growth factor stimulation, the cells are lysed to extract the signal transduction proteins using any technique known in the art. Preferably, the cell lysis is initiated between about 1-360 minutes after growth factor stimulation, and more preferably at two different time intervals: (1) at about 1-5 minutes after growth factor stimulation; and (2) between about 30-180 minutes after growth factor stimulation. Alternatively, the lysate can be stored at -80°C until use.

In some aspects described herein, the anticancer drug comprises an agent that interferes with the function of activated signal transduction pathway components in cancer cells. Non-limiting examples of such agents include those listed below in Table 1.

**Table 1**

| **EGFR (ErbB1) (A)** | **HER-2 (ErbB2) (C)** | **HER-3 (ErbB3) (E)** | **HER-4 (ErbB4) target** |
|---|---|---|---|
| Cetuximab Panitumumab Matuzumab Nimotuzumab ErbB1 vaccine | Trastuzumab (Herceptin®) Pertuzumab (2C4) BMS-599626* | Antibody (U3) | |
| | *Heterodimerization HER-1/2; Phase 1 | | |
| | | | |

| **EGFR (ErbB1) (B)** | **HER-2 (ErbB2) (D)** | **ErbB1/2 (F)** | **ErbB1/2/4 (G)** |
|---|---|---|---|
| Erlotinib Gefitinib | CP-724714 (Pfizer) | Lapatinib (Tykerb®) HKI-272* | Canertinib* ARRY-334543 |
| EKB 569* | | HKI-357 (Preclinical) | JNJ-26483327 |
| CL-387-785** | | BIBW 2992** | JNJ-26483327 |
| *(Wyeth, Irreversible, II CRC) | | *Wyeth, Irreversible, I/II NSCLC, Breast ** Boehringer | *Pfizer, Irreversible, II |
| **(Wyeth, Irreversible, Preclinical) | | Ingelheim, Irreversible, I/II Prostate, Ovarian, Breast | NSCLC, Breast |
| | | | |

| **Raf (H)** | **SRC (H)** | **Mek: (I)** | **NFkB-IkB (I)** |
|---|---|---|---|
| Sorafenib PLX4032 (Plexxikon) | AZ | PD-325901 (II: NSCLC) AZD6244 - Array/Az XL518 Exelisis/DNA | |
| | | | |

| **mTor (J)** | **PI3K (J)** | **VEGFR2 and VEGFR1 (K)** | **VEGFR1/2/3:** |
|---|---|---|---|
| Rad 001 : Everolimus* | PX-866* | Avastin (DNA) | AZD 2171 (NSCLC, CRC) |
| Temsirolimus** | | HuMV833* | AMG-706 (+ PDGFR) |
| AP-23573*** | | VEGF-Trap** | |
| *Everolimus (Novartis, combination with Gefetinib/Erlotinib; I/II: NSCLC, Glioblastoma) | * P110alpha specific inhibition; | * (PDL) anti-VEGFa | |
| **Temsirolimus (Wyeth, combination with Gefetinib/Erlotinib; I/II: NSCLC, Glioblastoma) ***AP-23573 (Ariad, I/II : Endometrial) | ProlX Pharma; Preclinical NSCLC | **Regeneron/Aventis (Receptor mimic) (Phase 2) | |
| | | | |

| **VEGFR2 target (L)** | | | **EPH A-D** |
|---|---|---|---|
| DC101* | CDP-791 (UCB) | Bay-579352 (+ PDGFR) | |
| IMC-IC11** | CP-547632* | ABT-869* | |
| IMC1121B Fully humanized | AG13736** | BMS-540215 (+FGFR1) | |
| CDP-791*** | E-7080 (Eisai) | KRN-951 | |
| Pazopanib**** | CHIR-258*** | BBIW | |
| | OSI-930 (+ cKit, PDGFR) | | |
| *Imclone (Phase 2/3?) | | *(+CSF1R, Erk, Flt-3, PDGFR) | |
| **Chimeric IgG1 against VEGFR2 | *OSI, PFIZER: (+ ErbB1 + PDGFR) (NSCLC, Ovarian Phase 2) | | |
| ***Celltech, pegalated di-Fab antibody against R2 | **Pfizer: VEGFR1,2 and PDGFRbeta) (RCC II) | | |
| **** GSK, Multiple myeloma, ovarian,RCC Phase 3 enrollment completed, sarcoma II) | ***(VEGFR1,2 FGFR3,PDGFR) | | |

| | | | |
|---|---|---|---|
| | | | |

| **VEGFR 2/ErbB1/2 (ErbB1)/cMet/FGFR (M)** | **VEGFR2/3/Raf/PDGFR/cKit/F It-3 (N)** | **TIE 1/2** | **VEGFR2/1/3, Flt-3, cFMS, PDGFR/cKit (O)** |
|---|---|---|---|
| ZD6474* | Sorafenib* | | PTK787 (Not cFMS, FLT-3) |
| XL647** | | | Sunitinib |
| AEE 788*** | | | XL-999 |
| | | | SU-6668 (Pfizer) |
| | | | GSK |
| | | | AZ (AZD2171) |
| | | | BMS |
| | | | Novartis (AEE-788) |
| | | | Amgen |
| | | | Others |
| *(vandetanib) (Phase III: thyroid, NSCLC) | *(RCC, HCC, NSCLC(III), Melanoma(III)) | | |
| **(Exelixis; Also EPHB2): (Patient resistant to Erlotinib; Asian patients) (Phase 2) | | | |
| ***(Novartis, Phase1/2) | | | |
| | | | |

| **PDGFR target (P)** | **Ab1 target: (Q)** | **FTL 3** | **RET** |
|---|---|---|---|
| Tandutinib Nilotinib | Imatinib Dasatinib Nilotinib AT-9283 AZD-0530 Bosutinib | | |
| | | | |

| **Kit target (R)** | **HGFR1/2** | **FGFR1-4** | **IGF-1 R Target (S)** |
|---|---|---|---|
| AMG-706 XL-880 XL-999 | | Chiron | Merck Pfizer Novartis |
| | | | |

| **HSP90 inhibitors:** | **Anti-Mitotic Drugs:** | **Other targets:** | |
|---|---|---|---|
| IPI-504* | Docetaxel* | HDAC inhibitors | |
| 17-AAG** | Paclitaxel** Vinblastine, Vincristine, | BCL2 | |
| | | Chemotherapeutics (breakdown) | |
| | Vinorelbine*** | | |
| | | Proteosome inhibitors | |
| *(Infinity Pharma, Mutant ErbB1, I/II multiple myeloma, GIST) | *(Microtubule stabilizer; Adjuvant and advanced Breast cancer; NSCLC, Androgen independent Prostate cancer) | | |
| | **(Microtubule stabilizer; Adjuvant and advanced Breast cancer; NSCLC, Ovarian cancer, AIDS related Kaposi sarcoma) | | |
| **(Kosan, I/II solid tumors) | | | |
| | ***(Microtubule De-stabilizers) | | |

In certain aspects described herein, the anticancer drug comprises an anti-signaling agent (*i.e*., a cytostatic drug) such as a monoclonal antibody or a tyrosine kinase inhibitor; an anti-proliferative agent; a chemotherapeutic agent (*i.e*., a cytotoxic drug); a hormonal therapeutic agent; a radiotherapeutic agent; a vaccine; and/or any other compound with the ability to reduce or abrogate the uncontrolled growth of aberrant cells such as cancerous cells. In some aspects, the isolated cells are treated with one or more anti-signaling agents, anti-proliferative agents, and/or hormonal therapeutic agents in combination with at least one chemotherapeutic agent.

Examples of anti-signaling agents include, without limitation, monoclonal antibodies such as trastuzumab (Herceptin®), pertuzumab (2C4), alemtuzumab (Campath®), bevacizumab (Avastin®), cetuximab (Erbitux®), gemtuzumab (Mylotarg®), panitumumab (Vectibix™), rituximab (Rituxan®), and tositumomab (BEXXAR®); tyrosine kinase inhibitors such as gefitinib (Iressa®), sunitinib (Sutent®), erlotinib (Tarceva®), lapatinib (GW-572016; Tykerb®), canertinib (CI 1033), semaxinib (SU5416), vatalanib (PTK787/ZK222584), sorafenib (BAY 43-9006; Nexavar®), imatinib mesylate (Gleevec®), leflunomide (SU101), vandetanib (ZACTIMA™; ZD6474), pilitinib, CP-654577, CP-724714, HKI-272, PKI-166, AEE788, BMS-599626, HKI-357, BIBW-2992, ARRY-334543, JNJ-26483327, and combinations thereof.

Exemplary anti-proliferative agents include mTOR inhibitors such as sirolimus (rapamycin), temsirolimus (CCI-779), everolimus (RAD001), BEZ-235, and XL765; AKT inhibitors such as 1L6-hydroxymethyl-chiro-inositol-2-(R)-2-O-methyl-3-O-octadecyl-*sn-*glycerocarbonate, 9-methoxy-2-methylellipticinium acetate, 1,3-dihydro-1-(1-((4-(6-phenyl-1H-imidazo[4,5-g]quinoxalin-7-yl)phenyl)methyl)-4-piperidinyl)-2H-benzimidazol-2-one, 10-(4'-(N-diethylamino)butyl)-2-chlorophenoxazine, 3-formylchromone thiosemicarbazone (Cu(II)Cl₂ complex), API-2, a 15-mer peptide derived from amino acids 10-24 of the proto-oncogene TCL1 (Hiromura et al., J. Biol. Chem., 279:53407-53418 (2004), KP372-1, and the compounds described in Kozikowski et al., J. Am. Chem. Soc., 125:1144-1145 (2003) and Kau et al., Cancer Cell, 4:463-476 (2003); PI3K inhibitors such as PX-866, wortmannin, LY 294002, quercetin, tetrodotoxin citrate, thioperamide maleate, GDC-0941 (957054-30-7), IC87114, PI-103, PIK93, BEZ-235 (NVP-BEZ235), TGX-115, ZSTK474, (-)-deguelin, NU 7026, myricetin, tandutinib, GDC-0941 bismesylate, GSK690693, KU-55933, MK-2206, OSU-03012, perifosine, triciribine, XL-147, PIK75, TGX-221, NU 7441, PI 828, XL-765, and WHI-P 154; MEK inhibitors such as PD98059, ARRY-162, RDEA119, U0126, GDC-0973, PD184161, AZD6244, AZD8330, PD-0325901, and ARRY-142886; IGF-1R inhibitors such as BMS-536924; and combinations thereof.

Non-limiting examples of pan-HER inhibitors include PF-00299804, neratinib (HKI-272), AC480 (BMS-599626), BMS-690154, PF-02341066, HM781-36B, CI-1033, BIBW-2992, and combinations thereof.

Non-limiting examples of chemotherapeutic agents include platinum-based drugs (*e.g*., oxaliplatin, cisplatin, carboplatin, spiroplatin, iproplatin, satraplatin, *etc*.), alkylating agents (*e.g*., cyclophosphamide, ifosfamide, chlorambucil, busulfan, melphalan, mechlorethamine, uramustine, thiotepa, nitrosoureas, *etc*.), anti-metabolites (*e.g*., *5-*fluorouracil, 5-fluorocytosine, azathioprine, 6-mercaptopurine, methotrexate, leucovorin, capecitabine, cytarabine, floxuridine, fludarabine, gemcitabine (Gemzar®), pemetrexed (ALIMTA®), raltitrexed, *etc*.), plant alkaloids (*e.g*., vincristine, vinblastine, vinorelbine, vindesine, podophyllotoxin, paclitaxel (Taxol®), docetaxel (Taxotere®), *etc*.), topoisomerase inhibitors (*e.g*., irinotecan, topotecan, amsacrine, etoposide (VP16), etoposide phosphate, teniposide, *etc*.), antitumor antibiotics (*e.g*., doxorubicin, adriamycin, daunorubicin, epirubicin, actinomycin, bleomycin, mitomycin, mitoxantrone, plicamycin, *etc*.), pharmaceutically acceptable salts thereof, stereoisomers thereof, derivatives thereof, analogs thereof, and combinations thereof.

Examples of hormonal therapeutic agents include, without limitation, aromatase inhibitors (*e.g*., aminoglutethimide, anastrozole (Arimidex®), letrozole (Femara®), vorozole, exemestane (Aromasin®), 4-androstene-3,6,17-trione (6-OXO), 1,4,6-androstatrien-3,17-dione (ATD), formestane (Lentaron®), *etc*.), selective estrogen receptor modulators (*e.g*., bazedoxifene, clomifene, fulvestrant, lasofoxifene, raloxifene, tamoxifen, toremifene, *etc*.), steroids (*e.g*., dexamethasone), finasteride, and gonadotropin-releasing hormone agonists (GnRH) such as goserelin, pharmaceutically acceptable salts thereof, stereoisomers thereof, derivatives thereof, analogs thereof, and combinations thereof.

Non-limiting examples of cancer vaccines useful according to the present disclosure include ANYARA from Active Biotech, DCVax-LB from Northwest Biotherapeutics, EP-2101 from IDM Pharma, GV1001 from Pharmexa, 10-2055 from Idera Pharmaceuticals, INGN 225 from Introgen Therapeutics and Stimuvax from Biomira/Merck.

Examples of radiotherapeutic agents include, but are not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

Non-limiting examples of compounds that modulate HER2 activity are described herein and include monoclonal antibodies, tyrosine kinase inhibitors, and combinations thereof. In preferred aspects, the HER2-modulating compound inhibits HER2 activity and/or blocks HER2 signaling, *e.g*., is a HER2 inhibitor. Examples of HER2 inhibitors include, but are not limited to, monoclonal antibodies such as trastuzumab (Herceptin®) and pertuzumab (2C4); small molecule tyrosine kinase inhibitors such as gefitinib (Iressa®), erlotinib (Tarceva®), pelitinib, CP-654577, CP-724714, canertinib (CI 1033), HKI-272, lapatinib (GW-572016; Tykerb®), PKI-166, AEE788, BMS-599626, HKI-357, BIBW-2992, ARRY-334543, JNJ-26483327, and combinations thereof. In other aspects, the HER2-modulating compound activates the HER2 pathway, *e.g*., is a HER2 activator.

Non-limiting examples of compounds that modulate c-Met activity are described herein and include monoclonal antibodies, small molecule inhibitors, and combinations thereof. In preferred aspects, the c-Met-modulating compound inhibits c-Met activity and/or blocks c-Met signaling, *e.g*., is a c-Met inhibitor. Examples of c-Met inhibitors include, but are not limited to, monoclonal antibodies such as AMG102 and MetMAb; small molecule inhibitors of c-Met such as ARQ197, JNJ-38877605, PF-2341066, PF-04217903, SGX523, GSK 1363089/ XL880, XL184, MGCD265, and MK-2461; and combinations thereof. In other aspects, the c-Met-modulating compound activates the c-Met pathway, *e.g*., is a c-Met activator.

Non-limiting examples of signal transduction molecules and pathways include those shown in Table 2.

**Table 2**

| **Pathway 1** | ErbB1 | ErbB1 Phospho | ErbB1 Shc | ErbB1 ubiquitin | ErbB1-PI3K | PTEN | | |
|---|---|---|---|---|---|---|---|---|
| **Pathway 2** | ErbB1 | ErbB1 VIII | ErbB1 VIII Phospho | ErbB1 VIII Shc | ErbB1VIII ubiquitin | ErbB1VII I PI3K | PTEN | |
| **Pathway 3** | ErbB2 | ErbB2 Phospho | HER-2 Shc | ErbB2: PI3K Complex | ErbB2 ubiquitin | PTEN | | |
| **Pathway 4** | ErbB2 | P95Truncated ErbB2 | ErbB2Phosph o | P95Truncated ERBB2 Phospho | HER-2 She | ERBB2: PI3K Complex | ErbB2 ubiquitin | P95ErbB2:P I3K |
| **Pathway 5** | ErbB3 | ErbB3 Phospho | ErbB3:PI3K Complex | ErbB3 PI3K Phospho | ErbB3:Shc | | | |
| **Pathway 6** | ErbB4 | ErbB4 Phospho | ErbB4:Shc | | | | | |
| **Pathway 7** | IGF-1R | IGF-1RPhospho | IGF-1R:IRS | IRS:PI3K | Phospho IRS | IGF-1R :PI3K | | |
| **Pathway 8** | INSR | INSRPhospho | | | | | | |
| **Pathway 9** | KIT | KIT Phospho | | | | | | |
| **Pathway 10** | FLT3 | FLT3Phospho | | | | | | |
| **Pathway 11** | HGFR 1 | HGFR 1 Phospho | | | | | | |
| **Pathway 12** | HGFR 2 | HGFR 2 Phospho | | | | | | |
| **Pathway 13** | RET | RET Phospho | | | | | | |
| **Pathway 14** | PDGFR alpha | PDGFR alpha Phospho | | | | | | |
| **Pathway 15** | PDGFR beta | PDGFR beta Phospho | | | | | | |
| **Pathway 16** | VEGFR 1 | VEGFR 1 Phospho | VEGFR 1: PLCycomplex | VEGFR 1: Src | | | | |
| **Pathway 17** | VEGFR 2 | VEGFR 2 Phospho | VEGFR 2: PLCγ complex | VEGFR 2: Src | VEGFR-2/heparin sulphate complex | VEGFR-2, VE-cadherin complex | | |
| **Pathway 18** | VEGFR 3 | VEGFR 3 Phospho | | | | | | |
| **Pathway 19** | FGFR 1 | FGFR 1 Phospho | | | | | | |
| **Pathway 20** | FGFR 2 | FGFR 2 Phospho | | | | | | |
| **Pathway 21** | FGFR 3 | FGFR 3 Phospho | | | | | | |
| **Pathway 22** | FGFR 4 | FGFR 4 Phospho | | | | | | |
| **Pathway 23** | TIE 1 | TIE 1 Phospho | | | | | | |
| **Pathway 24** | TIE 2 | TIE 2 Phospho | | | | | | |
| **Pathway 25** | EPHA | EPHA Phospho | | | | | | |
| **Pathway 26** | EPHB | EPHB Phospho | | | | | | |
| **Pathway 27** | NFkB-IkB complex | phospho-IκB (S32) Total IkB | Total NFκB Phospho NFκB(S536) | Total P65 Phospho P65 IkBa | | | | |
| **Pathway 28** | ER | Phospho ER | ER-AIB1 | Other ER complexes | | | | |
| **Pathway 29** | PR | Phospho Pr | | PR complexes | | | | |
| **Pathway 30** | Hedgehog Pathway | | | | | | | |
| **Pathway 31** | Wnt pathway | | | | | | | |
| **Pathway 32** | Notch Pathway | | | | | | | |
| **Pathway 33** | Total Mek Phospho Mek (S217/S2 21) | Total Erk Phospho Erk (T202/Y204) | Total Rsk-1 Phospho Rsk-1 (T357/S363) | Total Stat3 Phospho Stat-3 (Y705) (S727) Total Stat 1 Phospho Stat1 (Y 701) | Phospho Bad (S112) Bad (total) | Total Fak Phospho Fak (Y576) | Total cSrc Phospho cSrc(Y416) | Total Ras Phospho Ras |
| **Pathway 34** | Akt (Total) Phospho Akt (T473) | Phospho Akt (T308) | Phospho Bad (S112) Bad (total) | Phospho Bad (S136) | Bad:14-3-3 complex | Total mTor Phospho mTor (S2448) | Total p70S6K Phospho p70S6K (T229) (T389) | GSK3beta Total (Phospho Ser 9) |
| **Pathway 35** | Total Jnk Phospho Jnk (T183/Y1 85) | Total P38 Phospho P38 (T180/Y182) | Total Rb Phospho Rb (S249/T252) Phospho Rb (S780) | Total p53 Phospho p53 (S392) Phospho p53 (S20) | phospho-CREB(S133 ) Total CREB | Total c-Jun phospho-c-Jun; (S63) | Total Paxillin Phospho Paxillin (Y118) | |
| **Pathway 36** | Ki67 | Cleaved Caspase 3,8,9 others | TOPO2 | | | | | |
| **Pathway 37** | TGFbeta | | | | | | | |

Non-limiting examples of analytes such as signal transduction molecules that can be interrogated in a sample such as a cellular extract from a cell line or tumor tissue include, without limitation, receptor tyrosine kinases, non-receptor tyrosine kinases, tyrosine kinase signaling cascade components, nuclear hormone receptors, nuclear receptor coactivators, nuclear receptor repressors, and combinations thereof. In certain instances, the plurality of signal transduction molecules is selected from the group consisting of EGFR (ErbB1), HER2 (ErbB2), p95HER2, HER3 (ErbB3), HER4 (ErbB4), PI3K, SHC, Raf, SRC, MEK, NFkB-IkB, mTOR, PI3K (*e.g*., PIK3CA and/or PIK3R1), VEGF, VEGFR1, VEGFR2, VEGFR3, EPH-A, EPH-B, EPH-C, EPH-D, c-MET, FGFR, c-KIT, FLT-3, TIE-1, TIE-2, c-FMS, PDGFRA, PDGFRB, Abl, FTL 3, RET, HGFR, FGFR1, FGFR2, FGFR3, FGFR4, IGF-1R, ER, PR, NCOR, AIB1, AKT, ERK2 (MAPK1), ERK1 (MAPK3), PDK1, PDK2, PTEN, SGK3, 4E-BP1, P70S6K, protein tyrosine phosphatases (*e.g*., PTP1B, PTPN13, BDP1, *etc*.), receptor dimers, GSK-3β, PIP2, PIP3, p27, and combinations thereof.

In one particular aspect, the present disclosure comprises determining the expression level (*e.g*., total amount) and/or activation level (*e.g*., level of phosphorylation or complex formation) of at least one, two, three, four, five, six, seven, eight, nine, ten, or more markers such as the following analytes: HER1, HER2, HER3, p95HER2, cMET, IGF-1R, cKIT, PI3K (*e.g*., PIK3CA and/or PIK3R1), SHC, and/or VEGFR (*e.g*., VEGFR1, 2, and/or 3).

In certain preferred aspects, the present disclosure comprises (i) determining the expression level of at least one or more of HER1, HER2, HER3, cMET, IGF-1R, PI3K, and/or SHC and/or (ii) determining the activation level of at least one or more of HER1, HER2, HER3, cMET, IGF-1R, PI3K, and/or SHC. In some aspects, the activation level corresponds to a level of phosphorylation of HER1, HER2, HER3, cMET, IGF-1R, and/or SHC. In certain other aspects, the activation level corresponds to a level of a PI3K complex. Examples of PI3K complexes include, without limitation, one or more complexes comprising a dimerized receptor tyrosine kinase pair, a PI3K p85 subunit (*e.g*., PIK3R1), and a PI3K p110 subunit (*e.g*., an α or β subunit such as PIK3CA or PIK3CB); *see, e.g.,* WO2013/033623 (which claims priority from U.S. Provisional Application No. 61/530,621, filed September 2, 2011).

In certain aspects, the present disclosure further comprises determining the expression level (*e.g*., total amount) and/or activation level (*e.g*., level of phosphorylation or complex formation) of one or more (*e.g*., at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more) additional analytes in a cellular extract. In some aspects, the one or more (*e.g*., at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more) additional analytes comprises one or more signal transduction molecules selected from the group consisting of receptor tyrosine kinases, non-receptor tyrosine kinases, tyrosine kinase signaling cascade components, nuclear hormone receptors, nuclear receptor coactivators, nuclear receptor repressors, and combinations thereof.

In particular aspects, the present disclosure further comprises determining the expression level (*e.g*., total amount) and/or activation level (*e.g*., level of phosphorylation or complex formation) of one or any combination of 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more of the following additional analytes: HER4, AKT, ERK1 (MAPK3), ERK2 (MAPK1), MEK, PTEN, SGK3, 4E-BP1, PDK1, PDK2, GSK-3β, Raf, SRC, NFkB-IkB, mTOR, EPH-A, EPH-B, EPH-C, EPH-D, FLT-3, TIE-1, TIE-2, c-FMS, Abl, FTL 3, RET, FGFR1, FGFR2, FGFR3, FGFR4, ER, PR, NCOR, AIB1, RON, PIP2, PIP3, p27, PDGFR (*e.g*., PDGFRA and/or B), p70S6K, protein tyrosine phosphatases (*e.g*., PTP1B, PTPN13, BDP1, *etc*.), receptor dimers, and combinations thereof.

In some aspects, determining the expression level of the one or more analytes comprises detecting the total amount of each of the one or more analytes in the cellular extract with one or more antibodies specific for the corresponding analyte. In particular aspects, the antibodies bind to the analyte irrespective of the activation state of the analyte to be detected, *i.e.,* the antibodies detect both the non-activated and activated forms of the analyte.

Total expression level and/or status can be determined using any of a variety of techniques. In certain aspects, the total expression level and/or status of each of the one or more analytes such as signal transduction molecules in a sample such as a cellular extract from a cell line (*e.g*., lung cancer cell line) or tumor tissue (*e.g*., lung tumor tissue) is detected with an immunoassay such as a single detection assay or a proximity dual detection assay (*e.g*., a Collaborative Enzyme Enhanced Reactive Immunoassay (CEER)) as described herein.

In certain aspects, determining the expression (*e.g*., total) levels of the one or more analytes comprises:
(i) incubating (*e.g*., contacting) a cellular extract produced from a cell with one or a plurality of dilution series of capture antibodies (*e.g*., capture antibodies specific for one or more analytes) to form a plurality of captured analytes, wherein the capture antibodies are restrained on a solid support (*e.g*., to transform the analytes present in the cellular extract into complexes of captured analytes comprising the analytes and capture antibodies);
(ii) incubating (*e.g*., contacting) the plurality of captured analytes with detection antibodies comprising one or a plurality of first and second activation state-independent antibodies specific for the corresponding analytes (*e.g*., first and second activation state-independent antibodies specific for the one or more analytes) to form a plurality of detectable captured analytes (*e.g*., to transform the complexes of captured analytes into complexes of detectable captured analytes comprising the captured analytes and detection antibodies),
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(iii) incubating (*e.g*., contacting) the plurality of detectable captured analytes with a second member of the signal amplification pair to generate an amplified signal; and
(iv) detecting the amplified signal generated from the first and second members of the signal amplification pair.

In certain other aspects, determining the expression (*e.g*., total) levels of the one or more analytes that are truncated receptors (*e.g*., p95HER2) comprises:
(i) incubating (*e.g*., contacting) a cellular extract produced from a cell with a plurality of beads specific for an extracellular domain (ECD) binding region of a full-length receptor (*e.g*., full-length HER2);
(ii) removing the plurality of beads from the cellular extract, thereby removing the full-length receptor (*e.g*., full-length HER2)to form a cellular extract devoid of the full-length receptor (*e.g*., full-length HER2) (*e.g*., to transform the cellular extract into a cellular extract devoid of a specific full-length receptor or family of full-length receptors);
(iii) incubating (*e.g*., contacting) the cellular extract devoid of the full-length receptor (*e.g*., full-length HER2) with one or a plurality of capture antibodies specific for an intracellular domain (ICD) binding region of the full-length receptor (*e.g*., full-length HER2) to form a plurality of captured truncated receptors, wherein the capture antibodies are restrained on a solid support (*e.g*., to transform the truncated receptors present in a full-length receptor-depleted cellular extract into complexes of truncated receptors and capture antibodies);
(iv) incubating the plurality of captured truncated receptors with detection antibodies comprising one or a plurality of first and second activation state-independent antibodies specific for an ICD binding region of the full-length receptor (*e.g*., full-length HER2) to form a plurality of detectable captured truncated receptors (*e.g*., to transform the complexes of captured truncated receptors into complexes of detectable captured truncated receptors comprising the captured truncated receptors and detection antibodies), wh
   erein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(v) incubating (*e.g*., contacting) the plurality of detectable captured truncated receptors with a second member of the signal amplification pair to generate an amplified signal; and
(vi) detecting the amplified signal generated from the first and second members of the signal amplification pair.

The first activation state-independent antibodies may be directly labeled with the facilitating moiety or indirectly labeled with the facilitating moiety, *e.g*., via hybridization between an oligonucleotide conjugated to the first activation state-independent antibodies and a complementary oligonucleotide conjugated to the facilitating moiety. Similarly, the second activation state-independent antibodies may be directly labeled with the first member of the signal amplification pair or indirectly labeled with the first member of the signal amplification pair, *e.g*., via binding between a first member of a binding pair conjugated to the second activation state-independent antibodies and a second member of the binding pair conjugated to the first member of the signal amplification pair. In certain instances, the first member of the binding pair is biotin and the second member of the binding pair is an avidin such as streptavidin or neutravidin.

In some aspects, the facilitating moiety may be, for example, glucose oxidase. In certain instances, the glucose oxidase and the first activation state-independent antibodies can be conjugated to a sulfhydryl-activated dextran molecule as described in, *e.g*., Examples 16-17 of PCT Publication No. WO2009/108637. The sulfhydryl-activated dextran molecule typically has a molecular weight of about 500kDa (*e.g*., about 250, 300, 350, 400, 450, 500, 550, 600, 650, 700, or 750kDa). In other aspects, the oxidizing agent may be, for example, hydrogen peroxide (H₂O₂). In yet other aspects, the first member of the signal amplification pair may be, for example, a peroxidase such as horseradish peroxidase (HRP). In further aspects, the second member of the signal amplification pair may be, for example, a tyramide reagent (*e.g*., biotin-tyramide). Preferably, the amplified signal is generated by peroxidase oxidization of biotin-tyramide to produce an activated tyramide (*e.g*., to transform the biotin-tyramide into an activated tyramide). The activated tyramide may be directly detected or indirectly detected, *e.g*., upon the addition of a signal-detecting reagent. Non-limiting examples of signal-detecting reagents include streptavidin-labeled fluorophores and combinations of streptavidin-labeled peroxidases and chromogenic reagents such as, *e.g*., 3,3',5,5'-tetramethylbenzidine (TMB).

In certain instances, the horseradish peroxidase and the second activation state-independent antibodies can be conjugated to a sulfhydryl-activated dextran molecule. The sulfhydryl-activated dextran molecule typically has a molecular weight of about 70kDa (*e.g*., about 40, 45, 50, 55, 60, 65, 70, 75, 80, 85, 90, 95, or 100kDa).

The truncated receptor is typically a fragment of the full-length receptor and shares an intracellular domain (ICD) binding region with the full-length receptor. In certain aspects, the full-length receptor comprises an extracellular domain (ECD) binding region, a transmembrane domain, and an intracellular domain (ICD) binding region. Without being bound to any particular theory, the truncated receptor may arise through the proteolytic processing of the ECD of the full-length receptor or by alternative initiation of translation from methionine residues that are located before, within, or after the transmembrane domain, *e.g.,* to create a truncated receptor with a shortened ECD or a truncated receptor comprising a membrane-associated or cytosolic ICD fragment.

In certain preferred aspects, the truncated receptor is p95HER2 and the corresponding full-length receptor is HER2. However, one skilled in the art will appreciate that the methods described herein for detecting truncated proteins can be applied to a number of different proteins including, but not limited to, the EGFR VIII mutant (implicated in glioblastoma, colorectal cancer, *etc*.), other truncated receptor tyrosine kinases, caspases, and the like. Example 12 of PCT Publication No. WO2009/108637 provides an exemplary embodiment of the assay methods of the present dislcosure for detecting truncated receptors such as p95HER2 in cells using a multiplex, high-throughput, proximity dual detection microarray ELISA having superior dynamic range.

In some aspects, the plurality of beads specific for an ECD binding region comprises a streptavidin-biotin pair, wherein the streptavidin is attached to the bead and the biotin is attached to an antibody. In certain instances, the antibody is specific for the ECD binding region of the full-length receptor (*e.g*., full-length HER2).

In some apsects, each dilution series of capture antibodies comprises a series of descending capture antibody concentrations. In certain instances, the capture antibodies are serially diluted at least 2-fold (*e.g*., 2, 5, 10, 20, 50, 100, 500, or 1000-fold) to produce a dilution series comprising a set number (*e.g*., 2, 3, 4, 5, 6, 7, 8, 9, 10, 15, 20, 25, or more) of descending capture antibody concentrations which are spotted onto an array. Preferably, at least 2, 3, 4, 5, or 6 replicates of each capture antibody dilution are spotted onto the array.

In other aspects, the solid support comprises glass (*e.g*., a glass slide), plastic, chips, pins, filters, beads, paper, membrane (*e.g*., nylon, nitrocellulose, polyvinylidene fluoride (PVDF), *etc*.), fiber bundles, or any other suitable substrate. In a preferred aspect, the capture antibodies are restrained (*e.g*., via covalent or noncovalent interactions) on glass slides coated with a nitrocellulose polymer such as, for example, FAST® Slides, which are commercially available from Whatman Inc. (Florham Park, NJ). Exemplary methods for constructing antibody arrays suitable for use in accordance with the disclosure are described, *e.g*., in PCT Publication No. WO2009/108637.

In further aspects, determining the activation levels of the one or more analytes comprises detecting a phosphorylation level of the one or more analytes in the cellular extract with antibodies specific for the phosphorylated form of each of the analytes to be detected.

Phosphorylation levels and/or status can be determined using any of a variety of techniques. For example, it is well known in the art that phosphorylated proteins can be detected via immunoassays using antibodies that specifically recognize the phosphorylated form of the protein (*see, e.g*., Lin et al., Br. J. Cancer, 93:1372-1381 (2005)). Immunoassays generally include immunoblotting (*e.g*., Western blotting), RIA, and ELISA. More specific types of immunoassays include antigen capture/antigen competition, antibody capture/antigen competition, two-antibody sandwiches, antibody capture/antibody excess, and antibody capture/antigen excess. Methods of making antibodies are described herein and in Harlow and Lane, Antibodies: A Laboratory Manual, 1988, Cold Spring Harbor Laboratory Press, Cold Spring Harbor, NY, USA. Phospho-specifc antibodies can be made *de novo* or obtained from commercial or noncommercial sources. Phosphorylation levels and/or status can also be determined by metabolically labeling cells with radioactive phosphate in the form of [γ-³²P]ATP or [γ-³³P]ATP. Phosphorylated proteins become radioactive and hence traceable and quantifiable through scintillation counting, radiography, and the like (see, e.g., Wang et al., J. Biol. Chem., 253:7605-7608 (1978)). For example, metabolically labeled proteins can be extracted from cells, separated by gel electrophoresis, transferred to a membrane, probed with an antibody specific for a particular analyte and subjected to autoradiography to detect ³²P or ³³P. Alternatively, the gel can be subjected to autoradiography prior to membrane transference and antibody probing.

In particular apsects, the activation (*e.g*., phosphorylation) level and/or status of each of the one or more analytes in a sample such as a cellular extract from a cell line (*e.g*., lung cancer cell line) or tumor tissue (*e.g*., lung tumor tissue) is detected with an immunoassay such as a single detection assay or a proximity dual detection assay (*e.g*., a Collaborative Enzyme Enhanced Reactive Immunoassay (CEER)) as described herein.

In certain aspects, determining the activation (*e.g*., phosphorylation) level of the one or more analytes comprises:
(i) incubating (*e.g*., contacting) a cellular extract produced from a cell with a dilution series of capture antibodies (*e.g*., capture antibodies specific for one or more analytes) to form a plurality of captured analytes, wherein the capture antibodies are restrained on a solid support (*e.g*., to transform the analytes present in the cellular extract into complexes of captured analytes comprising the analytes and capture antibodies);
(ii) incubating (*e.g*., contacting) the plurality of captured analytes with detection antibodies comprising activation state-independent antibodies specific for the corresponding analytes (*e.g*., activation state-independent antibodies specific for the one or more analytes) and activation state-dependent antibodies specific for the corresponding analytes (*e.g*., activation state-dependent antibodies specific for the one or more analytes) to form a plurality of detectable captured analytes (*e.g*., to transform the complexes of captured analytes into complexes of detectable captured analytes comprising the captured analytes and detection antibodies),
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating (*e.g*., contacting) the plurality of detectable captured analytes with a second member of the signal amplification pair to generate an amplified signal; and
(iv) detecting the amplified signal generated from the first and second members of the signal amplification pair.

In certain other apsects, determining the activation (*e.g*., phosphorylation) level of the one or more analytes that are truncated receptors (*e.g*., p95HER2) comprises:
(i) incubating (*e.g*., contacting) a cellular extract produced from a cell with a plurality of beads specific for an extracellular domain (ECD) binding region of a full-length receptor (*e.g*., full-length HER2);
(ii) removing the plurality of beads from the cellular extract, thereby removing the full-length receptor (*e.g*., full-length HER2) to form a cellular extract devoid of the full-length receptor (*e.g*., full-length HER2) (*e.g*., to transform the cellular extract into a cellular extract devoid of a specific full-length receptor or family of full-length receptors);
(iii) incubating (*e.g*., contacting) the cellular extract devoid of the full-length receptor (*e.g*., full-length HER2) with a plurality of capture antibodies specific for an intracellular domain (ICD) binding region of the full-length receptor (*e.g*., full-length HER2) to form a plurality of captured truncated receptors, wherein the capture antibodies are restrained on a solid support (*e.g*., to transform the truncated receptors present in a full-length receptor-depleted cellular extract into complexes of truncated receptors and capture antibodies);
(iv) incubating (*e.g*., contacting) the plurality of captured truncated receptors with detection antibodies comprising activation state-independent antibodies and activation state-dependent antibodies specific for an ICD binding region of the full-length receptor (*e.g*., full-length HER2) to form a plurality of detectable captured truncated receptors (*e.g*., to transform the complexes of captured truncated receptors into complexes of detectable captured truncated receptors comprising the captured truncated receptors and detection antibodies), wh
   erein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(v) incubating (*e.g*., contacting) the plurality of detectable captured truncated receptors with a second member of the signal amplification pair to generate an amplified signal; and
(vi) detecting the amplified signal generated from the first and second members of the signal amplification pair.

The activation state-independent antibodies may be directly labeled with the facilitating moiety or indirectly labeled with the facilitating moiety, *e.g*., via hybridization between an oligonucleotide conjugated to the activation state-independent antibodies and a complementary oligonucleotide conjugated to the facilitating moiety. Similarly, the activation state-dependent antibodies may be directly labeled with the first member of the signal amplification pair or indirectly labeled with the first member of the signal amplification pair, *e.g.,* via binding between a first member of a binding pair conjugated to the activation state-dependent antibodies and a second member of the binding pair conjugated to the first member of the signal amplification pair. In certain instances, the first member of the binding pair is biotin and the second member of the binding pair is an avidin such as streptavidin or neutravidin.

In some aspects, the facilitating moiety may be, for example, glucose oxidase. In certain instances, the glucose oxidase and the activation state-independent antibodies can be conjugated to a sulfhydryl-activated dextran molecule as described in, *e.g*., Examples 16-17 of PCT Publication No. WO2009/108637. The sulfhydryl-activated dextran molecule typically has a molecular weight of about 500kDa (*e.g*., about 250, 300, 350, 400, 450, 500, 550, 600, 650, 700, or 750kDa). In other aspects, the oxidizing agent may be, for example, hydrogen peroxide (H₂O₂). In yet other aspects, the first member of the signal amplification pair may be, for example, a peroxidase such as horseradish peroxidase (HRP). In further aspects, the second member of the signal amplification pair may be, for example, a tyramide reagent (*e.g*., biotin-tyramide). Preferably, the amplified signal is generated by peroxidase oxidization of biotin-tyramide to produce an activated tyramide (*e.g*., to transform the biotin-tyramide into an activated tyramide). The activated tyramide may be directly detected or indirectly detected, *e.g*., upon the addition of a signal-detecting reagent. Non-limiting examples of signal-detecting reagents include streptavidin-labeled fluorophores and combinations of streptavidin-labeled peroxidases and chromogenic reagents such as, *e.g*., 3,3',5,5'-tetramethylbenzidine (TMB).

In certain instances, the horseradish peroxidase and the activation state-dependent antibodies can be conjugated to a sulfhydryl-activated dextran molecule. The sulfhydryl-activated dextran molecule typically has a molecular weight of about 70kDa (*e.g*., about 40, 45, 50, 55, 60, 65, 70, 75, 80, 85, 90, 95, or 100kDa).

The truncated receptor is typically a fragment of the full-length receptor and shares an intracellular domain (ICD) binding region with the full-length receptor. In certain aspects, the full-length receptor comprises an extracellular domain (ECD) binding region, a transmembrane domain, and an intracellular domain (ICD) binding region. Without being bound to any particular theory, the truncated receptor may arise through the proteolytic processing of the ECD of the full-length receptor or by alternative initiation of translation from methionine residues that are located before, within, or after the transmembrane domain, *e.g.,* to create a truncated receptor with a shortened ECD or a truncated receptor comprising a membrane-associated or cytosolic ICD fragment.

In certain preferred aspects, the truncated receptor is p95HER2 and the corresponding full-length receptor is HER2. However, one skilled in the art will appreciate that the methods described herein for detecting truncated proteins can be applied to a number of different proteins including, but not limited to, the EGFR VIII mutant (implicated in glioblastoma, colorectal cancer, *etc*.), other truncated receptor tyrosine kinases, caspases, and the like. Example 12 of PCT Publication No. WO2009/108637 provides an exemplary embodiment of the assay methods of the present disclosure for detecting truncated receptors such as p95HER2 in cells using a multiplex, high-throughput, proximity dual detection microarray ELISA having superior dynamic range.

In some aspects, the plurality of beads specific for an ECD binding region comprises a streptavidin-biotin pair, wherein the streptavidin is attached to the bead and the biotin is attached to an antibody. In certain instances, the antibody is specific for the ECD binding region of the full-length receptor (*e.g*., full-length HER2).

In some aspects, each dilution series of capture antibodies comprises a series of descending capture antibody concentrations. In certain instances, the capture antibodies are serially diluted at least 2-fold (*e.g*., 2, 5, 10, 20, 50, 100, 500, or 1000-fold) to produce a dilution series comprising a set number (*e.g*., 2, 3, 4, 5, 6, 7, 8, 9, 10, 15, 20, 25, or more) of descending capture antibody concentrations which are spotted onto an array. Preferably, at least 2, 3, 4, 5, or 6 replicates of each capture antibody dilution are spotted onto the array.

In other aspects, the solid support comprises glass (*e.g*., a glass slide), plastic, chips, pins, filters, beads, paper, membrane (*e.g*., nylon, nitrocellulose, polyvinylidene fluoride (PVDF), *etc*.), fiber bundles, or any other suitable substrate. In a preferred aspect, the capture antibodies are restrained (*e.g*., via covalent or noncovalent interactions) on glass slides coated with a nitrocellulose polymer such as, for example, FAST® Slides, which are commercially available from Whatman Inc. (Florham Park, NJ). Exemplary methods for constructing antibody arrays suitable for use in accordance with the disclosure are described, *e.g*., in PCT Publication No. WO2009/108637.

### IV. Single Detection Assays

In some aspectst, the assay for detecting the expression and/or activation level or status of one or more analytes (*e.g*., one or more signal transduction molecules such as one or more components of the HER2 and/or c-Met signaling pathways) of interest in a cellular extract of cells such as tumor cells is a multiplex, high-throughput two-antibody assay having superior dynamic range. As a non-limiting example, the two antibodies used in the assay can comprise: (1) a capture antibody specific for a particular analyte of interest; and (2) a detection antibody specific for an activated form of the analyte (*i.e*., activation state-dependent antibody). The activation state-dependent antibody is capable of detecting, for example, the phosphorylation, ubiquitination, and/or complexation state of the analyte. Alternatively, the detection antibody comprises an activation state-independent antibody, which detects the total amount of the analyte in the cellular extract. The activation state-independent antibody is generally capable of detecting both the activated and non-activated forms of the analyte.

In one particular aspect, the two-antibody assay for detecting the expression or activation level of an analyte of interest comprises:
(i) incubating the cellular extract with one or a plurality of dilution series of capture antibodies to form a plurality of captured analytes;
(ii) incubating the plurality of captured analytes with detection antibodies specific for the corresponding analytes to form a plurality of detectable captured analytes, wherein the detection antibodies comprise activation state-dependent antibodies for detecting the activation (*e.g*., phosphorylation) level of the analyte or activation state-independent antibodies for detecting the expression level (*e.g*., total amount) of the analyte;
(iii) incubating the plurality of detectable captured analytes with first and second members of a signal amplification pair to generate an amplified signal; and
(iv) detecting the amplified signal generated from the first and second members of the signal amplification pair.

The two-antibody assays described herein are typically antibody-based arrays which comprise a plurality of different capture antibodies at a range of capture antibody concentrations that are coupled to the surface of a solid support in different addressable locations. Examples of suitable solid supports for use in accordance with the present disclosure are described above.

The capture antibodies and detection antibodies are preferably selected to minimize competition between them with respect to analyte binding (*i.e*., both capture and detection antibodies can simultaneously bind their corresponding signal transduction molecules).

In one aspect, the detection antibodies comprise a first member of a binding pair (*e.g*., biotin) and the first member of the signal amplification pair comprises a second member of the binding pair (*e.g*., streptavidin). The binding pair members can be coupled directly or indirectly to the detection antibodies or to the first member of the signal amplification pair using methods well-known in the art. In certain instances, the first member of the signal amplification pair is a peroxidase (*e.g*., horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, *etc*.), and the second member of the signal amplification pair is a tyramide reagent (*e.g*., biotin-tyramide). In these instances, the amplified signal is generated by peroxidase oxidization of the tyramide reagent to produce an activated tyramide in the presence of hydrogen peroxide (H₂O₂).

The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores include, but are not limited to, an Alexa Fluor® dye (*e.g*., Alexa Fluor® 555), fluorescein, fluorescein isothiocyanate (FITC), Oregon Green™; rhodamine, Texas red, tetrarhodamine isothiocynate (TRITC), a CyDye™ fluor (*e.g*., Cy2, Cy3, Cy5), and the like. The streptavidin label can be coupled directly or indirectly to the fluorophore or peroxidase using methods well-known in the art. Non-limiting examples of chromogenic reagents include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

An exemplary protocol for performing the two-antibody assays described herein is provided in Example 3 of PCT Publication No. WO2009/108637.

In another example of a two-antibody approach, the present disclosure provides a method for detecting the expression or activation level of a truncated receptor, the method comprising:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with a dilution series of one or a plurality of capture antibodies specific for an intracellular domain (ICD) binding region of the full-length receptor to form a plurality of captured truncated receptors;
(iv) incubating the plurality of captured truncated receptors with detection antibodies specific for an ICD binding region of the full-length receptor to form a plurality of detectable captured truncated receptors, wherein the detection antibodies comprise activation state-dependent antibodies for detecting the activation (*e.g*., phosphorylation) level of the truncated receptor or activation state-independent antibodies for detecting the expression level (*e.g*., total amount) of the truncated receptor;
(v) incubating the plurality of detectable captured truncated receptors with first and second members of a signal amplification pair to generate an amplified signal; and
(vi) detecting an amplified signal generated from the first and second members of the signal amplification pair.

In certain apsects, the truncated receptor is p95HER2 and the full-length receptor is HER2. In certain other aspects, the plurality of beads specific for an extracellular domain (ECD) binding region comprises a streptavidin-biotin pair, wherein the biotin is attached to the bead and the biotin is attached to an antibody (*e.g*., wherein the antibody is specific for the ECD binding region of the full-length receptor).

Figure 14A of PCT Publication No. WO2009/108637 shows that beads coated with an antibody directed to the extracellular domain (ECD) of a receptor of interest binds the full-length receptor (*e.g*., HER2), but not the truncated receptor (*e.g*., p95HER2) to remove any full-length receptor from the assay. Figure 14B of PCT Publication No. WO2009/108637 shows that the truncated receptor (*e.g*., p95HER2), once bound to a capture antibody, may then be detected by a detection antibody that is specific for the intracellular domain (ICD) of the full-length receptor (*e.g*., HER2). The detection antibody may be directly conjugated to horseradish peroxidase (HRP). Tyramide signal amplification (TSA) may then be performed to generate a signal to be detected. The expression level or activation state of the truncated receptor (*e.g*., p95HER2) can be interrogated to determine, *e.g*., its total concentration or its phosphorylation state, ubiquitination state, and/or complexation state.

In another aspect, the present disclosure provides kits for performing the two-antibody assays described above comprising: (a) a dilution series of one or a plurality of capture antibodies restrained on a solid support; and (b) one or a plurality of detection antibodies (*e.g*., activation state-independent antibodies and/or activation state-dependent antibodies). In some instances, the kits can further contain instructions for methods of using the kit to detect the expression levels and/or activation states of one or a plurality of signal transduction molecules of cells such as tumor cells. The kits may also contain any of the additional reagents described above with respect to performing the specific methods of the present disclosure such as, for example, first and second members of the signal amplification pair, tyramide signal amplification reagents, wash buffers, *etc.*

### V. Proximity Dual Detection Assays

In some aspects, the assay for detecting the expression and/or activation level of one or more analytes (*e.g*., one or more signal transduction molecules such as one or more components of the HER2 and/or c-Met signaling pathways) of interest in a cellular extract of cells such as tumor cells is a multiplex, high-throughput proximity (*i.e*., three-antibody) assay having superior dynamic range. As a non-limiting example, the three antibodies used in the proximity assay can comprise: (1) a capture antibody specific for a particular analyte of interest; (2) a detection antibody specific for an activated form of the analyte (*i.e*., activation state-dependent antibody); and (3) a detection antibody which detects the total amount of the analyte (*i.e*., activation state-independent antibody). The activation state-dependent antibody is capable of detecting, *e.g*., the phosphorylation, ubiquitination, and/or complexation state of the analyte, while the activation state-independent antibody is capable of detecting the total amount (*i.e*., both the activated and non-activated forms) of the analyte.

In one particular aspect, the proximity assay for detecting the activation level or status of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of activation state-independent antibodies and one or a plurality of activation state-dependent antibodies specific for the corresponding analytes to form a plurality of detectable captured analytes, wh
   erein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular aspect, the proximity assay for detecting the activation level or status of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of activation state-independent antibodies and one or a plurality of activation state-dependent antibodies specific for an ICD binding region of the full-length receptor to form a plurality of detectable captured truncated receptors,
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain aspects, the truncated receptor is p95HER2 and the full-length receptor is HER2. In certain other aspect, the plurality of beads specific for an extracellular domain (ECD) binding region comprises a streptavidin-biotin pair, wherein the biotin is attached to the bead and the biotin is attached to an antibody (*e.g*., wherein the antibody is specific for the ECD binding region of the full-length receptor).

In alternative aspect, the activation state-dependent antibodies can be labeled with a facilitating moiety and the activation state-independent antibodies can be labeled with a first member of a signal amplification pair.

As another non-limiting example, the three antibodies used in the proximity assay can comprise: (1) a capture antibody specific for a particular analyte of interest; (2) a first detection antibody which detects the total amount of the analyte (*i.e*., a first activation state-independent antibody); and (3) a second detection antibody which detects the total amount of the analyte (*i.e*., a second activation state-independent antibody). In preferred aspects, the first and second activation state-independent antibodies recognize different (*e.g*., distinct) epitopes on the analyte.

In one particular aspect, the proximity assay for detecting the expression level of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of first and second activation state-independent antibodies specific for the corresponding analytes to form a plurality of detectable captured analytes,
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular aspect, the proximity assay for detecting the expression level of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of first and second activation state-independent antibodies specific for an ICD binding region of the full-length receptor to form a plurality of detectable captured truncated receptors, wh
   erein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain aspects, the truncated receptor is p95HER2 and the full-length receptor is HER2. In certain other aspects, the plurality of beads specific for an extracellular domain (ECD) binding region comprises a streptavidin-biotin pair, wherein the biotin is attached to the bead and the biotin is attached to an antibody (*e.g*., wherein the antibody is specific for the ECD binding region of the full-length receptor).

In alternative aspects, the first activation state-independent antibodies can be labeled with a first member of a signal amplification pair and the second activation state-independent antibodies can be labeled with a facilitating moiety.

The proximity assays described herein are typically antibody-based arrays which comprise one or a plurality of different capture antibodies at a range of capture antibody concentrations that are coupled to the surface of a solid support in different addressable locations. Examples of suitable solid supports for use in the present disclosure are described above.

The capture antibodies, activation state-independent antibodies, and activation state-dependent antibodies are preferably selected to minimize competition between them with respect to analyte binding (*i.e*., all antibodies can simultaneously bind their corresponding signal transduction molecules).

In some aspects, activation state-independent antibodies for detecting activation levels of one or more of the analytes or, alternatively, first activation state-independent antibodies for detecting expression levels of one or more of the analytes further comprise a detectable moiety. In such instances, the amount of the detectable moiety is correlative to the amount of one or more of the analytes in the cellular extract. Examples of detectable moieties include, but are not limited to, fluorescent labels, chemically reactive labels, enzyme labels, radioactive labels, and the like. Preferably, the detectable moiety is a fluorophore such as an Alexa Fluor® dye (*e.g*., Alexa Fluor® 647), fluorescein, fluorescein isothiocyanate (FITC), Oregon Green™; rhodamine, Texas red, tetrarhodamine isothiocynate (TRITC), a CyDye™ fluor (*e.g*., Cy2, Cy3, Cy5), and the like. The detectable moiety can be coupled directly or indirectly to the activation state-independent antibodies using methods well-known in the art.

In certain instances, activation state-independent antibodies for detecting activation levels of one or more of the analytes or, alternatively, first activation state-independent antibodies for detecting expression levels of one or more of the analytes are directly labeled with the facilitating moiety. The facilitating moiety can be coupled to activation state-independent antibodies using methods well-known in the art. A suitable facilitating moiety for use in the present disclosure includes any molecule capable of generating an oxidizing agent which channels to (*i.e*., is directed to) and reacts with (*i.e*., binds, is bound by, or forms a complex with) another molecule in proximity (*i.e*., spatially near or close) to the facilitating moiety. Examples of facilitating moieties include, without limitation, enzymes such as glucose oxidase or any other enzyme that catalyzes an oxidation/reduction reaction involving molecular oxygen (O₂) as the electron acceptor, and photosensitizers such as methylene blue, rose bengal, porphyrins, squarate dyes, phthalocyanines, and the like. Non-limiting examples of oxidizing agents include hydrogen peroxide (H₂O₂), a singlet oxygen, and any other compound that transfers oxygen atoms or gains electrons in an oxidation/reduction reaction. Preferably, in the presence of a suitable substrate (*e.g*., glucose, light, *etc*.), the facilitating moiety (*e.g*., glucose oxidase, photosensitizer, *etc*.) generates an oxidizing agent (*e.g*., hydrogen peroxide (H₂O₂), single oxygen, *etc*.) which channels to and reacts with the first member of the signal amplification pair (*e.g*., horseradish peroxidase (HRP), hapten protected by a protecting group, an enzyme inactivated by thioether linkage to an enzyme inhibitor, *etc*.) when the two moieties are in proximity to each other.

In certain other instances, activation state-independent antibodies for detecting activation levels of one or more of the analytes or, alternatively, first activation state-independent antibodies for detecting expression levels of one or more of the analytes are indirectly labeled with the facilitating moiety via hybridization between an oligonucleotide linker conjugated to the activation state-independent antibodies and a complementary oligonucleotide linker conjugated to the facilitating moiety. The oligonucleotide linkers can be coupled to the facilitating moiety or to the activation state-independent antibodies using methods well-known in the art. In some aspects, the oligonucleotide linker conjugated to the facilitating moiety has 100% complementarity to the oligonucleotide linker conjugated to the activation state-independent antibodies. In other aspects, the oligonucleotide linker pair comprises at least one, two, three, four, five, six, or more mismatch regions, e.g., upon hybridization under stringent hybridization conditions. One skilled in the art will appreciate that activation state-independent antibodies specific for different analytes can either be conjugated to the same oligonucleotide linker or to different oligonucleotide linkers.

The length of the oligonucleotide linkers that are conjugated to the facilitating moiety or to the activation state-independent antibodies can vary. In general, the linker sequence can be at least about 5, 10, 15, 20, 25, 30, 35, 40, 45, 50, 75, or 100 nucleotides in length. Typically, random nucleic acid sequences are generated for coupling. As a non-limiting example, a library of oligonucleotide linkers can be designed to have three distinct contiguous domains: a spacer domain; signature domain; and conjugation domain. Preferably, the oligonucleotide linkers are designed for efficient coupling without destroying the function of the facilitating moiety or activation state-independent antibodies to which they are conjugated.

The oligonucleotide linker sequences can be designed to prevent or minimize any secondary structure formation under a variety of assay conditions. Melting temperatures are typically carefully monitored for each segment within the linker to allow their participation in the overall assay procedures. Generally, the range of melting temperatures of the segment of the linker sequence is between 1-10°C. Computer algorithms (*e.g*., OLIGO 6.0) for determining the melting temperature, secondary structure, and hairpin structure under defined ionic concentrations can be used to analyze each of the three different domains within each linker. The overall combined sequences can also be analyzed for their structural characterization and their comparability to other conjugated oligonucleotide linker sequences, *e.g*., whether they will hybridize under stringent hybridization conditions to a complementary oligonucleotide linker.

The spacer region of the oligonucleotide linker provides adequate separation of the conjugation domain from the oligonucleotide crosslinking site. The conjugation domain functions to link molecules labeled with a complementary oligonucleotide linker sequence to the conjugation domain via nucleic acid hybridization. The nucleic acid-mediated hybridization can be performed either before or after antibody-analyte (*i.e*., antigen) complex formation, providing a more flexible assay format. Unlike many direct antibody conjugation methods, linking relatively small oligonucleotides to antibodies or other molecules has minimal impact on the specific affinity of antibodies towards their target analyte or on the function of the conjugated molecules.

In some aspects, the signature sequence domain of the oligonucleotide linker can be used in complex multiplexed protein assays. Multiple antibodies can be conjugated with oligonucleotide linkers with different signature sequences. In multiplex immunoassays, reporter oligonucleotide sequences labeled with appropriate probes can be used to detect cross-reactivity between antibodies and their antigens in the multiplex assay format.

Oligonucleotide linkers can be conjugated to antibodies or other molecules using several different methods. For example, oligonucleotide linkers can be synthesized with a thiol group on either the 5' or 3' end. The thiol group can be deprotected using reducing agents (*e*.*g*., TCEP-HCl) and the resulting linkers can be purified by using a desalting spin column. The resulting deprotected oligonucleotide linkers can be conjugated to the primary amines of antibodies or other types of proteins using heterobifunctional cross linkers such as SMCC. Alternatively, 5'-phosphate groups on oligonucleotides can be treated with watersoluble carbodiimide EDC to form phosphate esters and subsequently coupled to amine-containing molecules. In certain instances, the diol on the 3'-ribose residue can be oxidized to aldehyde groups and then conjugated to the amine groups of antibodies or other types of proteins using reductive amination. In certain other instances, the oligonucleotide linker can be synthesized with a biotin modification on either the 3' or 5' end and conjugated to streptavidin-labeled molecules.

Oligonucleotide linkers can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). In general, the synthesis of oligonucleotides makes use of common nucleic acid protecting and coupling groups, such as dimethoxytrityl at the 5'-end and phosphoramidites at the 3'-end. Suitable reagents for oligonucleotide synthesis, methods for nucleic acid deprotection, and methods for nucleic acid purification are known to those of skill in the art.

In certain instances, activation state-dependent antibodies for detecting activation levels of one or more of the analytes or, alternatively, second activation state-independent antibodies for detecting expression levels of one or more of the analytes are directly labeled with the first member of the signal amplification pair. The signal amplification pair member can be coupled to activation state-dependent antibodies to detect activation levels or second activation state-independent antibodies to detect expression levels using methods well-known in the art. In certain other instances, activation state-dependent antibodies or second activation state-independent antibodies are indirectly labeled with the first member of the signal amplification pair via binding between a first member of a binding pair conjugated to the activation state-dependent antibodies or second activation state-independent antibodies and a second member of the binding pair conjugated to the first member of the signal amplification pair. The binding pair members (*e.g*., biotin/streptavidin) can be coupled to the signal amplification pair member or to the activation state-dependent antibodies or second activation state-independent antibodies using methods well-known in the art. Examples of signal amplification pair members include, but are not limited to, peroxidases such horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, and the like. Other examples of signal amplification pair members include haptens protected by a protecting group and enzymes inactivated by thioether linkage to an enzyme inhibitor.

In one example of proximity channeling, the facilitating moiety is glucose oxidase (GO) and the first member of the signal amplification pair is horseradish peroxidase (HRP). When the GO is contacted with a substrate such as glucose, it generates an oxidizing agent (*i.e*., hydrogen peroxide (H₂O₂)). If the HRP is within channeling proximity to the GO, the H₂O₂ generated by the GO is channeled to and complexes with the HRP to form an HRP-H₂O₂ complex, which, in the presence of the second member of the signal amplification pair (*e.g*., a chemiluminescent substrate such as luminol or isoluminol or a fluorogenic substrate such as tyramide (*e.g*., biotin-tyramide), homovanillic acid, or 4-hydroxyphenyl acetic acid), generates an amplified signal. Methods of using GO and HRP in a proximity assay are described in, *e.g*., Langry *et al*., U.S. Dept. of Energy Report No. UCRL-ID-136797 (1999). When biotin-tyramide is used as the second member of the signal amplification pair, the HRP-H₂O₂ complex oxidizes the tyramide to generate a reactive tyramide radical that covalently binds nearby nucleophilic residues. The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores include, but are not limited to, an Alexa Fluor® dye (*e.g*., Alexa Fluor® 555), fluorescein, fluorescein isothiocyanate (FITC), Oregon Green™; rhodamine, Texas red, tetrarhodamine isothiocynate (TRITC), a CyDye™ fluor (*e.g*., Cy2, Cy3, Cy5), and the like. The streptavidin label can be coupled directly or indirectly to the fluorophore or peroxidase using methods well-known in the art. Non-limiting examples of chromogenic reagents include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

In another example of proximity channeling, the facilitating moiety is a photosensitizer and the first member of the signal amplification pair is a large molecule labeled with multiple haptens that are protected with protecting groups that prevent binding of the haptens to a specific binding partner (*e.g*., ligand, antibody, *etc.*). For example, the signal amplification pair member can be a dextran molecule labeled with protected biotin, coumarin, and/or fluorescein molecules. Suitable protecting groups include, but are not limited to, phenoxy-, analino-, olefin-, thioether-, and selenoether-protecting groups. Additional photosensitizers and protected hapten molecules suitable for use in the proximity assays of the present invention are described in U.S. Patent No. 5,807,675. When the photosensitizer is excited with light, it generates an oxidizing agent (*i.e*., singlet oxygen). If the hapten molecules are within channeling proximity to the photosensitizer, the singlet oxygen generated by the photosensitizer is channeled to and reacts with thioethers on the protecting groups of the haptens to yield carbonyl groups (ketones or aldehydes) and sulphinic acid, releasing the protecting groups from the haptens. The unprotected haptens are then available to specifically bind to the second member of the signal amplification pair (*e.g*., a specific binding partner that can generate a detectable signal). For example, when the hapten is biotin, the specific binding partner can be an enzyme-labeled streptavidin. Exemplary enzymes include alkaline phosphatase, β-galactosidase, HRP, *etc.* After washing to remove unbound reagents, the detectable signal can be generated by adding a detectable (*e.g*., fluorescent, chemiluminescent, chromogenic, *etc.*) substrate of the enzyme and detected using suitable methods and instrumentation known in the art. Alternatively, the detectable signal can be amplified using tyramide signal amplification and the activated tyramide either directly detected or detected upon the addition of a signal-detecting reagent as described above.

In yet another example of proximity channeling, the facilitating moiety is a photosensitizer and the first member of the signal amplification pair is an enzyme-inhibitor complex. The enzyme and inhibitor (*e.g*., phosphonic acid-labeled dextran) are linked together by a cleavable linker (*e.g*., thioether). When the photosensitizer is excited with light, it generates an oxidizing agent (*i.e*., singlet oxygen). If the enzyme-inhibitor complex is within channeling proximity to the photosensitizer, the singlet oxygen generated by the photosensitizer is channeled to and reacts with the cleavable linker, releasing the inhibitor from the enzyme, thereby activating the enzyme. An enzyme substrate is added to generate a detectable signal, or alternatively, an amplification reagent is added to generate an amplified signal.

In a further example of proximity channeling, the facilitating moiety is HRP, the first member of the signal amplification pair is a protected hapten or an enzyme-inhibitor complex as described above, and the protecting groups comprise p-alkoxy phenol. The addition of phenylenediamine and H₂O₂ generates a reactive phenylene diimine which channels to the protected hapten or the enzyme-inhibitor complex and reacts with p-alkoxy phenol protecting groups to yield exposed haptens or a reactive enzyme. The amplified signal is generated and detected as described above (*see, e.g*., U.S. Patent Nos. 5,532,138 and 5,445,944).

An exemplary protocol for performing the proximity assays described herein is provided in Example 4 of PCT Publication No. WO2009/108637.

In another aspect, the present disclosure provides kits for performing the proximity assays described above comprising: (a) a dilution series of one or a plurality of capture antibodies restrained on a solid support; and (b) one or a plurality of detection antibodies (*e.g*., a combination of activation state-independent antibodies and activation state-dependent antibodies for detecting activation levels and/or a combination of first and second activation state-independent antibodies for detecting expression levels). In some instances, the kits can further contain instructions for methods of using the kit to detect the expression and/or activation status of one or a plurality of signal transduction molecules of cells such as tumor cells. The kits may also contain any of the additional reagents described above with respect to performing the specific methods of the present disclosure such as, for example, first and second members of the signal amplification pair, tyramide signal amplification reagents, substrates for the facilitating moiety, wash buffers, *etc.*

### VI. Methods of Genotyping

A variety of means can be used to genotype an individual at a polymorphic site in one or more genes such as oncogenes and/or tumor suppressor genes to determine whether a sample (*e.g*., a nucleic acid sample) contains a specific variant allele (*e.g*., somatic mutation) and/or haplotype. For example, enzymatic amplification of nucleic acid from an individual can be conveniently used to obtain nucleic acid for subsequent analysis. The presence or absence of a specific variant allele (*e.g*., somatic mutation) or haplotype in one or more genes of interest can also be determined directly from the individual's nucleic acid without enzymatic amplification. In certain aspects, an individual is genotyped at one, two, three, four, five, six, or more polymorphic sites such as a single nucleotide polymorphism (SNP) in one or more genes of interest.

Genotyping of nucleic acid from an individual, whether amplified or not, can be performed using any of various techniques. Useful techniques include, without limitation, assays such as polymerase chain reaction (PCR) based analysis assays, sequence analysis assays, electrophoretic analysis assays, restriction length polymorphism analysis assays, hybridization analysis assays, allele-specific hybridization, oligonucleotide ligation allele-specific elongation/ligation, allele-specific amplification, single-base extension, molecular inversion probe, invasive cleavage, selective termination, restriction length polymorphism, sequencing, single strand conformation polymorphism (SSCP), single strand chain polymorphism, mismatch-cleaving, and denaturing gradient gel electrophoresis, all of which can be used alone or in combination. As used herein, the term "nucleic acid" includes a polynucleotide such as a single- or double-stranded DNA or RNA molecule including, for example, genomic DNA, cDNA and mRNA. This term encompasses nucleic acid molecules of both natural and synthetic origin as well as molecules of linear, circular, or branched configuration representing either the sense or antisense strand, or both, of a native nucleic acid molecule. It is understood that such nucleic acids can be unpurified, purified, or attached, for example, to a synthetic material such as a bead or column matrix.

In particular aspects, the presence or absence of a variant allele (*e.g*., somatic mutation) in one or more genes such as oncogenes and/or tumor suppressor genes is determined using a genotyping assay as described in WO2013/026027, which claims priority from U.S. Provisional Application No. 61/525,137, filed August 18, 2011, and U.S. Provisional Application No. 61/588,151, filed January 18, 2012.

Material containing nucleic acid is routinely obtained from individuals. Such material is any biological matter from which nucleic acid can be prepared. As non-limiting examples, material can be whole blood, serum, plasma, saliva, cheek swab, sputum, or other bodily fluid or tissue that contains nucleic acid. In one aspect, a method of the present disclosure is practiced with whole blood, which can be obtained readily by non-invasive means and used to prepare genomic DNA. In another aspect, genotyping involves amplification of an individual's nucleic acid using the polymerase chain reaction (PCR). Use of PCR for the amplification of nucleic acids is well known in the art (*see, e.g*., Mullis et al. (Eds.), The Polymerase Chain Reaction, Birkhäuser, Boston, (1994)). In yet another aspect, PCR amplification is performed using one or more fluorescently labeled primers. In a further aspect, PCR amplification is performed using one or more labeled or unlabeled primers that contain a DNA minor groove binder.

Any of a variety of different primers can be used to amplify an individual's nucleic acid by PCR in order to determine the presence or absence of a variant allele (*e.g*., somatic mutation) in a method of the disclosure. As understood by one skilled in the art, primers for PCR analysis can be designed based on the sequence flanking the polymorphic site(s) of interest in the gene of interest. As a non-limiting example, a sequence primer can contain from about 15 to about 30 nucleotides of a sequence upstream or downstream of the polymorphic site of interest in the gene of interest. Such primers generally are designed to have sufficient guanine and cytosine content to attain a high melting temperature which allows for a stable annealing step in the amplification reaction. Several computer programs, such as Primer Select, are available to aid in the design of PCR primers.

A Taqman® allelic discrimination assay available from Applied Biosystems can be useful for genotyping an individual at a polymorphic site to thereby determine the presence or absence of a particular variant allele (*e.g*., somatic mutation) or haplotype in the gene of interest. In a Taqman® allelic discrimination assay, a specific fluorescent dye-labeled probe for each allele is constructed. The probes contain different fluorescent reporter dyes such as FAM and VIC™ to differentiate amplification of each allele. In addition, each probe has a quencher dye at one end which quenches fluorescence by fluorescence resonance energy transfer. During PCR, each probe anneals specifically to complementary sequences in the nucleic acid from the individual. The 5' nuclease activity of Taq polymerase is used to cleave only probe that hybridizes to the allele. Cleavage separates the reporter dye from the quencher dye, resulting in increased fluorescence by the reporter dye. Thus, the fluorescence signal generated by PCR amplification indicates which alleles are present in the sample. Mismatches between a probe and allele reduce the efficiency of both probe hybridization and cleavage by Taq polymerase, resulting in little to no fluorescent signal. Those skilled in the art understand that improved specificity in allelic discrimination assays can be achieved by conjugating a DNA minor groove binder (MGB) group to a DNA probe as described, *e.g*., in Kutyavin et al., Nuc. Acids Research 28:655-661 (2000). Minor groove binders include, but are not limited to, compounds such as dihydrocyclopyrroloindole tripeptide (DPI3).

Sequence analysis can also be useful for genotyping an individual according to the methods described herein to determine the presence or absence of a particular variant allele (*e.g*., somatic mutation) or haplotype in the gene of interest. As is known by those skilled in the art, a variant allele of interest can be detected by sequence analysis using the appropriate primers, which are designed based on the sequence flanking the polymorphic site of interest in the gene of interest. For example, a variant allele in a gene of interest can be detected by sequence analysis using primers designed by one of skill in the art. Additional or alternative sequence primers can contain from about 15 to about 30 nucleotides of a sequence that corresponds to a sequence about 40 to about 400 base pairs upstream or downstream of the polymorphic site of interest in the gene of interest. Such primers are generally designed to have sufficient guanine and cytosine content to attain a high melting temperature which allows for a stable annealing step in the sequencing reaction.

The term "sequence analysis" includes any manual or automated process by which the order of nucleotides in a nucleic acid is determined. As an example, sequence analysis can be used to determine the nucleotide sequence of a sample of DNA. The term sequence analysis encompasses, without limitation, chemical and enzymatic methods such as dideoxy enzymatic methods including, for example, Maxam-Gilbert and Sanger sequencing as well as variations thereof. The term sequence analysis further encompasses, but is not limited to, capillary array DNA sequencing, which relies on capillary electrophoresis and laser-induced fluorescence detection and can be performed using instruments such as the MegaBACE 1000 or ABI 3700. As additional non-limiting examples, the term sequence analysis encompasses thermal cycle sequencing (*see*, Sears et al., Biotechniques 13:626-633 (1992)); solid-phase sequencing (*see*, Zimmerman et al., Methods Mol. Cell Biol. 3:39-42 (1992); and sequencing with mass spectrometry, such as matrix-assisted laser desorption/ionization time-of-flight mass spectrometry (*see*, MALDI-TOF MS; Fu et al., Nature Biotech. 16:381-384 (1998)). The term sequence analysis further includes, but is not limited to, sequencing by hybridization (SBH), which relies on an array of all possible short oligonucleotides to identify a segment of sequence (*see*, Chee et al., Science 274:610-614 (1996); Drmanac et al., Science 260:1649-1652 (1993); and Drmanac et al., Nature Biotech. 16:54-58 (1998)). One skilled in the art understands that these and additional variations are encompassed by the term sequence analysis as defined herein.

Electrophoretic analysis also can be useful in genotyping an individual according to the methods of the present disclosure to determine the presence or absence of a particular variant allele (*e.g*., somatic mutation) or haplotype in the gene of interest. "Electrophoretic analysis" as used herein in reference to one or more nucleic acids such as amplified fragments includes a process whereby charged molecules are moved through a stationary medium under the influence of an electric field. Electrophoretic migration separates nucleic acids primarily on the basis of their charge, which is in proportion to their size, with smaller molecules migrating more quickly. The term electrophoretic analysis includes, without limitation, analysis using slab gel electrophoresis, such as agarose or polyacrylamide gel electrophoresis, or capillary electrophoresis. Capillary electrophoretic analysis generally occurs inside a small-diameter (50-100 m) quartz capillary in the presence of high (kilovolt-level) separating voltages with separation times of a few minutes. Using capillary electrophoretic analysis, nucleic acids are conveniently detected by UV absorption or fluorescent labeling, and single-base resolution can be obtained on fragments up to several hundred base pairs. Such methods of electrophoretic analysis, and variations thereof, are well known in the art, as described, for example, in Ausubel et al., Current Protocols in Molecular Biology Chapter 2 (Supplement 45) John Wiley & Sons, Inc. New York (1999).

Restriction fragment length polymorphism (RFLP) analysis can also be useful for genotyping an individual according to the methods of the present disclosure to determine the presence or absence of a particular variant allele (*e.g*., somatic mutation) or haplotype in the gene of interest (*see*, Jarcho *et al. in* Dracopoli et al., Current Protocols in Human Genetics pages 2.7.1-2.7.5, John Wiley & Sons, New York; Innis et al., (Ed.), PCR Protocols, San Diego: Academic Press, Inc. (1990)). As used herein, "restriction fragment length polymorphism analysis" includes any method for distinguishing polymorphic alleles using a restriction enzyme, which is an endonuclease that catalyzes degradation of nucleic acid following recognition of a specific base sequence, generally a palindrome or inverted repeat. One skilled in the art understands that the use of RFLP analysis depends upon an enzyme that can differentiate a variant allele from a wild-type or other allele at a polymorphic site.

In addition, allele-specific oligonucleotide hybridization can be useful for genotyping an individual in the methods described herein to determine the presence or absence of a particular variant allele (*e.g*., somatic mutation) or haplotype in the gene of interest. Allele-specific oligonucleotide hybridization is based on the use of a labeled oligonucleotide probe having a sequence perfectly complementary, for example, to the sequence encompassing the variant allele. Under appropriate conditions, the variant allele-specific probe hybridizes to a nucleic acid containing the variant allele but does not hybridize to the one or more other alleles, which have one or more nucleotide mismatches as compared to the probe. If desired, a second allele-specific oligonucleotide probe that matches an alternate (*e.g*., wild-type) allele can also be used. Similarly, the technique of allele-specific oligonucleotide amplification can be used to selectively amplify, for example, a variant allele by using an allele-specific oligonucleotide primer that is perfectly complementary to the nucleotide sequence of the variant allele but which has one or more mismatches as compared to other alleles (Mullis *et al., supra*)*.* One skilled in the art understands that the one or more nucleotide mismatches that distinguish between the variant allele and other alleles are often located in the center of an allele-specific oligonucleotide primer to be used in the allele-specific oligonucleotide hybridization. In contrast, an allele-specific oligonucleotide primer to be used in PCR amplification generally contains the one or more nucleotide mismatches that distinguish between the variant and other alleles at the 3' end of the primer.

A heteroduplex mobility assay (HMA) is another well-known assay that can be used for genotyping in the methods of the present disclosure to determine the presence or absence of a particular variant allele (*e.g*., somatic mutation) or haplotype in the gene of interest. HMA is useful for detecting the presence of a variant allele since a DNA duplex carrying a mismatch has reduced mobility in a polyacrylamide gel compared to the mobility of a perfectly base-paired duplex (*see*, Delwart et al., Science, 262:1257-1261 (1993); White et al., Genomics, 12:301-306 (1992)).

The technique of single strand conformational polymorphism (SSCP) can also be useful for genotyping in the methods described herein to determine the presence or absence of a particular variant allele (*e.g*., somatic mutation) or haplotype in the gene of interest (*see*, Hayashi, Methods Applic., 1:34-38 (1991)). This technique is used to detect variant alleles based on differences in the secondary structure of single-stranded DNA that produce an altered electrophoretic mobility upon non-denaturing gel electrophoresis. Variant alleles are detected by comparison of the electrophoretic pattern of the test fragment to corresponding standard fragments containing known alleles.

Denaturing gradient gel electrophoresis (DGGE) can also be useful in the methods of the disclosure to determine the presence or absence of a particular variant allele (*e.g*., somatic mutation) or haplotype in the gene of interest. In DGGE, double-stranded DNA is electrophoresed in a gel containing an increasing concentration of denaturant; double-stranded fragments made up of mismatched alleles have segments that melt more rapidly, causing such fragments to migrate differently as compared to perfectly complementary sequences (*see*, Sheffield et al., "Identifying DNA Polymorphisms by Denaturing Gradient Gel Electrophoresis" in Innis *et al*., *supra*, 1990).

Other molecular methods useful for genotyping an individual are known in the art and useful in the methods of the present disclosure. Such well-known genotyping approaches include, without limitation, automated sequencing and RNase mismatch techniques (*see*, Winter et al., Proc. Natl. Acad. Sci., 82:7575-7579 (1985)). Furthermore, one skilled in the art understands that, where the presence or absence of multiple variant alleles is to be determined, individual variant alleles can be detected by any combination of molecular methods. *See*, in general, Birren et al. (Eds.) Genome Analysis: A Laboratory Manual Volume 1 (Analyzing DNA) New York, Cold Spring Harbor Laboratory Press (1997). In addition, one skilled in the art understands that multiple variant alleles can be detected in individual reactions or in a single reaction (a "multiplex" assay).

### VII. Production of Antibodies

The generation and selection of antibodies not already commercially available for analyzing the levels of expression and activation of signal transduction molecules in tumor cells in accordance with the immunoassays of the present disclosure can be accomplished several ways. For example, one way is to express and/or purify a polypeptide of interest (*i.e*., antigen) using protein expression and purification methods known in the art, while another way is to synthesize the polypeptide of interest using solid phase peptide synthesis methods known in the art. *See, e.g.,* Guide to Protein Purification, Murray P. Deutcher, ed., Meth. Enzymol., Vol. 182 (1990); Solid Phase Peptide Synthesis, Greg B. Fields, ed., Meth. Enzymol., Vol. 289 (1997); Kiso et al., Chem. Pharm. Bull., 38:1192-99 (1990); Mostafavi et al., Biomed. Pept. Proteins Nucleic Acids, 1:255-60, (1995); and Fujiwara et al., Chem. Pharm. Bull., 44:1326-31 (1996). The purified or synthesized polypeptide can then be injected, for example, into mice or rabbits, to generate polyclonal or monoclonal antibodies. One skilled in the art will recognize that many procedures are available for the production of antibodies, for example, as described in Antibodies, A Laboratory Manual, Harlow and Lane, Eds., Cold Spring Harbor Laboratory, Cold Spring Harbor, N.Y. (1988). One skilled in the art will also appreciate that binding fragments or Fab fragments which mimic (*e.g*., retain the functional binding regions of) antibodies can also be prepared from genetic information by various procedures. *See, e.g.,* Antibody Engineering: A Practical Approach, Borrebaeck, Ed., Oxford University Press, Oxford (1995); and Huse et al., J. Immunol., 149:3914-3920 (1992).

Those skilled in the art will recognize that many approaches can be taken in producing antibodies or binding fragments and screening and selecting for affinity and specificity for the various polypeptides of interest, but these approaches do not change the scope of the present invention.

A more detailed description of polyclonal antibodies, monoclonal antibodies, humanized antibodies, human antibodies, bispecific antibodies, fragments thereof, and methods of purifying antibodies is found in PCT Publication No. WO 2010/132723.

One of skill in the art will appreciate that any binding molecule having a function similar to an antibody, *e.g*., a binding molecule or binding partner which is specific for one or more analytes of interest in a sample, can also be used in the methods of the present disclosure. Examples of suitable antibody-like molecules include, but are not limited to, domain antibodies, unibodies, nanobodies, shark antigen reactive proteins, avimers, adnectins, anticalms, affinity ligands, phylomers, aptamers, affibodies, trinectins, and the like.

### VIII. Methods of Administration

According to the methods of the present disclosure, the anticancer drugs described herein are administered to a subject by any convenient means known in the art. The methods of the present disclosure can be used to predict the therapeutic efficacy of an anticancer drug or a combination of anticancer drugs in a subject having a lung tumor. The methods of the present disclosurecan also be used to predict the response of a lung tumor to treatment with an anticancer drug or a combination of anticancer drugs. The methods of the disclosure can also be used to select or identify a suitable anticancer drug or a combination of anticancer drugs for the treatment of a lung tumor. One skilled in the art will appreciate that one or more anticancer drugs described herein can be administered alone or as part of a combined therapeutic approach with conventional chemotherapy, radiotherapy, hormonal therapy, immunotherapy, and/or surgery.

In certain aspects, the anticancer drug comprises an anti-signaling agent (*i.e*., a cytostatic drug) such as a monoclonal antibody or a tyrosine kinase inhibitor; an anti-proliferative agent; a chemotherapeutic agent (*i.e*., a cytotoxic drug); a hormonal therapeutic agent; a radiotherapeutic agent; a vaccine; and/or any other compound with the ability to reduce or abrogate the uncontrolled growth of aberrant cells such as cancerous cells. In some aspects, the subject is treated with one or more anti-signaling agents, anti-proliferative agents, and/or hormonal therapeutic agents in combination with at least one chemotherapeutic agent. Exemplary monoclonal antibodies, tyrosine kinase inhibitors, anti-proliferative agents, chemotherapeutic agents, hormonal therapeutic agents, radiotherapeutic agents, and vaccines are described above.

In some apsects, the anticancer drugs described herein can be co-administered with conventional immunotherapeutic agents including, but not limited to, immunostimulants (*e.g*., Bacillus Calmette-Guérin (BCG), levamisole, interleukin-2, alpha-interferon, *etc.*), immunotoxins (*e.g*., anti-CD33 monoclonal antibody-calicheamicin conjugate, anti-CD22 monoclonal antibody-pseudomonas exotoxin conjugate, *etc.*), and radioimmunotherapy (*e.g*., anti-CD20 monoclonal antibody conjugated to ¹¹¹In, ⁹⁰Y, or ¹³¹I, *etc.*).

Anticancer drugs can be administered with a suitable pharmaceutical excipient as necessary and can be carried out via any of the accepted modes of administration. Thus, administration can be, for example, oral, buccal, sublingual, gingival, palatal, intravenous, topical, subcutaneous, transcutaneous, transdermal, intramuscular, intra-joint, parenteral, intra-arteriole, intradermal, intraventricular, intracranial, intraperitoneal, intravesical, intrathecal, intralesional, intranasal, rectal, vaginal, or by inhalation. By "co-administer" it is meant that an anticancer drug is administered at the same time, just prior to, or just after the administration of a second drug (*e.g*., another anticancer drug, a drug useful for reducing the side-effects associated with anticancer drug therapy, a radiotherapeutic agent, a hormonal therapeutic agent, an immunotherapeutic agent, *etc.*).

A therapeutically effective amount of an anticancer drug may be administered repeatedly, *e.g*., at least 2, 3, 4, 5, 6, 7, 8, or more times, or the dose may be administered by continuous infusion. The dose may take the form of solid, semi-solid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" refers to physically discrete units suitable as unitary dosages for human subjects and other mammals, each unit containing a predetermined quantity of an anticancer drug calculated to produce the desired onset, tolerability, and/or therapeutic effects, in association with a suitable pharmaceutical excipient (*e.g*., an ampoule). In addition, more concentrated dosage forms may be prepared, from which the more dilute unit dosage forms may then be produced. The more concentrated dosage forms thus will contain substantially more than, *e.g*., at least 1, 2, 3, 4, 5, 6, 7, 8, 9, 10, or more times the amount of the anticancer drug.

Methods for preparing such dosage forms are known to those skilled in the art (*see*, *e.g.,* REMINGTON'S PHARMACEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e.g*., *REMINGTON'S PHARMACEUTICAL SCIENCES, supra*)*.*

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g*., Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e*., the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

In some aspects, the therapeutically effective dose takes the form of a pill, tablet, or capsule, and thus, the dosage form can contain, along with an anticancer drug, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof. An anticancer drug can also be formulated into a suppository disposed, for example, in a polyethylene glycol (PEG) carrier.

Liquid dosage forms can be prepared by dissolving or dispersing an anticancer drug and optionally one or more pharmaceutically acceptable adjuvants in a carrier such as, for example, aqueous saline (*e.g*., 0.9% w/v sodium chloride), aqueous dextrose, glycerol, ethanol, and the like, to form a solution or suspension, *e.g*., for oral, topical, or intravenous administration. An anticancer drug can also be formulated into a retention enema.

For topical administration, the therapeutically effective dose can be in the form of emulsions, lotions, gels, foams, creams, jellies, solutions, suspensions, ointments, and transdermal patches. For administration by inhalation, an anticancer drug can be delivered as a dry powder or in liquid form via a nebulizer. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, *e.g*., bicarbonate, for reconstitution prior to administration, or the buffer may be included in the lyophilized dosage form for reconstitution with, *e.g*., water. The lyophilized dosage form may further comprise a suitable vasoconstrictor, *e.g*., epinephrine. The lyophilized dosage form can be provided in a syringe, optionally packaged in combination with the buffer for reconstitution, such that the reconstituted dosage form can be immediately administered to a subject.

A subject can also be monitored at periodic time intervals to assess the efficacy of a certain therapeutic regimen. For example, the activation states of certain signal transduction molecules may change based on the therapeutic effect of treatment with one or more of the anticancer drugs described herein. The subject can be monitored to assess response and understand the effects of certain drugs or treatments in an individualized approach. Additionally, subjects who initially respond to a specific anticancer drug or combination of anticancer drugs may become refractory to the drug or drug combination, indicating that these subjects have developed acquired drug resistance. These subjects can be discontinued on their current therapy and an alternative treatment prescribed in accordance with the methods of the present invention.

### IX. Example

The following example is offered to illustrate, but not to limit, the claimed invention.

### Example 1. Signatures of Drug Sensitivity in Non-Small Cell Lung Cancer.

This example describes the profiling of receptor tyrosine kinase pathway activation and gene mutations in eight human lung tumor cell lines and 50 human lung tumor tissue samples to define molecular pathways. A panel of eight kinase inhibitors was used to determine whether blocking pathway activation affected tumor cell growth. The HER1 pathway in HER1 mutant cell lines HCC827 and H1975 was found to be highly activated and sensitive to HER1 inhibition. H1993 is a c-MET amplified cell line showing c-MET and HER1 pathway activation and responsiveness to c-MET inhibitor treatment. IGF-1R pathway activated H358 and A549 cells are sensitive to IGF-1R inhibition. The downstream PI3K inhibitor, BEZ-235, effectively inhibited tumor cell growth in most of the cell lines tested, except the H1993 and H1650 cells, while the MEK inhibitor PD-325901 was effective in blocking the growth of KRAS mutated cell line H1734, but not H358, A549 and H460. Hierarchical clustering of primary tumor samples with the corresponding tumor cell lines based on their pathway signatures revealed similar profiles for HER1, c-MET and IGF-1R pathway activation and predict potential treatment options for the primary tumors based on the tumor cell lines response to the panel of kinase inhibitors.

The disclosure of Gong et al., Int. J. Proteomics, Vol. 2011, Article ID 215496 (2011) is hereby cited.

### MATERIALS AND METHODS

### Human lung tumor cell lines, lung cancer tissue samples and kinase inhibitors:

Eight NSCLC cell lines: HCC827, H1975, H1734, H1993, H358, H1650, A549, and H460 were selected and represent the major NSCLC cancer subtypes, adenocarcinoma and large-cell lung carcinoma. The cell lines were purchased from ATCC (Table 3). Fifty lung adenocarcinoma samples were collected from patients operated on for lung cancer at the University of Michigan. Collection and use of all tissue samples were approved by the Human Subjects Institutional Review Boards of the University of Michigan. The demographic information of the patients is shown in Table 4. The primary tumor samples were snap frozen and cryostat-sectioned to identify regions representing >70% tumor cellularity for subsequent pathway analysis. The samples (∼2 cubic millimeters in size) were shipped to Prometheus Laboratories on dry ice for analysis. Eight kinase inhibitors representing a diverse panel of potential cancer therapeutics were purchased from Selleck Chemicals (Houston, TX). The collection included specific as well as multiple RTK inhibitors, i.e., compounds targeting the cellular kinase pathways: Her1/2/4 (epidermal growth factor receptors) inhibitors (Erlotinib for Her1, Lapatinib for Her1/2, Gefitinib for Her1/2/4, and BIBW-2992 is an irreversible inhibitor for Her1/2), c-Met (hepatocyte growth factor receptor) inhibitor, PF-2341066, IGF-1R (insulin-like growth factor-1 receptor) inhibitor BMS-536924, MEK (mitogen-activated protein kinase kinase) inhibitor, PD-325901 and PI3K (phosphatidylinositol-3-kinase) and mTOR (mammalian target of rapamycin) inhibitor BEZ-235.

**Table 3. Clinical features of the eight lung tumor cell lines.**

| **Name** | **ATCC Cat. No.** | **Type** | **Source** |
|---|---|---|---|
| HCC827 | CRL-2868 | Adenocarcinoma | Primary, lung epithelial |
| H1975 | CRL-5908 | Adenocarcinoma | Primary, lung epithelial |
| H1734 | CRL-5891 | Adenocarcinoma | Primary, lung epithelial |
| H1993 | CRL-5909 | Adenocarcinoma | Metastatic, lymph node |
| H358 | CRL-5807 | Bronchioalveolar carcinoma | Primary, lung epithelial |
| H1650 | CRL-5883 | Bronchioalveolar carcinoma | Metastatic, pleural effusion |
| A549 | CCL-185 | Large-cell carcinoma | Primary, lung epithelial |
| H460 | HTB-177 | Large-cell carcinoma | Metastatic, pleural effusion |

**Table 4. Clinical Characteristics of 50 human lung tumors.**

| **Average Age of Patients (range)** | 68.2 (51-90) |
|---|---|
| **Gender** | |
| Female | 24 |
| Male | 26 |
| **Tumor Stage** | |
| I | 32 |
| II | 7 |
| III-IV | 11 |
| **Current Status of Patients** | |
| Dead | 23 |
| Alive | 27 |

### Preparation of lysates from cell lines and primary tumor samples:

Tumor cells were cultured in their respective growth medium recommended by ATCC plus 10% fetal bovine serum (FBS). Cells were grown in 35-mm 6-well cell culture plates until reaching 80% confluence. After washing the cells with phosphate buffered saline (PBS) 3 times, the cell culture plate was placed on ice and then the plate was carefully tilted on its side for 10 sec to completely remove all residual media. Then, 150 µL of ice cold lysis buffer was added to each plate and the plate then left on ice for 5 min. The lysed cells were scraped off and together with the crude lysate transferred to a 1.5 mL centrifuge tube. The mixture was vortexed in the tube, placed on ice for 15 min and then centrifuged at 14,000 rpm for 15 min at 4°C. The supernatant was transferred to another centrifuge tube and stored at -70°C until analysis. The frozen tumor samples were similarly processed by the addition of 4 volumes of ice cold lysis buffer per tissue volume and homogenized in a Powergren High Throughput Homogenizer (Fisher Scientific) at a speed setting of 7 for 2 min. The homogenate was transferred to a 1.5 mL centrifuge tube and centrifuged at 14,000 rpm for 15 min at 4°C. The supernatant from the tumor lysate was harvested and stored at -70°C until analysis.

### Profiling of signaling pathways using the CEER™ assay in tumor cell lines and tissue samples:

The principal of the CEER™ assay is based on the capture of the target protein by a target-specific antibody printed in two dilutions on the surface of a microarray slide. Measurement of the activation status of the captured target protein is revealed by the formation of a unique immuno-complex, requiring the co-localization of two detecting enzyme-conjugated antibodies on the same target protein captured on the microarray surface as illustrated in Figure 7. Formation of this complex is initiated by the binding of the first detecting antibody, which is coupled to glucose oxidase (GO), to an epitope on the captured target protein that is different from the epitope recognized by the capture antibody, followed by the binding of a second detecting antibody, which is coupled to horseradish peroxidase (HRP), to a phosphorylated tyrosine (p-Tyr) residue on the target protein. Upon the addition of glucose, the immobilized GO on the captured target protein produces H₂O₂ and due to the close proximity, the locally generated H₂O₂ is then utilized by the HRP coupled to the p-Tyr-specific second detecting antibody to generate a chemical signal that can be amplified with biotinyl-tyramide. The sensitivity and specificity for the detection of the phosphorylated target protein is greatly enhanced by this collaborative reaction and amplification process, which is mediated by the simultaneous binding of three different antibodies on the same target protein.

### Inhibition of activated signaling pathways in tumor cell lines by kinase inhibitors:

The tumor cells were cultured in their respective growth medium with 10% FBS in 35mm 6-well cell culture plates until they reached ∼80% confluence. The cells were then starved overnight in serum-free medium, followed by a 4-hour treatment with various concentrations of the kinase inhibitor. Afterwards, cell lysates were prepared from the treated cells as before and aliquots of the lysates subjected to the CEER™ assay.

### Inhibition of tumor cell line growth by kinase inhibitors:

The tumor cells were seeded into 96-well cell culture plates and maintained in culture for 24 hours. After washing, the cultured cells were incubated in their respective medium containing 5% FBS and various concentrations of the indicated inhibitor for 48 hr. Determination of tumor cell growth inhibition was performed by adding 100 µL of the combined Cell Titer-Glo® Buffer and Cell Titer-Glo® Substrate Labeling Reagent (Promega) to each well of the plates, followed by incubation at room temperature for 10 min to stabilize the luminescence. The luminescent signal from the cell samples was detected by using an M5 micro-titer plate reader. For studies involving treatment with more than one inhibitor, the selected inhibitor that showed more than 25% inhibition of tumor cell growth at 10 µM concentration when treated individually was further tested in combination treatment with another inhibitor. A 5 µM concentration of each inhibitor was combined to make a 10 µM dose, and the same half log dilution was made as in the single drug treatment for adding to the cells. Tumor cells were treated for 48 hr and cell viability was measured as in the single inhibitor treatment.

### Anchorage-independent inhibition of tumor cell line growth by kinase inhibitors:

A single cell suspension of 3000 cells from each of the eight tumor cell lines in 1 mL mixture of 1.2% Agarose (Seaplaque; FMC, Rockland, ME) in DMEM (Life Technologies, Carlsbad, CA) plus 10% FBS was added on top of 1% soft agarose that had been allowed to gel previously in the wells of a 35-mm 6-well cell culture plate. The plates were kept at 4°C for 2 hr to solidify the cell-containing layer. The plates were then incubated at 37°C in a CO₂ incubator with 2 mL of medium containing various concentrations of the kinase inhibitor. The medium with the inhibitor was changed every 3 days. After 2 weeks, cell colonies larger than 10 cells were scored under a Nikon inverted-phase microscope [Gong et al., Cancer Res., 59(24):6239-45 (1999)].

### Genotyping of lung tumor tissue samples:

Genomic DNA was isolated from human tumor tissue samples using the DNeasy kit (Qiagen, Valencia, CA). Primers and probes for all of the measured SNPs were obtained from the ABI TaqMan® SNP Genotyping Assay (Applied Biosystems), using the Assay-by-Design service for which we provided the sequences, or the Assay-on-Demand service when the assays were already designed by Applied Biosystems. Reactions were performed in 5 µL volume and contained 10 ng DNA, 1x TaqMan Universal Mastermix (Applied Biosystems), 200 nM of each probe and 900 nM of each primer. Cycling conditions on the ABI PRISM® 7900HT Sequence Detection System (Applied Biosystems) were 10 min at 95°C, followed by 40 cycles of 15 sec at 95°C and 1 min at 60°C. After cycling, the end-point fluorescence was measured and the amplified sequences determined by DNA sequencing analysis. Alleles were assigned using the SDS 2.1 software (Applied Biosystems).

### Lung tumor tissue sample clustering analysis and heat map generation:

Hierarchical clustering analysis was performed on the 50 lung tumor tissue samples to explore whether the pathway activation profiles determined by the CEER™ assay and the gene mutational analysis done for these samples could segregate them into distinct subsets that are similar to the pathway activation and mutational signatures of the tumor cell lines. The general construction of a hierarchical agglomerative classification was achieved by using an algorithm to find the two closest objects and merge them into a cluster, and then find and merge the next two closest points, where a point is either an individual object or a cluster of objects. A heat map of one-dimensional hierarchical clustering result was generated in the analysis to demonstrate the sample clustering structure based on pathway activation signatures and mutational status.

### CEER™ assay procedure for profiling of signaling pathways in tumor cell lines:

The assay was performed by printing 500 pL of a commercially available capture antibody per spot at two dilution concentrations of 0.5 and 1.0 mg/mL in triplicates to yield six spots per row for each capture antibody on a 3X2 cm nitrocellulose-coated glass slide (FAST®, Whatman) using a Nano-Plotter NP2.1 printer (GeSIM mbH, Germany) as illustrated in Figure 8, upper panel. The capture antibodies against mouse IgG, human epidermal growth factor receptor 1 (Her1), human epidermal growth factor receptor 2 (Her2), human epidermal growth factor receptor 3 (Her3), human hepatocyte growth factor receptor (cMET), human insulin-like growth factor-1 receptor (IGF1R), human stem cell growth factor receptor (cKit), human phosphatidylinositol-3-kinase (PI3K), human Src homology 2 domain-containing protein (SHC) and human cytokeratin (CK), were printed in this order in 10 rows from top to bottom in each slide. In the assay of the tumor tissue samples, printing of the capture antibody against cKit was omitted because activation of this signaling pathway was not observed in any of the lung cancer cell lines. The anti-mouse-IgG printed on the first row was used as a negative control while the pan-cytokeratin capture antibody printed on the last row was used as a positive control. After printing, the slides were rinsed 2 times with TBST (50 mM Tris/150 mM NaCl/0.1% Tween-20, pH 7.2-7.4), blocked with 80 µL of Whatman Blocking Buffer (Whatman) for 1 hr at room temperature (RT), and then washed 2 times with TBST. For measurement of the activated pathways in the lung cell lines, serially diluted cell lysates in 80 µL of reaction buffer (2% bovine serum albumin (BSA)/0.1% Triton X-100/TBS [TBST without Tween-20], pH 7.2-7.4) were added to the slides according to the number of cells' lysate being assayed as illustrated in Figure 8, left panel, and incubated for 1 hr at RT. After incubation, the slides were washed 4 times with TBST, 3 min each time. Then a combination of each pair of respective commercially available detecting antibodies for each of the targeted kinases, one conjugated to GO and the other to HRP, were added in 80 µL of the reaction buffer followed by incubation for 2 hrs at RT. The unbound detecting antibody conjugates were removed by washing with TBST. Then, 80 µL of the signal generation and amplification solution (5 µg/mL biotinyl tyramide [Perkin Elmer Life Sciences] in 50 mM glucose in PBS) was added to each slide and incubated for 15 min in the dark. The slides were then washed with TBST 4 times, with 3 min each time. The local deposition of the generated biotin-tyramide signal was revealed upon the addition of a signal-detecting reagent, streptavidin-labeled Alexa647 (Invitrogen) in PBS at 0.5 µg/mL (1:4000 dilution) in 2% BSA/0.1% Triton/TBS and incubated for 40 min. Upon completion of the incubation, the slides were washed 4 times with TBST, dried, and kept in the dark until scanning on the microarray scanner. Each slide was scanned at three photomultiplier gain settings to increase the effective dynamic range. Background corrected signal intensities were averaged for replicate spots printed in triplicate. The relative fluorescence value of the respective reagent blank was subtracted from each sample to give a relative fluorescence unit (RFU) according the number of cells' extract. The calculated RFUs with the corresponding number of cells' extracts were used to generate the dose-response pathway activation curves for each cell line.

### CEER™ assay procedure for profiling of signaling pathways in tumor tissue samples:

For comparison of the relative levels of signaling pathway activation within the 50 tumor tissue samples and the eight tumor cell lines, we used a mixture of three cell lines, BT474, T47D and HCC827, as a standard to generate the dose-response curves for each of the activated pathways profiled by the CEER™ assay. BT474 is a breast cancer cell line from ATCC (Cat. No. HTB-20) exhibiting a strongly activated Her2 pathway. T47D is another breast cancer cell line from ATCC (Cat. No. HTB-133) that showed robustly activated Her3 and IGF1R pathways while, HCC827, a lung tumor cell line employed in the present study was selected because it exhibited a highly activated Her1 and cMET pathway. To generate the standard curves, lysates corresponding to 300 BT474 cells, which had been pretreated with 10 nM Heregulin for 15 min, 1000 T47D cells, which had been pretreated with 100 ng/mL IGF-1 for 15 min, and 1000 HCC827 cells, which had been pretreated with 100 nM EGF for 15 min, were combined to give the starting standard cell lysate concentration, which was then serially diluted to yield a total of six concentrations based on the original number of cells employed to produce the lysate. Each of the original and diluted cell lysate concentrations, according to decreasing cell numbers, was added to the six capture antibody pre-printed slides on the top and bottom two rows in the panel with one lysate concentration per slide as illustrated in Figure 8, right panel. By combining these three cell lysates, we generated signals to cover most of the activated pathways detected in the tumor cell lines. Each subsequent two rows of slides in the middle of the panel were loaded with extracts from the tumor tissue samples or tumor cell lines with the top row corresponding to 10 µg and the bottom row corresponding to 1 µg protein concentration and one sample per column. After loading of the slides in each panel, they were subjected to the CEER™ assay as described before and the generated data from the lysate standards were fitted to a five parameter equation by nonlinear regression, simultaneously fitting two dilutions of the capture antibody, to generate the standard curves for each pathway. The individual computed cell unit (CU) for the tumor tissue samples or cell lines in each pathway was determined from the standard curves.

### Method for preparation of the antibody/GO conjugate and the anti-p-Tyr/HRP conjugate:

The antibody/enzyme conjugate was linked through a dextran molecule incorporated with free sulfhydryl groups. The sulfhydryl-incorporated dextran was prepared by first converting some of the hydroxyl groups on a 500 KDa dextran molecule to carboxymethyl ether groups through reaction with bromoacetic acid in a sodium hydroxide solution. The incorporated carboxymethyl grouping was then coupled to the amino functional group of cysteamine (HSCH₂CH₂NH₂) through water soluble carbodiimide to yield the sulfhydryl-incorporated dextran. To conjugate the respective antibody and enzyme to the sulfhydryl-incorporated dextran, the antibody or enzyme was first activated through coupling of the free amino functional groups on the antibody or enzyme with a bi-functional cross-linker, succinimidyl-4-(N-maleimidomethyl)-cyclohexane-1-carboxylate (SMCC, Pierce) according to the manufacturer's instruction. Using a 1:5 molar ratio of antibody to enzyme, the resulting maleimidomethyl incorporated antibody and enzyme are then reacted simultaneously with the sulfhydryl incorporated dextran to form the antibody-enzyme conjugate, which was then purified by size-exclusion high-performance liquid chromatography.

### RESULTS

### Profiling of the activated kinase pathways in tumor cell lines:

The activated cell signaling pathways for eight lung tumor cell lines were profiled using the CEER™ assay. The assay measured the activated (p-Tyr) Her1, Her2, Her3, c-Met, IGF-1R, c-Kit, PI3K, and SHC levels in the cells based on the number of cells being assayed. The tumor cells were cultured in presence of 10% FBS and harvested at about 80% confluence for preparation of cell lysates and signaling pathway profiling. Serial dilutions of tumor cell lysates equivalent to 10 - 10,000 cells were assayed, and the raw data captured on the slides are shown in Figure 1A and a graphic representative of the data is shown in Figure 1B. As seen in FigurelB, the dose-response curves representing the level of activation in each RTK pathway is inversely proportional to the number of cells being assayed. The more activated the pathway, the less number of cells are needed to generate the maximal signal. Thus, the relative activation of the activated pathways can be determined based on the EC₅₀ value of the number of cells being assayed in each pathway activation curve (Table 5).

**Table 5. Relative activation levels of the RTK pathways determined by the CEER™ assay in the eight lung tumor cell lines expressed as EC₅₀ values based on the number of cells' lysate being assayed.**

| **Cell line** | **Her1** | **Her2** | **Her3** | **c-Met** | **IGF-1 R** | **PI3K** | **SHC** |
|---|---|---|---|---|---|---|---|
| HCC827 | 21 | 353 | 19,203 | 108 | 6,961 | 6,254 | |
| H1975 | | 1,390 | | 1,760 | | | 4670 |
| H1734 | 3,996 | | | | | | |
| H1993 | | 167 | 31,202 | <10 | | | 408 |
| H358 | 4,383 | 5,108 | | 547074 | 10,3267 | | |
| H1650 | 3,214 | 1,705 | | | | | |
| A549 | 1,234 | 6,169 | | 2,950 | 1,730 | | |
| H460 | | | | | 206,648 | | |

As shown in Figure 1B, each of the eight tumor cell lines exhibited a distinct RTK activation pattern. The lung adenocarcinoma cell line, HCC827, exhibited the greatest number of activated RTK pathways, with Her1, c-Met and Her2 being highly activated, PI3K being moderately activated, and Her3 as well as IGF-1R being lowly activated. The other adenocarcinoma cell line H1975 showed only moderate activation of Her2, c-Met and SHC pathways and a low activation of the IGF-1R pathway. The remaining adenocarcinoma cell line H1734 exhibited a moderate activation of the Her1 pathway and a very low activation of the Her2 and c-Met pathways. By contrast, the adenocarcinoma cell line H1993 from a metastatic tumor showed a very potent activation of the c-Met pathway, with also a high activation of the Her2 and SHC pathways, and a moderate activation of the Her3 and Her1 pathways. Both primary and metastatic bronchioalveolar carcinoma cell lines H358 and H1650 showed a moderate activation of the Her1 and Her2 pathways, with H358 also exhibited a moderate activation of the c-Met and IGF-1R pathways. The large-cell carcinoma cell line A549 exhibited a moderate activation of the Her1 and IGF-1R pathways with a low activation of the c-Met and Her2 pathways, whereas the metastatic large-cell carcinoma cell line H460 showed only a very low activation of the IGF-1R pathway.

### Inhibition of activated signaling pathways in tumor cell lines by kinase inhibitors:

Profiling of the eight tumor cell lines showed that the Her1 and Her2 pathways are highly activated in the HCC827 cells and thus treatment of these cells with an irreversible Her1/2 inhibitor BIBW-2992 should be able to block the activation of these pathways. Indeed, the data presented in Figure 2, showed that a potent dose-dependent inhibition of the Her1 and Her2 pathways in the HCC827 cells was observed by treatment with BIBW-2992. Other Her1 and/or Her2 pathway-activated cell lines, H1975 and H1650, likewise had these pathway activations blocked by the treatment with BIBW-2992. What's remarkable is that the H1975 cell line harbors the T790M and L858R mutations in the EGFR gene (information obtained from the Sanger Institute website), which confer resistance to EGFR kinase inhibitors, Gefitinib and Erlotinib, responded to the irreversible EGFR kinase inhibitor BIBW-2992. Two other additional Her1 and/or Her2 pathway-activated cell lines, H358 and H1734, also had their activation blocked by the Her1/2 kinase inhibitors, Gefitinib and Lapatinib, respectively. In the c-Met amplified cell line H1993, activation of this pathway was blocked by the treatment with PF-2341066, a c-Met kinase inhibitor. The c-Met inhibitor also inhibited the Her1 signaling pathway in this cell line, most likely due to cross-talk between the Her1 and c-Met pathways. Treatment with the IGF-1R kinase inhibitor BMS-536924 was able to block the activated IGF-1R pathway exhibited by the A549 and H460 cell lines.

### Inhibition of tumor cell line proliferation by kinase inhibitors:

Since one purpose of this study was to correlate the activated RTK pathways and gene mutations found in the tumor cell lines with the appropriate kinase inhibitors to determine whether treatment with the inhibitors could inhibit the growth of these cells, the eight tumor cell lines were treated with the selected kinase inhibitors and the results shown in Figure 3. As expected, the lung adenocarcinoma cell line HCC827, which exhibited highly activated Her1 and Her2 pathways as well as EGFR mutation, responded exceedingly well to the Her1 inhibitors, Erlotinib and Gefitinib. In addition, two other Her1/2 inhibitors, Lapatinib and BIBW-2992, were also able to inhibit the proliferation of this cell line. By contrast, the specific c-Met inhibitor PF-2341066 was not able to inhibit the proliferation of this cell line even though it exhibited the activated C-Met pathway. Moreover, inhibition of proliferation by BMS-536924, an IGF-1R inhibitor, in the HCC827 cells could be due to cross-talk between c-Met and IGF-1R pathways. The proliferation of two other Her1 and Her2 pathway activated adenocarcinoma cell lines, H1734 and H1975, was also inhibited by Erlotinib. Moreover, the irreversible Her1/2 kinase inhibitor, BIBW-2992, was also able to potently inhibit the growth of the H1975 cell line, which harbored the T790M and L858R mutations in the EGFR gene and thus rendering the cells resistant to Gefitinib and Lapatinib treatment. In addition, the IGF-1R inhibitor BMS-536924 was also able to inhibit the growth of this cell line. In regard to the metastatic adenocarcinoma cell line H1993, which exhibited a potently activated c-Met pathway, the c-Met inhibitor PF-2341066 was able to block its proliferation very effectively. What is more, the downstream MEK inhibitor PD-325901 was also able to block the proliferation of the H1993 cells, whereas the irreversible Her1/2 inhibitor BIBW-2992 and the PI3K inhibitor BEZ-235 were able to block the proliferation of this cell line but only weakly. Proliferation of the carcinoma cell line H358, which exhibited a moderate activation of the Her1, Her2 and IGF-1R pathways, was weakly blocked by the Her1/2 inhibitors, Lapatinib and Gefitinib, but the IGF-1R inhibitor BMS-536924 was able to inhibit its proliferation more effectively. Surprisingly, growth of the H358 cell line was not inhibited by the c-Met inhibitor PF-2341066, even though it exhibited a moderately activated c-Met pathway. Growth of the metastatic carcinoma cell line H1650, which harbored moderately activated Her1 and Her2 pathways, was inhibited by the irreversible Her1/2 inhibitor BIBW-2292. Proliferation of the large-cell carcinoma cell line A549, which exhibited moderately activated Her1 and IGF-1R pathways, was inhibited by the PI3K inhibitor BEZ-235 and by the IGF-1R inhibitor BMS-536924 and weakly by the MEK inhibitor PD-325901. Growth of the remaining metastatic large-cell carcinoma cell line H460, which harbored the PIK3CA gene mutation and an activated IGF-1R pathway, was inhibited by the PI3K inhibitor BEZ-235 but weakly inhibited by the IGF-1R inhibitor BMS-536924.

### Anchorage-independent growth inhibition of tumor cell lines by kinase inhibitors:

Since anchorage-independent growth is a hallmark of transformed cells, we wanted to ensure that the kinase inhibitors which were able to strongly block tumor cell proliferation in tissue culture were also able to inhibit the same tumor cells' proliferation in an anchorage-independent manner. As seen in Figure 4, Erlotinib, which inhibited the growth of HCC827 cells cultured in anchorage-dependent cell culture plates, was also able to reduce the size of the cell colonies grown in agar plates in an anchorage-independent fashion. Similarly, reduction of cell colony size formation was also observed by the treatment with the irreversible Her1/2 inhibitor BIBW-2992 in H1975 and H1650 cells as seen when these cells were grown in an anchorage-dependent manner. Large colony formation of H1993 cells, whose proliferation in tissue culture was potently blocked by treatment with the c-Met kinase inhibitor PF-2341066, was also inhibited by treatment with the same inhibitor. Proliferation of cell colony size in the H1734 cells was potently blocked by treatment with the downstream MEK inhibitor PD-325901 as seen when these cells were grown in cell culture. Likewise, the IGF-1R kinase inhibitor BMS-536924 was able to reduce cell colony size formation dose-dependently in the H358, A549 and H460 cells.

### Inhibition of tumor cell line growth by a combination of two kinase inhibitors:

Tumor cells often rely on signaling from multiple pathways and, hence, treating patients with a single agent can seldom eradicate tumor growth [Pao W. et al., PLoS Med 2, (73) (2005)]. A common clinical practice is to treat patients with combination therapies. In order to identify therapeutics with synergistic effects, we selected four tumor cell lines out of the eight and treated them with a combination of two kinase inhibitors, one inhibiting the appropriate RTK and the other inhibiting a downstream signaling pathway. As seen in Figure 5, the H1975 cells, whose growth was moderately inhibited by the Her1/2 RTK inhibitor BIBW-2992, but only weakly inhibited by the downstream MEK inhibitor PD-325901 and the downstream PI3K inhibitor BEZ-235, responded more effectively to a combination of BIBW-2992 with either PD-325901 or BEZ-235 with almost 100% growth inhibition of this cell line at 10 µM concentration, whereas a combination of the two downstream inhibitors, PD-325901 and BEZ-235, was much less effective. Likewise, combination of PD-325901 or BEZ-235 with the c-Met inhibitor PF-2341066 was more effective in blocking the proliferation of the H1993 cells, which exhibited a potently activated c-Met pathway, than by treating this cell line with the c-Met inhibitor PF-2341066 alone. Interestingly, a combination of both downstream kinase inhibitors, PD-325901 and BEZ-235, was the most effective in blocking the proliferation of this cell line. In the bronchioalveolar cell line H358, which exhibited Her1 and Her2 activation, treatment with the Her1 inhibitor Erlotinib in combination with either one of the two downstream inhibitors, PD-325901 or BEZ-235, showed synergistic inhibition of cell proliferation. A combination of the two downstream inhibitors, PD-0325901 and BEZ-235, was also highly effective. The same phenomenon was observed in the metastatic bronchioalveolar cell line H1650 when it was treated with the irreversible Her1/2 inhibitor BIBW-2992 in combination with one of the downstream inhibitor BEZ-235 or a combination of the two downstream inhibitors, PD-325901 and BEZ-235.

### Clustering of tumor tissue samples with tumor cell lines:

Profiling of the 50 human lung tissue samples using the CEER™ assay revealed distinct activated signaling pathways in each of the tissue samples as shown in the heat map in Figure 6A (see also Table 6). Based on these results, unsupervised one dimensional clustering of the 50 lung tumor tissue samples with the corresponding cell lines H1993, H1975, HCC827, H1734, H1650, H358, H460 and A549, could be performed as shown in Figure 6B because a significant degree of shared pathway activation exists between the primary tumor tissue samples and the tumor cell lines. Clustering of the tumor tissue samples with their corresponding cell lines provides potential insight into targeted therapy based on the drug treatment results obtained from the tumor cell lines.

**Table 6. Profiling of signaling pathways by the CEER™ assay and mutational status in lung tumor samples and lung tumor cell lines. The numbers in the table correspond to the computed cell units (CU) determined for each sample and the higher the number the more activated the pathway. (WT=wild type, Mut= mutated and N/A= status cannot be determined).**

| Samples | Her1 | Her2 | Her3 | cMet | IGF1R | PI3K | SHC | **KRAS** (G34A, G37T) | **KRAS** (A183T) | **P53** (C726G, G818T) | **STK11** (C109T, G595T) |
|---|---|---|---|---|---|---|---|---|---|---|---|
| LC1 | 15.8 | 1.5 | 46.5 | 1.5 | 12.1 | 10.8 | 9.5 | WT | WT | WT | WT |
| LC2 | 20.6 | 1.3 | 102.9 | 0.9 | 12.1 | 29.5 | 8.9 | WT | WT | WT | WT |
| LC3 | 1.9 | 0.3 | 10.1 | 0.2 | 0 | 0 | 1.5 | WT | WT | Mut | WT |
| LC4 | 2.7 | 0.6 | 9 | 0 | 1.7 | 1.2 | 7.4 | Mut | WT | WT | WT |
| LC5 | 0 | 1.7 | 1.6 | 0.5 | 2.6 | 5.5 | 1.5 | WT | WT | WT | WT |
| LC6 | 13.9 | 2.2 | 28.8 | 2.6 | 11.7 | 4.6 | 13.8 | WT | WT | Mut | WT |
| LC7 | 0 | 1.9 | 5 | 0.9 | 5.1 | 11.6 | 2 | Mut | WT | WT | WT |
| LC8 | 23.7 | 1.1 | 39.5 | 2.6 | 20.5 | 20.9 | 17.1 | WT | WT | WT | WT |
| LC9 | 0.7 | 0.4 | 7.1 | 0.4 | 1 | 1 | 6.3 | WT | WT | WT | WT |
| LC10 | 14.9 | 1.4 | 58.2 | 0.3 | 1.3 | 0.8 | 2.2 | WT | WT | Mut | WT |
| LC11 | 16.8 | 4.3 | 39.4 | 1.2 | 10.7 | 11.2 | 10.5 | WT | WT | N/A | WT |
| LC12 | 43.6 | 1.1 | 67.5 | 0.6 | 18.2 | 11.4 | 22.9 | WT | WT | WT | WT |
| LC13 | 20.6 | 0.7 | 42.7 | 0.5 | 14.3 | 20.4 | 6.9 | WT | WT | Mut | WT |
| LC14 | 17.6 | 1.3 | 46.1 | 2.6 | 36.6 | 21.3 | 21.2 | WT | WT | WT | WT |
| LC15 | 3.1 | 0.2 | 4 | 0.2 | 0.6 | 3.4 | 1.1 | WT | Mut | WT | WT |
| LC16 | 18.2 | 0.8 | 38 | 1.5 | 26.8 | 18.6 | 3.9 | Mut | WT | WT | WT |
| LC17 | 3.6 | 0.2 | 827 | 0.1 | 7.5 | 1.3 | 2.9 | WT | WT | N/A | WT |
| LC18 | 9 | 1.1 | 11.8 | 0.8 | 10.2 | 18.5 | 6.2 | WT | WT | WT | WT |
| LC19 | 7.5 | 2.2 | 324.8 | 2 | 4.9 | 22.6 | 52.3 | WT | WT | WT | WT |
| LC20 | 9.5 | 1.4 | 41.1 | 0.3 | 2.1 | 0.8 | 1.7 | Mut | WT | WT | WT |
| LC21 | 0 | 2.4 | 4.9 | 1.1 | 2.9 | 6.3 | 7.5 | Mut | WT | WT | WT |
| LC22 | 0 | 0 | 0 | 0.6 | 0 | 0 | 0 | WT | WT | WT | WT |
| LC23 | 22.6 | 0.4 | 251.3 | 0.1 | 5.8 | 24.9 | 4.9 | Mut | N/A | Mut | WT |
| LC24 | 10.8 | 0.5 | 35.1 | 0.4 | 11 | 4.9 | 0.2 | WT | WT | N/A | WT |
| LC25 | 26.7 | 2.9 | 47.6 | 2.3 | 19.1 | 33.3 | 28.3 | WT | N/A | WT | MUT |
| LC26 | 13 | 1.3 | 31.6 | 1.1 | 16.2 | 26.6 | 15.7 | WT | N/A | WT | WT |
| LC27 | 14.3 | 0.7 | 46.7 | 0.1 | 6.2 | 3.4 | 8.7 | WT | WT | WT | WT |
| LC28 | 8.1 | 1.8 | 501.6 | 0.1 | 2.7 | 11.2 | 175.3 | WT | N/A | WT | WT |
| LC29 | 16.4 | 0.2 | 535.7 | 0.4 | 0.5 | 0.4 | 1.3 | WT | WT | Mut | WT |
| LC30 | 4.1 | 0.2 | 5.1 | 0 | 1.3 | 1.2 | 0 | Mut | WT | WT | WT |
| LC31 | 70 | 5.1 | 99.7 | 7.8 | 36.2 | 69.5 | 75.4 | WT | N/A | Mut | WT |
| LC32 | 16.5 | 0.9 | 29.5 | 1.3 | 16.9 | 15.4 | 5.2 | N/A | WT | Mut | WT |
| LC33 | 19.3 | 0.6 | 78.3 | 0.1 | 8.6 | 1.6 | 10.4 | WT | WT | WT | WT |
| LC34 | 16.1 | 0.6 | 21.4 | 1.6 | 10.1 | 17.8 | 5.8 | WT | WT | WT | WT |
| LC35 | 0 | 0.8 | 29.4 | 0.1 | 0 | 0 | 5.6 | WT | WT | WT | WT |
| LC36 | 18.9 | 0.9 | 61.6 | 0.3 | 7.2 | 2.9 | 8.9 | WT | WT | Mut | WT |
| LC37 | 9.3 | 0.8 | 25.9 | 0.7 | 5 | 0 | 11 | WT | WT | WT | WT |
| LC38 | 0 | 1.6 | 12 | 1.3 | 8.1 | 16.1 | 3.4 | WT | WT | WT | WT |
| LC39 | 11.8 | 2.7 | 31.1 | 0.7 | 9.9 | 14.8 | 3 | WT | WT | WT | WT |
| LC40 | 27.7 | 2.4 | 49.8 | 2.1 | 16.1 | 12.3 | 25.4 | N/A | N/A | N/A | WT |
| LC41 | 0 | 1.5 | 4.2 | 0.9 | 2.9 | 6.6 | 1.6 | WT | WT | WT | WT |
| LC42 | 13.5 | 1.3 | 49 | 0 | 10 | 38.3 | 4.1 | WT | N/A | WT | WT |
| LC43 | 19.3 | 2.1 | 26.4 | 1.4 | 13.5 | 23.7 | 10.7 | WT | N/A | WT | WT |
| LC44 | 10.3 | 0.5 | 28.1 | 1.1 | 13.3 | 9.2 | 2.5 | Mut | WT | WT | WT |
| LC45 | 128.7 | 13.4 | 239.1 | 3.2 | 20.4 | 81.4 | 96.2 | Mut | WT | WT | WT |
| LC46 | 4.3 | 0.4 | 13.6 | 0.1 | 2.2 | 2.4 | 1.2 | WT | WT | WT | WT |
| LC47 | 26.5 | 2.6 | 83.6 | 0.9 | 31.6 | 68.4 | 21.2 | WT | WT | WT | WT |
| LC48 | 0 | 0.1 | 4.2 | 0.1 | 2.4 | 3.3 | 4.8 | WT | WT | WT | WT |
| LC49 | 15.8 | 2.6 | 15.9 | 0.2 | 1.7 | 1.2 | 5.6 | WT | WT | WT | WT |
| LC50 | 16.8 | 0.7 | 28.7 | 1.2 | 9 | 27.8 | 15.7 | WT | WT | WT | WT |
| H1993 | 24.4 | 7.3 | 97.5 | 11.2 | 2.2 | 60.9 | 31.6 | WT | WT | Mut | Mut |
| H1975 | 15.6 | 5.5 | 329.8 | 2.1 | 9.2 | 53.8 | 44.9 | WT | WT | Mut | WT |
| HCC827 | 20.3 | 6.6 | 530.9 | 2.5 | 10.5 | 48.6 | 31.7 | WT | WT | WT | WT |
| H1734 | 11.2 | 2.8 | 125.9 | 1.7 | 5 | 18.4 | 11 | Mut | WT | Mut | WT |
| H1650 | 12.2 | 1.4 | 21.9 | 0.2 | 2.5 | 10.8 | 13.7 | WT | WT | Mut | WT |
| H460 | 0.2 | 0.1 | 3.1 | 0.7 | 1.1 | 0.4 | 1.9 | WT | Mut | WT | Mut |
| H358 | 4.4 | 0.9 | 14.8 | 1.5 | 9.2 | 15.5 | 3 | Mut | WT | WT | WT |
| A549 | 15.8 | 0.6 | 22 | 1.6 | 11.5 | 16.3 | 4.9 | Mut | WT | WT | Mut |

### Gene mutation analysis of tumor tissue samples:

Mutation analysis of three frequently mutated genes (KRAS, P53 and STK11) in 50 human lung tissue samples further supported the clustering of human tumor samples into the cell line groups (see Table 6). For example, patient samples LC16, LC44 and LC23 were originally aligned with tumor cell line H358 based on CEER™ assay profiling. This alignment was supported by the finding that these three tissue samples also harbored the G34A and G37T KRAS mutations as was found in the H358 cell line. Similarly, the original alignment of patient samples LC45 with cell line H1734 and LC21 with cell line A549 was substantiated by finding the same G34A and G37T KRAS mutations in both patient samples and cell lines. In addition, alignment of the patient sample LC15 with cell line H460 is supported by the finding of the A183T KRAS mutation in both patient sample and cell line. Likewise, the alignment of patient samples LC6, LC31 and LC13 with cell lines H1975 and H1734 was substantiated by the finding of the C726G and G818T P53 mutations in both patient samples and cell lines. Also, the alignment of patient samples LC32 and LC23 with cell line H1650 was supported by the finding of the same P53 mutations in both patient samples and cell line. Lastly, the alignment of patient sample LC25 with the H1993 cell line was supported by the finding of the C109T and G595T STK11 mutations in both patient sample and cell line. Thus, clustering of the patient tissue samples with the tumor cell lines based on mutational status of the KRAS, P53 and STK11 genes was also consistent with the clustering based on the RTK pathway signatures.

### DISCUSSION

Current lung cancer treatments are less than optimal, with a mean survival of less than one year for advanced lung cancer patients, regardless of treatment regimen [Vaporciyan et al., Holland-Frei Cancer Medicine, 5th Edition, BC Decker, pgs. 1227-1292 (2000)]. Emerging new treatment modalities are generally targeted to inhibit specific tyrosine kinases activated in the tumor cells through basically two independent approaches [Roggli et al., Hum Pathol., Jun;16(6):569-79 (1985)]. The first approach is to use a highly specific monoclonal antibody to target the membrane growth factor receptor kinase that is responsible for tumor cell growth, and the resulting antibody/antigen complex invokes the host immune system to kill the tumor cells. This approach is exemplified by the treatment of Her2/Neu receptor-positive breast cancer with Herceptin, a humanized monoclonal antibody against this receptor [Schiller et al., N Engl J Med., Jan 10; 346(2):92-8 (2002)]. However, the high cost of monoclonal antibody drugs could be a disadvantage for this approach. The second approach is to develop cell-penetrating small organic molecules that target the specific tyrosine kinases in the signaling pathways of the tumor cells. This approach is best exemplified by the use of Gleevec to block the activation of the BCR-ABL fusion kinase in chronic myelogenous leukemia [Comis RL, The Oncologist, Aug; 20(7):467-70 (2005)]. The design and synthesis of small molecule tyrosine kinase inhibitors have been greatly facilitated by the availability of crystal structures for the tyrosine kinases in the past decade and effective kinase inhibitors have thus been produced by many large and small pharmaceutical companies. Nevertheless, without prior knowledge of the activated kinase signaling pathways responsible for propagating and metastasis of the tumor cells, it is not possible to apply the targeted therapy approach with the available kinase inhibitors. Therefore, we have selected a panel of eight lung tumor cell lines that harbored the most frequently detected gene mutations: P53, KRAS, STK11, and EGFR as representative examples that cover the major human lung cancer subtypes.

Mutation analysis has provided valuable information in guiding targeted therapy for cancer patients. A good example is found in cancer patients who carry a KRAS gene mutation because these patients have been shown to be non-responsive to anti-EGFR therapeutics. Therefore, KRAS mutation testing is becoming routinely performed in patients who are being considered for anti-EGFR therapy with either Cetuximab or Panitumumab in Europe and the United States [Le Calvez et al., Cancer Res, 65:5076-5083 (2005); Pao et al., PLoS Med., 2,e17 (2005)]. Our current study further confirmed an important role of gene mutation analysis in guiding the use of drugs to treat lung cancer. For example, it has been reported that cancers from patients with lung adenocarcinoma that harbored mutations within the tyrosine kinase domain of the EGFR gene often responded initially to TKI drugs such as Gefitinib and Erlotinib [Sjoblom et al., Science; 314:268-74 (2006); Potti et al., Nat Med; 12:1294-300 (2006)] but usually developed drug-resistance later [Pao et al., Proc. Natl Acad. Sci. USA; 101:13306-13311 (2004); Lynch et al., N. Engl. J. Med, 350:2129-2139 (2004); Kwak et al., PNAS 102(21):7665-7670 (2005)]. Indeed, the HCC827, H1975 and H1650 lung tumor cell lines employed in this study harbored the EGFR gene mutation and they were found to be sensitive to EGFR inhibitor treatment. By contrast, KRAS gene mutation is associated with resistance to EGFR tyrosine kinase inhibitors [Paez et al., Science 304:1497-1500 (2004); Kobayashi et al., N. Engl. J. Med, 352 (20):786-792 (2005)]. This phenomenon is substantiated by our finding that the A549 and H460 cells, which harbored the KRAS mutated gene, did not respond to treatment with EGFR inhibitors but they are sensitive to the downstream PI3K inhibitor BEZ-235.

Although targeted therapy based on association of somatic mutation analysis and drug sensitivity has greatly facilitated lung cancer treatment, the profiling of oncogenic signal transduction pathways in human tumor cells and tissues offer yet another complementary approach to guide therapeutic treatment [Judith S. Sebolt-Leopold, Clin Cancer Res; 14(12):3651-6 (2008); Rusnak et al., Cell Prolif; 40:580-94 (2007); Paull et al., J Natl Cancer Inst; 81:1088-92 (1989); McDermott et al., Proc Natl Acad Sci USA; 104:19936-41 (2007); Nakatsu et al., Mol Pharmacol; 72:1171-80 (2007)]. Traditional signaling pathway profiling in tumor tissue samples by immunohistochemistry staining or western blotting methods are neither quantitative nor sensitive enough to have utility when only small amount of tumor tissue samples is available. The CEER™ assay is an assay we have developed to overcome the low sensitivity and specificity issues associated with these traditional methods. The CEER™ assay uses a multiplexed, proximity-based, collaborative immunoassay platform that can provide clinical information on a limited amount of tissue samples with high sensitivity and specificity. The principle of the assay is based on the formation of a unique immuno-complex that requires the co-localization of two detecting antibodies against a target protein once the protein is captured on the microarray surface. It is the formation of this complex that enables the generation of a highly specific and sensitive signal to reveal the activation status of the target protein. Using this assay, the activated Her1, Her2, Her3, c-Met, IGF-1R, PI3K and SHC pathways present in the eight lung tumor cell lines as well as the 50 human lung tumor tissue samples were profiled. Cell lines that exhibited one or more of these activated pathways were treated with the corresponding kinase inhibitors and the results demonstrated that this matching approach is effective in inhibiting the pathway activation and growth of these cell lines under both anchorage-dependent and anchorage-independent culture conditions. Moreover, in those cell lines in which significant growth inhibition could not be achieved with a single kinase inhibitor treatment (H1993, H358 and H1650), a combination treatment with two kinase inhibitors, one targeting the RTK and the other targeting a downstream kinase, was effective in blocking their proliferation. Thus, characterization of the activated signaling pathways and mutational status as well as their cell growth inhibition by kinase inhibitors in the lung tumor cell lines could facilitate the target-focused treatment of lung cancer.

Pathway profiling using the CEER™ assay and gene mutational analysis of the 50 tumor tissues collected from lung cancer patients clearly showed some similarities in the biomarkers between the lung tumor tissue samples and tumor cell lines. Therefore, the panel of pathway biomarkers and mutated genes can discriminate and cluster different tumor tissue samples with the corresponding tumor cell lines in both supervised and unsupervised clustering analysis and this information could be used to guide treatment options based on the drug sensitivity of the tumor cell lines.

## Claims

1. A method for predicting therapeutic efficacy or response to an anticancer drug in a subject having lung cancer, said method comprising:
(a) determining the phosphorylation level of HER1, HER2, and HER3 in a cellular extract produced from a cancer cell which has been isolated from said subject;
(b) comparing the phosphorylation level of HER1, HER2, and HER3 in the cellular extract to a phosphorylation level of HER1, HER2, and HER3 in each of the following lung cancer cell lines: HCC827, H1975, H1734, H1993, H358, H1650, A549, and H460;
wherein each anticancer drug selected from the group consisting of a HER1 inhibitor, a HER1/2 inhibitor, a HER1/2/4 inhibitor, a c-Met inhibitor, an IGF-1R inhibitor, a MEK inhibitor, a PI3K inhibitor, and a combination thereof has been determined to produce or not produce a response in each of the lung cancer cell lines;
(c) identifying similarities between the phosphorylation level of HER1, HER2, and HER3 in the cellular extract and in the lung cancer cell lines; and
(d) predicting that the lung cancer of the subject will respond to the same anticancer drug or combination thereof that produces a response in a lung cancer cell line of step (b) based on similarities between the phosphorylation level of HER1, HER2, and HER3 in the cellular extract and the phosphorylation level of HER1, HER2, and HER3 in the lung cancer cell line.

2. The method of claim 1, wherein the phosphorylation level of HER1, HER2, and HER3 is calibrated against a standard curve generated for HER1, HER2, and HER3.

3. The method of claim 1 or 2, wherein said lung cancer is a non-small cell lung cancer.

4. The method of any one of claims 1 to 3, wherein said cancer cell is stimulated *in vitro* with one or more growth factors.

5. The method of any one of claims 1 to 4, wherein said cancer cell has been isolated from a lung tumor tissue of said subject.

6. The method of any one of claims 1 to 5, wherein step (a) further comprises determining the phosphorylation level of a marker selected from the group consisting of a receptor tyrosine kinase, a non-receptor tyrosine kinase, a tyrosine kinase signaling cascade component, a nuclear hormone receptor, a nuclear receptor coactivator, a nuclear receptor repressor, and combinations thereof.

7. The method of any one of claims 1 to 6, wherein step (a) further comprises determining the phosphorylation level of at least one or more of cMET, IGF-1R, PI3K, and/or SHC.

8. The method of any one of claims 1 to 7, wherein the lung cancer cell line having the similar phosphorylation level of HER1, HER2, and HER3 is HCC827 and the lung cancer is predicted to respond to a HER1 inhibitor, a HER1/2 inhibitor, a HER1/2/4 inhibitor, or combinations thereof, or
wherein the lung cancer cell line having the similar phosphorylation level of HER1, HER2, and HER3 is HI975 and the lung cancer is predicted to respond to a HER1/2 inhibitor, a HER1 inhibitor, an IGF-1R inhibitor, or a combination of a HER1/2 inhibitor with a MEK inhibitor or a PI3K inhibitor, or
wherein the lung cancer cell line having the similar phosphorylation level of HER1, HER2, and HER3 is H1734 and the lung cancer is predicted to respond to a HER1/2 inhibitor, a HER1 inhibitor, a MEK inhibitor, or combinations thereof, or
wherein the lung cancer cell line having the similar phosphorylation level of HER1, HER2, and HER3 is HI993 and the lung cancer is predicted to respond to a c-Met inhibitor, a MEK inhibitor, a combination of a c-Met inhibitor with a MEK inhibitor or a PI3K inhibitor, or a combination of a MEK inhibitor and a PI3K inhibitor, or
wherein the lung cancer cell line having the similar phosphorylation level of HER1, HER2, and HER3 is H358 and the lung cancer is predicted to respond to a HER1/2 inhibitor, a HER1 inhibitor, a HER1/2/4 inhibitor, an IGF-1R inhibitor, a combination of a HER1 inhibitor with a MEK inhibitor or a PI3K inhibitor, or a combination of a MEK inhibitor and a PI3K inhibitor, or
wherein the lung cancer cell line having the similar phosphorylation level of HER1, HER2, and HER3 is H1650 and the lung cancer is predicted to respond to a HER1/2 inhibitor, a combination of a HER1/2 inhibitor with a PI3K inhibitor, or a combination of a MEK inhibitor and a PI3K inhibitor, or
wherein the lung cancer cell line having the similar phosphorylation level of HER1, HER2, and HER3 is A549 and the lung cancer is predicted to respond to an IGF-1R inhibitor, a MEK inhibitor, a PI3K inhibitor, or combinations thereof, or
wherein the lung cancer cell line having the similar phosphorylation level of HER1, HER2, and HER3 is H460 and the lung cancer is predicted to respond to an IGF-1R inhibitor, a PI3K inhibitor, or combinations thereof.

9. The method of any one of claims 1 to 8, wherein step (a) comprises determining the phosphorylation level of HER1, HER2, and HER3 with a Collaborative Enzyme Enhanced Reactive Immunoassay (CEER).

10. The method of any one of claims 1 to 9, further comprising genotyping nucleic acid obtained from said cancer cell to determine the presence or absence of a variant allele in a gene,
wherein said gene is optionally selected from the group consisting of an oncogene, a tumor suppressor gene, and combinations thereof, or
wherein said oncogene is optionally selected from the group consisting of KRAS, BRAF, PIK3CA, EGFR, and combinations thereof, or
wherein said tumor suppressor gene is optionally selected from the group consisting of P53, STK11, and combinations thereof.

11. The method of claim 10, wherein determining the presence or absence of said variant allele in conjunction with determining the phosphorylation level of HER1, HER2, and HER3 further aids or improves the prediction of therapeutic efficacy or response to an anticancer drug.

12. The method of claim 10 or 11, wherein said variant allele comprises a single nucleotide polymorphism (SNP).

## Patentansprüche

1. Verfahren zur Vorhersage der therapeutischen Wirksamkeit oder des therapeutischen Ansprechens auf ein Antikrebsmedikament in einem Subjekt mit Lungenkrebs, wobei das Verfahren Folgendes umfasst:
(a) Bestimmen des Phosphorylierungsniveaus von HER1, HER2 und HER3 in einem zellulären Extrakt, der aus einer Krebszelle, die aus dem Subjekt isoliert wurde, hergestellt wurde;
(b) Vergleichen des Phosphorylierungsniveaus von HER1, HER2 und HER3 im zellulären Extrakt mit einem Phosphorylierungsniveau von HER1, HER2 und HER3 in jeder der folgenden Lungenkrebszellreihen: HCC827, H1975, H1734, H1993, H358, H1650, A549 und H460;
wobei bei jedem Antikrebsmedikament ausgewählt aus der Gruppe bestehend aus einem HER1-Inhibitor, einem HER1/2-Inhibitor, einem HER1/2/4-Inhibitor, einem c-Met-Inhibitor, einem IGF-1R-Inhibitor, einem MEK-Inhibitor, einem PI3K-Inhibitor und einer Kombination davon, bestimmt wurde, ob es ein Ansprechen in den jeweiligen Lungenkrebszellreihen erzeugt oder nicht;
(c) Identifizieren von Ähnlichkeiten zwischen dem Phosphorylierungsniveau von HER1, HER2 und HER3 in dem Zellextrakt und in den Lungenkrebszellreihen; und
(d) Vorhersagen, dass der Lungenkrebs des Subjekts auf dasselbe Antikrebsmedikament oder eine Kombination davon, die ein Ansprechen in einer Lungenkrebszellreihe von Schritt (b) erzeugt, auf der Grundlage von Ähnlichkeiten zwischen dem Phosphorylierungsniveau von HER1, HER2 und HER3 in dem Zellextrakt und dem Phosphorylierungsniveau von HER1, HER2 und HER3 in der Lungenkrebszellreihe ansprechen wird.

2. Verfahren nach Anspruch 1, wobei das Phosphorylierungsniveau von HER1, HER2 und HER3 gegen eine für HER1, HER2 und HER3 erzeugte Standardkurve kalibriert wird.

3. Verwendung nach Anspruch 1 oder 2, wobei der Lungenkrebs ein nicht kleinzelliger Lungenkrebs ist.

4. Verfahren nach einem der Ansprüche 1 bis 3, wobei die Krebszelle *in vitro* mit einem oder mehreren Wachstumsfaktoren stimuliert wird.

5. Verfahren nach einem der Ansprüche 1 bis 4, wobei die Krebszelle aus einem Lungentumorgewebe des Subjekts isoliert wurde.

6. Verfahren nach einem der Ansprüche 1 bis 5, wobei der Schritt (a) ferner das Bestimmen des Phosphorylierungsniveaus eines Markers umfasst, der ausgewählt ist aus der Gruppe bestehend aus einer Rezeptortyrosinkinase, einer Nicht-Rezeptortyrosinkinase, einer Tyrosinkinase-Signalkaskadenkomponente, einem Kernhormonrezeptor, einem Kernrezeptor-Coaktivator, einem Kernrezeptorrepressor und Kombinationen davon.

7. Verfahren nach einem der Ansprüche 1 bis 6, wobei Schritt (a) ferner das Bestimmen des Phosphorylierungsniveaus von mindestens einem oder mehreren von cMET, IGF-1R, PI3K und/oder SHC umfasst.

8. Verfahren nach einem der Ansprüche 1 bis 7, wobei die Lungenkrebszellreihe mit dem ähnlichen Phosphorylierungsniveau von HER1, HER2 und HER3 HCC827 ist und vorhergesagt wird, dass der Lungenkrebs auf einen HER1-Inhibitor, einen HER1/2-Inhibitor, einen HER1/2/4-Inhibitor oder Kombinationen davon anspricht, oder
wobei die Lungenkrebszellreihe mit dem ähnlichen Phosphorylierungsniveau von HER1, HER2 und HER3 HI975 ist und vorhergesagt wird, dass der Lungenkrebs auf einen HER1/2-Inhibitor, einen HER1-Inhibitor, einen IGF-1R-Inhibitor oder eine Kombination von einem HER1/2-Inhibitor mit einem MEK-Inhibitor oder einem PI3K-Inhibitor anspricht, oder
wobei die Lungenkrebszellreihe mit dem ähnlichen Phosphorylierungsniveau von HER1, HER2 und HER3 H1734 ist und vorhergesagt wird, dass der Lungenkrebs auf einen HER1/2-Inhibitor, einen HER1-Inhibitor, einen MEK-Inhibitor oder Kombinationen davon anspricht, oder
wobei die Lungenkrebszellreihe mit dem ähnlichen Phosphorylierungsniveau von HER1, HER2 und HER3 HI993 ist und vorhergesagt wird, dass der Lungenkrebs auf einen c-Met-Inhibitor, einen MEK-Inhibitor, eine Kombination eines c-Met-Inhibitors mit einem MEK-Inhibitor oder einem PI3K-Inhibitor oder eine Kombination eines MEK-Inhibitors und eines PI3K-Inhibitors anspricht, oder
wobei die Lungenkrebszellreihe mit dem ähnlichen Phosphorylierungsniveau von HER1, HER2 und HER3 H358 ist und vorhergesagt wird, dass der Lungenkrebs auf einen HER1/2-Inhibitor, einen HER1-Inhibitor, einen HER1/2/4-Inhibitor, einen IGF-1R-Inhibitor, eine Kombination eines HER1-Inhibitors mit einem MEK-Inhibitor oder einem PI3K-Inhibitor oder eine Kombination eines MEK-Inhibitors und eines PI3K-Inhibitors anspricht, oder
wobei die Lungenkrebszellreihe mit dem ähnlichen Phosphorylierungsniveau von HER1, HER2 und HER3 HI650 ist und vorhergesagt wird, dass der Lungenkrebs auf einen HER1/2-Inhibitor, eine Kombination eines HER1/2-Inhibitors mit einem PI3K-Inhibitor oder eine Kombination eines MEK-Inhibitors und eines PI3K-Inhibitors anspricht, oder
wobei die Lungenkrebszellreihe mit dem ähnlichen Phosphorylierungsniveau von HER1, HER2 und HER3 A549 ist und vorhergesagt wird, dass der Lungenkrebs auf einen IGF-1R-Inhibitor, einen MEK-Inhibitor, einen PI3K-Inhibitor oder Kombinationen davon anspricht, oder
wobei die Lungenkrebszellreihe mit dem ähnlichen Phosphorylierungsniveau von HER1, HER2 und HER3 H460 ist und vorhergesagt wird, dass der Lungenkrebs auf einen IGF-1R-Inhibitor, einen PI3K-Inhibitor oder Kombinationen davon anspricht.

9. Verfahren nach einem der Ansprüche 1 bis 8, wobei Schritt (a) das Bestimmen des Phosphorylierungsniveaus von HER1, HER2 und HER3 mit einem mit einem kollaborativen, enzymverstärkten, reaktiven Immuntest (CEER) umfasst.

10. Verfahren nach einem der Ansprüche 1 bis 9, ferner umfassend das Genotypisieren von Nukleinsäure, die von der Krebszelle erhalten wird, um die Gegenwart oder Abwesenheit eines Variantenallels in einem Gen zu bestimmen,
wobei das Gen wahlweise ausgewählt ist aus der Gruppe bestehend aus einem Onkogen, einem Tumorsuppressorgen und Kombinationen davon, oder
wobei das Onkogen wahlweise ausgewählt ist aus der Gruppe bestehend aus KRAS, BRAF, PIK3CA, EGFR und Kombinationen davon, oder
wobei das Tumorsuppressorgen wahlweise ausgewählt ist aus der Gruppe bestehend aus P53, **STK11** und Kombinationen davon.

11. Verfahren nach Anspruch 10, wobei das Bestimmen der Gegenwart oder Abwesenheit des Variantenallels zusammen mit der Bestimmung des Phosphorylierungsniveaus von HER1, HER2 und HER3 die Vorhersage der therapeutischen Wirksamkeit oder des therapeutischen Ansprechens auf ein Antikrebsmedikament weiter unterstützt oder verbessert.

12. Verfahren nach Anspruch 10 oder 11, wobei das Variantenallel einen einzelnen Nukleotidpolymorphismus (SNP) umfasst.

## Revendications

1. Procédé pour prédire l'efficacité ou la réponse thérapeutique à un médicament anticancéreux chez un sujet souffrant d'un cancer du poumon, ledit procédé comprenant :
(a) la détermination du taux de phosphorylation de HER1, HER2 et HER3 dans un extrait cellulaire produit à partir d'une cellule cancéreuse qui a été isolée dudit sujet ;
(b) la comparaison du taux de phosphorylation de HER1, HER2 et HER3 dans l'extrait cellulaire à un taux de phosphorylation de HER1, HER2 et HER3 dans chacune des lignées cellulaires de cancer du poumon suivantes : HCC827, H1975, H1734, H1993, H358, H1650, A549 et H460 ;
dans lequel chaque médicament anticancéreux choisi dans le groupe constitué d'un inhibiteur de HER1, un inhibiteur de HER1/2, un inhibiteur de HER1/2/4, un inhibiteur de c-Met, un inhibiteur d'IGF-1R, un inhibiteur de MEK, un inhibiteur de PI3K, et une combinaison de ceux-ci a été déterminé comme produisant ou ne produisant pas de réponse dans chacune des lignées cellulaires de cancer du poumon ;
(c) l'identification de similarité entre les taux de phosphorylation de HER1, HER2 et HER3 dans l'extrait cellulaire et dans les lignées cellulaires de cancer du poumon ; et
(d) la prévision que le cancer du poumon du sujet répondra au même médicament anticancéreux ou à une combinaison de ceux-ci qui produit une réponse dans une lignée cellulaire de cancer du poumon de l'étape (b) sur la base de similarité entre le taux de phosphorylation de HER1, HER2 et HER3 dans l'extrait cellulaire et le taux de phosphorylation de HER1, HER2 et HER3 dans la lignée cellulaire de cancer du poumon.

2. Procédé selon la revendication 1, dans lequel le taux de phosphorylation de HER1, HER2 et HER3 est étalonné vis-à-vis d'une courbe étalon générée pour HER1, HER2 et HER3.

3. Procédé selon la revendication 1 ou 2, dans lequel ledit cancer du poumon est un cancer du poumon non à petites cellules.

4. Procédé selon l'une quelconque des revendications 1 à 3, dans lequel ladite cellule cancéreuse est stimulée *in vitro* avec un ou plusieurs facteurs de croissance.

5. Procédé selon l'une quelconque des revendications 1 à 4, dans lequel ladite cellule cancéreuse a été isolée à partir d'un tissu tumoral de poumon dudit sujet.

6. Procédé selon l'une quelconque des revendications 1 à 5, dans lequel l'étape (a) comprend en outre la détermination du taux de phosphorylation d'un marqueur choisi dans le groupe constitué d'une tyrosine kinase réceptrice, une tyrosine kinase non réceptrice, un composant de cascade de signalisation de tyrosine kinase, un récepteur hormonal nucléaire, un coactivateur de récepteur nucléaire, un répresseur de récepteur nucléaire, et des combinaisons de ceux-ci.

7. Procédé selon l'une quelconque des revendications 1 à 6, dans lequel l'étape (a) comprend en outre la détermination du taux de phosphorylation d'au moins un ou plus parmi cMET, IGF-1R, PI3K et/ou SHC.

8. Procédé selon l'une quelconque des revendications 1 à 7, dans lequel la lignée cellulaire de cancer du poumon ayant le taux de phosphorylation similaire de HER1, HER2 et HER3 est HCC827 et il est prédit que le cancer du poumon répondra à un inhibiteur de HER1, à un inhibiteur de HER1/2, à un inhibiteur de HER1/2/4, ou à des combinaisons de ceux-ci, ou
dans lequel la lignée cellulaire de cancer du poumon ayant le taux de phosphorylation similaire de HER1, HER2 et HER3 est HI975 et il est prédit que le cancer du poumon répondra à un inhibiteur de HER1/2, à un inhibiteur de HER1, à un inhibiteur d'IGF-1R, ou à une combinaison d'un inhibiteur de HER1/2 avec un inhibiteur de MEK ou un inhibiteur de PI3K, ou
dans lequel la lignée cellulaire de cancer du poumon ayant le taux de phosphorylation similaire de HER1, HER2 et HER3 est H1734 et il est prédit que le cancer du poumon répondra à un inhibiteur de HER1/2, à un inhibiteur de HER1, à un inhibiteur de MEK, ou à des combinaisons de ceux-ci, ou
dans lequel la lignée cellulaire de cancer du poumon ayant le taux de phosphorylation similaire de HER1, HER2 et HER3 est HI993 et il est prédit que le cancer du poumon répondra à un inhibiteur de c-Met, à un inhibiteur de MEK, à une combinaison d'un inhibiteur de c-Met avec un inhibiteur de MEK ou un inhibiteur de PI3K, ou à une combinaison d'un inhibiteur de MEK et d'un inhibiteur de PI3K, ou
dans lequel la lignée cellulaire de cancer du poumon ayant le taux de phosphorylation similaire de HER1, HER2 et HER3 est H358 et il est prédit que le cancer du poumon répondra à un inhibiteur de HER1/2, à un inhibiteur de HER1, à un inhibiteur de HER1/2/4, à un inhibiteur d'IGF-1R, à une combinaison d'un inhibiteur de HER1 avec un inhibiteur de MEK ou un inhibiteur de PI3K, ou à une combinaison d'un inhibiteur de MEK et d'un inhibiteur de PI3K, ou
dans lequel la lignée cellulaire de cancer du poumon ayant le taux de phosphorylation similaire de HER1, HER2 et HER3 est HI650 et il est prédit que le cancer du poumon répondra à un inhibiteur de HER1/2, à une combinaison d'un inhibiteur de HER1/2 avec un inhibiteur de PI3K, ou à une combinaison d'un inhibiteur de MEK et d'un inhibiteur de PI3K, ou
dans lequel la lignée cellulaire de cancer du poumon ayant le taux de phosphorylation similaire de HER1, HER2 et HER3 est A549 et il est prédit que le cancer du poumon répondra à un inhibiteur d'IGF-1R, à un inhibiteur de MEK, à un inhibiteur de PI3K, ou à des combinaisons de ceux-ci, ou
dans lequel la lignée cellulaire de cancer du poumon ayant le taux de phosphorylation similaire de HER1, HER2 et HER3 est H460 et il est prédit que le cancer du poumon répondra à un inhibiteur d'IGF-1R, à un inhibiteur de PI3K, ou à des combinaisons de ceux-ci.

9. Procédé selon l'une quelconque des revendications 1 à 8, dans lequel l'étape (a) comprend la détermination du taux de phosphorylation de HER1, HER2 et HER3 avec un dosage immunologique réactif amélioré par enzyme collaborative (CEER).

10. Procédé selon l'une quelconque des revendications 1 à 9, comprenant en outre un génotypage d'un acide nucléique obtenu à partir de ladite cellule cancéreuse pour déterminer la présence ou l'absence d'un allèle variant dans un gène,
dans lequel ledit gène est éventuellement choisi dans le groupe constitué d'un oncogène, un gène suppresseur de tumeur, et des combinaisons de ceux-ci, ou
dans lequel ledit oncogène est éventuellement choisi dans le groupe constitué de KRAS, BRAF, PIK3CA, EGFR, et des combinaisons de ceux-ci, ou
dans lequel ledit gène suppresseur de tumeur est éventuellement choisi dans le groupe constitué de P53, STK11, et des combinaisons de ceux-ci.

11. Procédé selon la revendication 10, dans lequel la détermination de la présence ou de l'absence dudit allèle variant conjointement avec la détermination du taux de phosphorylation de HER1, HER2 et HER3 assiste ou améliore en outre la prévision d'efficacité ou la réponse thérapeutique à un médicament anticancéreux.

12. Procédé selon la revendication 10 ou 11, dans lequel ledit allèle variant comprend un polymorphisme mononucléotidique (SNP).
